# EUROPEAN PATENT SPECIFICATION

(11) **EP 1 053 330 B1**
(45) Date of publication and mention of the grant of the patent: **04.05.2005**
(21) Application number: 99904857.2
(22) Date of filing: 04.02.1999
(51) Int. Cl.: C12N 15/31, C07K 14/31, A61K 38/16

(54) **IDENTIFICATION, PRODUCTION AND USE OF STAPHYLOKINASE DERIVATIVES WITH REDUCED IMMUNOGENICITY AND/OR REDUCED CLEARANCE**
IDENTIFIZIERUNG, PRODUKTION UND VERWENDUNG VON STAPHYLOKINASE DERIVATEN MIT REDUZIERTER IMMUNOGENIZITÄT UND/ODER REDUZIERTER 'CLEARANCE'
IDENTIFICATION, PRODUCTION ET UTILISATION DE DERIVES DE LA STAPHYLOKINASE AVEC IMMUNOGENECITE ET/OU CLEARANCE REDUITES

(30) Priority: 04.02.1998 EP 98200323; 06.02.1998 EP 98200365
(43) Date of publication of application: 22.11.2000
(73) Proprietor: Thromb-X N.V., 3000 Leuven (BE); Collen, Désiré José, B-3020 Winksele-Herent (BE)
(72) Inventor: COLLEN, Désiré, José, B-3020 Winksele-Herent (BE)
(74) Representative: Van Someren, Petronella F. H. M.
(86) International application number: PCT/EP1999/000748
(87) International publication number: WO 1999/040198

(56) References cited:
- EP-A- 0 721 982
- COLLEN, D. ET AL: "Thrombolytic properties of poorly immunogenic variants of recombinant staphylokinase." FIBRINOLYSIS & PROTEOLYSIS, (JUNE, 1998) VOL. 12, NO. SUPPL. 1, PP. 30. MEETING INFO.: XIVTH INTERNATIONAL CONGRESS ON FIBRINOLYSIS AND THROMBOLYSIS LJUBLJANA, SLOVENIA JUNE 22-26, 1998 , XP002111034
- COLLEN D ET AL: "Recombinant staphylokinase variants with altered immunoreactivity. III: Species variability of antibody binding patterns." CIRCULATION, (1997 JAN 21) 95 (2) 455-62. , XP002111035
- COLLEN D ET AL: "Recombinant staphylokinase variants with altered immunoreactivity. II: Thrombolytic properties and antibody induction." CIRCULATION, (1996 JUL 15) 94 (2) 207-16. , XP002111036
- COLLEN D ET AL: "Recombinant staphylokinase variants with altered immunoreactivity. I: Construction and characterization." CIRCULATION, (1996 JUL 15) 94 (2) 197-206. , XP002111037
- COLLEN D ET AL: "Recombinant staphylokinase variants with altered immunoreactivity. IV: Identification of variants with reduced antibody induction but intact potency." CIRCULATION, (1997 JAN 21) 95 (2) 463-72. , XP002111038
- INADA Y. ET AL: 'Biomedical and biotechnological applications of PEG- and PM-modified proteins.' TIBTECH vol. 13, 1995, pages 86 - 91

### SEQUENCE LISTING

<110> Leuven Research & Development VZW
<120> Identification, production and use of new staphylokinase derivatives with reduced immunogenicity and/or reduced clearance
<130> leuvresdev102
<140> PCT/EP/00748
   <141> 1999-02-04
<160> 10
<170> PatentIn Ver. 2.1
<210> 1
   <211> 20
   <212> DNA
   <213> Artificial Sequence
<400> 1
   caggaaacag aattcaggag                                    20
<210> 2
   <211> 28
   <212> DNA
   <213> Artificial Sequence
<220>
   <223> Description of Artificial Sequence: mutagenic primer LY58
<400> 2
   caaaacagcc aagcttcatt cattcagc                           28
<210> 3
   <211> 28
   <212> DNA
   <213> Artificial Sequence
<220>
   <223> Description of Artificial Sequence: selection primer LY34
<400> 3
   caaaacagcc gagcttcatt cattcagc 28
<210> 4
   <211> 41
   <212> DNA
   <213> Artificial Sequence
<220>
   <223> Description of Artificial Sequence: mutagenic primer LY58
<400> 4
   ttcagcatgc tgcagttatt tcttttctgc aacaaccttg g 41
<210> 5
   <211> 27
   <212> DNA
   <213> Artificial Sequence
<220>
   <223> Description of Artificial Sequence: primer 818D
<400> 5
   caaacagcca agcttcattc attcagc 27
<210> 6
   <211> 27
   <212> DNA
   <213> Artificial Sequence
<220>
   <223> Description of Artificial Sequence: backward primer for construction of K102C
<400> 6
   tatgataaga attgcaaaaa agaagaa 27
<210> 7
   <211> 27
   <212> DNA
   <213> Artificial Sequence
<220>
   <223> Description of Artificial Sequence: forward primer for construction of K102C
<400> 7
   ttcttctttt ttgcaattct tatcata 27
<210> 8
   <211> 27
   <212> DNA
   <213> Artificial Sequence
<220>
   <223> Description of Artificial Sequence: backward primer for construction of K109C
<400> 8
   aaaaagaaga aacgtgctct ttcccta 27
<210> 9
   <211> 27
   <212> DNA
   <213> Artificial Sequence
<220>
   <223> Description of Artificial Sequence: forward primer for construction of K109C
<400> 9
   tagggaaaga gcacgtttct tcttttt 27
<210> 10
   <211> 136
   <212> PRT
   <213> Staphylococcus aureus
<400> 10
   Ser Ser Ser Phe Asp Lys Gly Lys Tyr Lys Lys Gly Asp Asp Ala Ser
   1 5 10 15
   Tyr Phe Glu Pro Thr Gly Pro Tyr Leu Met Val Asn Val Thr Gly Val
   20 25 30
   Asp Ser Lys Gly Asn Glu Leu Leu Ser Pro His Tyr Val Glu Phe Pro
   35 40 45
   Ile Lys Pro Gly Thr Thr Leu Thr Lys Glu Lys Ile Glu Tyr Tyr Val
   50 55 60
   Glu Trp Ala Leu Asp Ala Thr Ala Tyr Lys Glu Phe Arg Val Val Glu
   65 70 75 80
   Leu Asp Pro Ser Ala Lys Ile Glu Val Thr Tyr Tyr Asp Lys Asn Lys
   85 90 95
   Lys Lys Glu Glu Thr Lys Ser Phe Pro Ile Thr Glu Lys Gly Phe Val
   100 105 110
   Val Pro Asp Leu Ser Glu His Ile Lys Asn Pro Gly Phe Asn Leu Ile
   115 120 125
   Thr Lys Val Val Ile Glu Lys Lys
   130 135

## Description

The present invention relates to new staphylokinase derivatives with reduced immunogenicity which can be administered by continuous infusion or by single intravenous bolus injection, to their identification, production and use in the treatment of arterial thrombosis and to the preparation of a pharmaceutical composition for treating arterial thrombosis. More in particular the invention relates to the use of engineered staphylokinase derivatives for the preparation of a pharmaceutical composition for treating myocardial infarction.

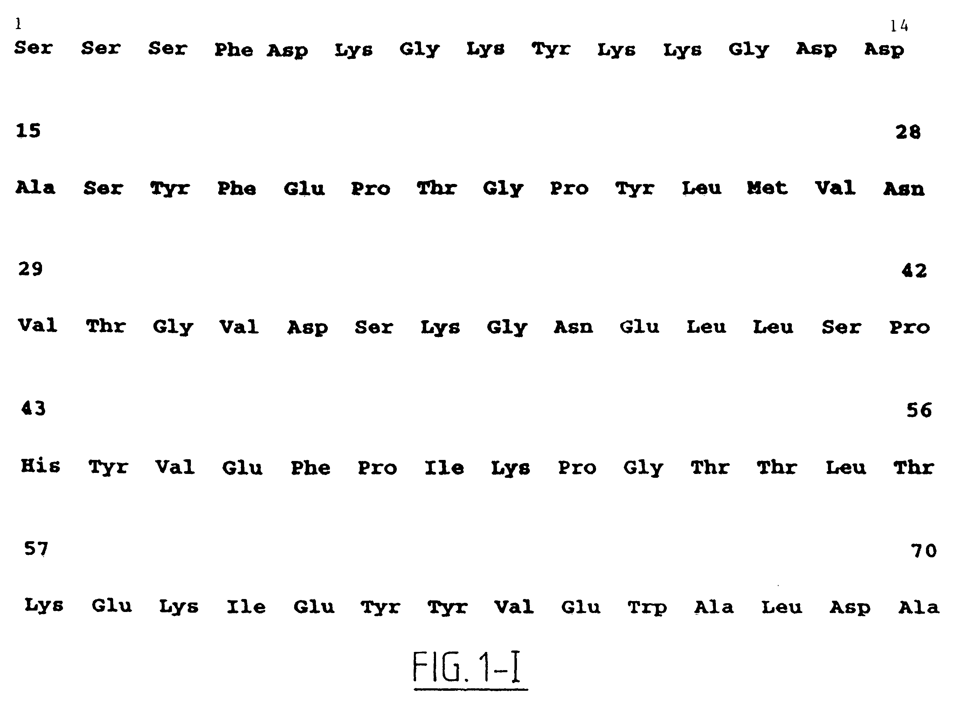

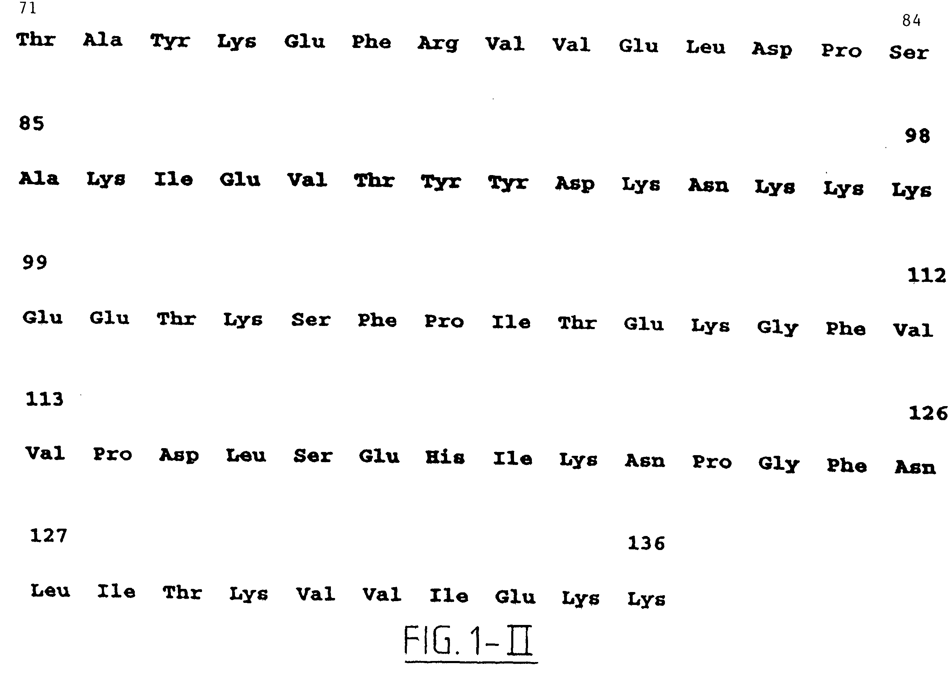

Staphylokinase, a protein produced by certain strains of Staphylococcus aureus, which was shown to have profibrinolytic properties more than 4 decades ago (1, 2) appears to constitute a potent thrombolytic agent in patients with acute myocardial infarction (3, 4). The staphylokinase gene has been cloned from the bacteriophages sakφC (5) and sak42D (6) as well as from the genomic DNA (sakSTAR) of a lysogenic Staphylococcus aureus strain (7). The staphylokinase gene encodes a protein of 163 amino acids, with amino acid 28 corresponding to the NH₂-terminal residue of full length mature staphylokinase (6, 8, 9). The mature protein sequence of the wild-type variant SakSTAR (9) is represented in Figure 1. only four nucleotide differences were found in the coding regions of the sakφC, sak42D and sakSTAR genes, one of which constituted a silent mutation (6, 8, 9). In a plasma milieu, staphylokinase is able to dissolve fibrin clots without associated fibrinogen degradation (10-12). This fibrin-specificity of staphylokinase is the result of reduced inhibition by α₂-antiplasmin of plasmin.staphylokinase complex bound to fibrin, recycling of staphylokinase from the plasmin.staphylokinase complex following inhibition by α₂-antiplasmin, and prevention of the conversion of circulating plasminogen.staphylokinase to plasmin.staphylokinase by α₂-antiplasmin (13-15). In addition staphylokinase has a weak affinity for circulating but a high affinity for fibrin-bound plasminogen (16) and staphylokinase requires NH₂-terminal processing by plasmin to display its plasminogen activating potential (17). In several experimental animal models, staphylokinase appears to be equipotent to streptokinase for the dissolution of whole blood or plasma clots, but significantly more potent for the dissolution of platelet-rich or retracted thrombi (18, 19). Staphylokinase is a heterologous protein and is immunogenic in man. The intrinsic immunogenicity of staphylokinase, like that of streptokinase, clearly hampers its unrestricted use. Not only will patients with preexisting high antibody titers be refractory to the thrombolytic effect of these agents, but allergic side effects and occasional life-threatening anaphylaxis may occur (20). Because both streptokinase and staphylokinase are heterologous proteins, it is not obvious that their immunogenicity could be reduced by protein engineering. Indeed, no successful attempts to generate active low molecular weight fragments from streptokinase have been reported. In staphylokinase, deletion of the NH₂-terminal 17 amino acids or the COOH-terminal 2 amino acids inactivates the molecule, which in addition is very sensitive to inactivation by site-specific mutagenesis (21).

It is therefore the object of the present invention to provide less immunogenic variants of staphylokinase having preferably a higher specific activity and/or lower plasma clearance and/or increased thrombolytic potency.

In the research that ultimately led to the present invention it was already found that the wild-type staphylokinase variant SakSTAR (9) contains three non-overlapping immunodominant epitopes, at least two of which can be eliminated by specific site-directed mutagenesis, without inactivation of the molecule. This has been disclosed in EP-95200023.0 (22). These engineered staphylokinase variants are less reactive with antibodies elicited in patients treated with wild-type staphylokinase, and are significantly less immunogenic than wild-type staphylokinase, as demonstrated in rabbit and baboon models and in patients with peripheral arterial occlusion (22).

Disclosed are general methods for the identification, production and use of staphylokinase derivatives showing a reduced antigenicity and immunogenicity as compared to wild-type staphylokinase as well as for variants with selective derivatization with polyethylene glycol. The derivatives preferably have a higher specific activity and/or lower plasma clearance and/or increased thrombolytic potency. The derivatives have essentially the amino acid sequence of wild-type staphylokinase or modified versions thereof and essentially intact biological activities, but have a reduced reactivity with a panel of murine monoclonal antibodies and/or with antibodies induced in patients by treatment with wild-type SakSTAR. The polyethylene glycol substituted ("pegylated") variants have reduced plasma clearances rendering them particularly suited for use by single intravenous bolus administration. Instead of PEG other pharmaceutically acceptable macromolecules can be used.

More in particular, disclosed are staphylokinase derivatives SakSTAR(K35X,G36X,E65X,K74X, E80X,D82X,K102X,E108X,K109X,K121X,K130X, K135X,K136X, +137X) having the amino acid sequence as depicted in figure 1 in which the amino acids Lys in position 35, Gly in position 36, Glu in position 65, Lys in position 74, Glu in position 80, Asp in position 82, Lys in position 102, Glu in position 108, Lys in position 109, Lys in position 121, Lys in position 130, Lys in position 135 and/or Lys in position 136 have been replaced with other amino acids and/or in which one amino acid has been added at the COOH-terminus, thus altering the immunogenicity after administration in patients, without markedly reducing the specific activity.

Disclosed are staphylokinase derivatives listed in Tables 1, 3, 4, 5, 6, 7, 8, 13, 19 and 20, having the amino acid sequence as depicted in figure 1 in which the indicated amino acids have been replaced by other amino acids thus reducing the absorption of SakSTAR-specific antibodies from plasma of patients treated with staphylokinase, without reducing the specific activity.

Derivatives in which the specific activity is increased and the immunogenicity is decreased are the following:
SakSTAR(K74A,E75A,R77A), SakSTAR(K35A,E75A), SakSTAR(E75A), SakSTAR(E80A,D82A), SakSTAR(E80A), SakSTAR(D82A), SakSTAR(E75A,D82A), SakSTAR(S34G,G36R, H43R), SakSTAR(K35A), SakSTAR(E80A), SakSTAR(D82A,S84A), SakSTAR(T90A), SakSTAR(Y92A), SakSTAR(K130A), SakSTAR(V132A), SakSTAR(S34G,G36R,H43R), SakSTAR(G36R), SakSTAR(H43R), SakSTAR(G36R,K74R), SakSTAR(K35E), SakSTAR(K74Q), SakSTAR(K130T), SakSTAR(V132L), SakSTAR(V132T), SakSTAR(V132N), SakSTAR(V132R), SakSTAR(K130T,K135R), SakSTAR(G36R,K130T,K135R), SakSTAR(K74R,K130T,K135R), SakSTAR(K74Q,K130T,K135R), SakSTAR(G36R,K74R,K130T,
K135R), SakSTAR(G36R,K74Q,K130T,K135R), SakSTAR(G36R, H43R,K74R,K130T,K135R), SakSTAR(E65A,K74Q,K130T,K135R), SakSTAR(E65Q,K74Q,K130T,K135R), SakSTAR(K74Q,K86A, K130T,K135R), SakSTAR(E65Q,T71S,K74Q,K130T,K135R), SakSTAR(K74Q,K130A,K135R), SakSTAR(E65Q,K74Q,K130A, K135R), SakSTAR(K74Q,K130E,K135R), SakSTAR(K74Q,K130E, V132R,K135R), SakSTAR(E65Q,K74Q,T90A,K130A,K135R), SakSTAR(E65Q,K74Q,N95A,K130A,K135R), SakSTAR(E65Q,K74Q, E118A,K130A,K135R), SakSTAR(E65Q,K74Q,N95A,E118A,K130A, K135R), SakSTAR(N95A,K130A,K135R), SakSTAR(E65Q,K74Q, K109A,K130,K135R), SakSTAR(E65Q,K74Q,E108A,K109A, K130T,K135R), SakSTAR(E65Q,K74Q,K121A,K130T,K135R), SakSTAR(E65Q,K74Q,N95A,E118A,K130A,K135R,K136A,+137K), SakSTAR(E80A,D82A,K130T,K135R), SakSTAR(K74R,E80A,D82A, K130T,K135R), SakSTAR(K74Q,E80A,D82A,K130T,K135R), SakSTAR(K35A,K74R,E80A,D82A,K130T,K135R), SakSTAR(E65D, K74R,E80A,D82A,K130T,K135R), SakSTAR(E65S,K74R,E80A, D82A,K130T,K135R), SakSTAR(S34G,G36R,K74R,K130T,K135R), SakSTAR(E65A,K74R,E80A,D82A,K130T,K135R), SakSTAR(E65N, K74R,E80A,D82A,K130T,K135R), SakSTAR(E65Q,K74R,E80A, D82A,K130T,K135R), SakSTAR(K57A,E58A,E61A,E80A,D82A, K130T,K135R), SakSTAR(E65D,K74Q,E80A,D82A,K130T,K135R), SakSTAR(E65Q,K74Q,E80A,D82A,K130T,K135R), SakSTAR(K35A, E65D,K74Q,E80A,D82A,K130T,K135R), SakSTAR(K74R,E80A,D82A, S103A,K130T,K135R), SakSTAR(E65D,K74R,E80A,D82A,K109A, K130T,K135R), SakSTAR(E65D,K74R,E80A,D82A,K130T, K135R,K136A), SakSTAR(E65Q,K74Q,D82A,S84A,K130T,K135R), SakSTAR(K35A,K74Q,E80A,D82A,K130T,K135R), SakSTAR(K35A, E65D,K74R,E80A,D82A,K130T,K135R).

Of these SakSTAR(E65D,K74R,E80A,D82A,K130T, K135R) having the code SY19 and SakSTAR(K35A,E65Q,K74R, E80A,D82A,T90A,E99D,T101S,E108A,K109A,K130T,K135R) having the code SY161 are especially preferred.

Besides the above described substitution derivatives the invention relates to derivatives having in addition an amino acid substituted with Cys. This type of substitution may result in dimerization and/or increased specific activity and/or reduced clearance and/or increased thrombolytic potency. Reduced plasma clearance is in particular obtained when the derivative is substituted with polyethylene glycol.

Preferred embodiments of such staphylokinase derivatives are those wherein the Cys is chemically modified with polyethylene glycol with molecular weights up to 20 kDa. In particular embodiments selected amino acids in the NH₂-terminal region of 10 amino acids, are substituted with Cys, which is chemically modified with polyethylene glycol with molecular weights up to 20 kDa. These derivatives are characterized by a significantly reduced plasma clearance and maintained thrombolytic potency upon single intravenous bolus administration at a reduced dose.

More in particular the serine in position 2 or 3 is substituted with a cystein and the cystein is chemically'modified with polyethylene glycol having a molecular weight of 5, 10 or 20 kDa. Preferred embodiments of these derivatives are SY161(S3C-MP5), SY161(S3C-P10), SY161(S3C-P20), SY19(S3C-MP5), SY19(S3C-SP5), SY19(S2C-SP5,S3C-SP5), SY19(S3C-P20), SY19(S3C-P10) all as defined in table 20.

The presence of cysteins allows the formation of dimers of two staphylokinase derivatives of the invention.

Disclosed is also a method for producing the derivatives of the invention by preparing a DNA fragment comprising at least the part of the coding sequence of staphylokinase that provides for its biological activity; performing in vitro site-directed mutagenesis on the DNA fragment to replace one or more codons for wild-type amino acids by a codon for another amino acid; cloning the mutated DNA fragment in a suitable vector; transforming or transfecting a suitable host cell with the vector; culturing the host cell under conditions suitable for expressing the DNA fragment; purifying the expressed staphylokinase derivative to homogeneity and derivatizing the variant with polyethylene glycol.

Preferably the DNA fragment is a 453 bp EcoRI-HindIII fragment of the plasmid pMEX602sakB (22, 23), the in vitro site-directed mutagenesis is preferably performed by spliced overlap extension polymerase chain reaction. Such overlap extension PCR is preferably performed with Vent DNA polymerase (New England Biolabs) or Taq polymerase (Boehringer Mannheim) and with available or generated wildtype sakSTAR or sakSTAR variants as template (24).

The invention also relates to pharmaceutical compositions comprising at least one of the staphylokinase derivatives according to the invention together with a suitable excipient, for treatment of arterial thrombosis. Pharmaceutical compositions, containing less immunogenic staphylokinase variants or "pegylated" staphylokinase variants as the active ingredient, for treating arterial thrombosis in human or veterinary practice may take the form of powders or solutions and may be used for intravenous, intraarterial or parenteral administration. Such compositions may be prepared by combining (e.g. mixing, dissolving etc.) the active compound with pharmaceutically acceptable excipients of neutral character (such as aqueous or non-aqueous solvents, stabilizers, emulsifiers, detergents, additives), and further, if necessary with dyes.

Furthermore the invention relates to the use of the staphylokinase derivatives for the treatment of arterial thrombosis, in particular myocardial infarction, and to the use of staphylokinase derivatives for the preparation of a pharmaceutical composition for the treatment of arterial thrombosis, in particular myocardial infarction. In the above and the following the terms "derivatives", "mutants" and "variants" are used interchangeably.

The present invention will be demonstrated in more detail in the following examples. In the Examples reference is made to the following figures:
**Fig 1.** Protein sequence of wild-type staphylokinase, SakSTAR. Numbering starts with the NH2-terminal amino acid of mature full length staphylokinase.
**Fig 2.** Time course of neutralizing activities (left panel) and specific IgG against administered agent (right panel) following intra-arterial infusion of SakSTAR (open circles, n= 9), SakSTAR(K74A) (closed circles, n= 11) or SakSTAR(K74A,E75A, R77A) (open squares, n= 6) in patients with peripheral arterial occlusion. The data represent median values and interquartile ranges, in µg/mL.
**Fig 3.** Protein sequence of wild-type staphylokinase, SakSTAR with indicated amino acid substititutions.
   squares: single amino acid substitutions;
   circles: combined (2 to 3) amino acid to Ala substitutions.
**Fig. 4.** Temperature stability of SakSTAR, (A); SakSTAR(K74Q,E80A,D82A,K130T, K135R) (B); SakSTAR(E65D,K74R,E80A,D82A,K130T,K135R), (C); and SakSTAR(K35A,E65D,K74Q,E80A,D82A, K130T,K135R), (D). (O): 4°C: (•): 20°C; (∇): 37°C; (▼): 56°C; (□): 70°C.
**Fig 5.** Time course of neutralizing activities (left panel) and specific IgG against administered agent (right panel) following intra-arterial infusion of SakSTAR (circles, n= 6), SakSTAR(K74Q,E80A,D82A,K130T,K135R) (squares, n= 6) or SakSTAR(E65D,K74R,E80A,D82A,K130T,K135R) (triangles, n= 6) in patients with peripheral arterial occlusion. The data represent median values and 15-85 percentile ranges, in µg/mL.

### EXAMPLES

### EXAMPLE 1

### Epitope mapping of wild-type staphylokinase

The epitope specificity of a panel of 15 murine MAbs (22) raised against wild-type SakSTAR was determined by real-time biospecific interaction analysis (BIA) with the BIAcore instrument (Pharmacia, Biosensor AB, Uppsala, Sweden). The MAbs were immobilized on the surface of the Sencor Chip CM5 with the Amine Coupling Kit (Pharmacia Biosensor AB) as recommended by the manufacturer (25). Immobilization was performed from protein solutions at a concentration of 20 µg/mL in 10 mmol/L sodium acetate at pH 5.0 at a flow rate of 5 µL/min during 6 minutes. This resulted in covalent attachment of 5,000 to 10,000 resonance unit (RU) of antibody (corresponding to 0.035 to 0.07 pmol/mm²). The SakSTAR solutions were passed by continuous flow at 20°C past the sensor surface. At least four concentrations of each analyte (range, 50 nmol/L to 50 mol/L) in 10 mmol/L HEPES, 3.4 mmol/L EDTA, 0.15 mol/L NaCl, and 0.005% Surfactant P20, pH 7.2, were injected at a flow rate of 5 uL/min during 6 minutes in the association phase. Then sample was replaced by buffer, also at a flow rate of 5 µL/min during 6 minutes. After each cycle, the surface of the sensor chip was regenerated by injection of 5 µL of 15 mmol/L HCl. Apparent association (kₐₛₛ) and apparent dissociation (k_{diss}) rate constants were derived from the sensorgrams as described in detail elsewhere (26), and association equilibrium constants (K_{A}) calculated as their ratio.

Determination of the equilibrium association constants for the binding of wild-type and variant SakSTAR to insolubilized MAbs (Table 1) yielded apparent association constants of 10⁷ to 10⁸ (mol/L)⁻¹, which are one to two orders of magnitude lower than the apparent association constants previously obtained for the binding of these MAbs to insolubilized wild-type SakSTAR (22). If the MAbs instead of the SakSTAR variants are insolubilized, avidity effects of the bivalent MAbs are avoided. The present values are indeed in better agreement with known association constants of Mabs, and therefore this "reversed" procedure was used throughout the present invention.

In the tables the column indicated with "Variant" states the various staphylokinase derivatives which are identified by listing between brackets the substituted amino acids in single letter symbols followed by their position number in the mature staphylokinase sequence and by the substituting amino acids in single letter symbol; the column "Exp." indicates expression levels in mg/L, and the column "Spec. Act." indicates the specific activity in Home Units as defined in example 2. Indications "17G11", "26A2" etc. refer to monoclonal antibodies binding to the indicated epitopes I, II and III as defined in reference 22. Epitope I is recognized by the antibody cluster 17G11, 26A2, 30A2, 2B12 and 3G10, whereas epitope II is recognized by the antibody cluster 18F12, 14H5, 28H4, 32B2 and 7F10, and epitope III by the antibody cluster 7H11, 25E1, 40C8, 24C4 and 1A10. Human plasma "Pool" indicates a plasma pool from initially 16 and subsequently 10 patients immunized by treatment with SakSTAR, "Subpool B" indicates a plasma pool from three patients that absorbed less than 50% of the induced antibodies with SakSTAR(K35A,E38A,K74A,E75A,R77A) and "Subpool C" indicates a plasma pool from 3 patients that absorbed >90% of the induced antibodies with SakSTAR(K35A,E38A,K74A,E75A,R77A) (22).

In tables 6, 7 and 8 an additional pool of plasma from 40 patients immunized by treatment with SakSTAR (Pool 40) was also used.

### EXAMPLE 2

### Construction, epitope mapping with murine monoclonal antibodies and absorption with pooled plasma of immunized patients, of "alanine-to-wild-type" reversal variants of "charged-cluster-to-alanine" mutants of staphylokinase

### 1. Introduction

As stated above, wild-type staphylokinase (SakSTAR variant (9)) contains three non-overlapping immunodominant epitopes, two of which can be eliminated by specific site-directed substitution of clusters of two (K35A,E38A or E80A,D82A) or three (K74A,E75A,R77A) charged amino acids with Ala (22). The combination mutants SakSTAR(K35A,E38A,K74A,E75A,R77A) in which Lys35, Glu38, Lys74, Glu75 and Arg77, and SakSTAR(K74A,E75A, R77A,E80A,D82A) in which Lys74, Glu75, Arg77, Glu80 and Asp82 were substituted with Ala (previously identified as SakSTAR.M3.8 and SakSTAR.M8.9, respectively (22)), were found to have a reduced reactivity with murine monoclonal antibodies against two of the three immunodominant epitopes and to absorb on average only 2/3 of the neutralizing antibodies elicited in 16 patients by treatment with wild-type SakSTAR (22). These mutants also induced less antibody formation than wild-type SakSTAR in experimental thrombolysis models in rabbits and baboons, and in patients with peripheral arterial occlusion (22). However, their specific activities were reduced to approximately 50% of that of wild-type SakSTAR, which would be of some concern with respect to the clinical use of these compounds.

In an effort to improve the activity and stability without loss of the reduced antibody recognition, the effect of a systematic reversal of one or more of these substituted amino acids to the wild-type residues was studied. Fourteen new mutants were constructed, purified and characterized in terms of specific activity, reactivity with the panel of murine monoclonal antibodies, and absorption of antibodies from plasma of patients treated with wild-type SakSTAR (Table 1). The present example thus focusses on reversal from alanine to the wild-type residue of one or more of the seven amino acids of SakSTAR listed above i.e. K35, E38, K74, E75, R77, E80 and D82.

### 2. Reagents and Methods

The source of all reagents used in the present study has previously been reported (22). Restriction enzymes were purchased from Pharmacia (Uppsala, Sweden) or Boehringer Mannheim (Mannheim, Germany). T4 DNA ligase, Klenow Fragment of E. coli DNA polymerase I and alkaline phosphatase were obtained from Boehringer Mannheim. Enzyme reactions were performed using the conditions suggested by the suppliers. Plasmid DNA was isolated using a QIAGEN-purification protocol (provided by Westburg, Leusden, The Netherlands). pMEX.602sakB (i.e. pMEX.SakSTAR) was constructed as described elsewhere (23). SakSTAR, SakSTAR(K35A,E38A), SakSTAR(K74A,E75A,R77A), SakSTAR(E80A,D82A), SakSTAR(K35A,E38A,K74A,E75A,R77A) and Sak-STAR(K74A,E75A,R77A,E80A,D82A) were produced and purified as described elsewhere (22). Transformations of E. coli were performed utilizing the calcium phosphate procedure. DNA sequencing was performed using the dideoxy chain termination reaction method and the Automated Laser fluorescent A.L.F.™ (Pharmacia). The chromogenic substrate (S2403) L-Pyroglutamyl-L-phenylalanyl-L-lysine-p-nitroanaline hydrochloride was purchased from Chromogenix (Belgium). ¹²⁵I-labeled fibrinogen was purchased from Amersham (UK). All other methods used in the present example have been previously described (22,27) .

### 3. Construction of expression plasmids

The plasmids encoding SakSTAR(K35A,E38A,K74A, E75A), SakSTAR(E38A,E75A,R77A), SakSTAR(E38A,E75A), SakSTAR(K35A,E75A,R77A), SakSTAR(K35A,E75A), SakSTAR(E80A), SakSTAR(D82A), SakSTAR(E75A,D82A), Sak-STAR(K74A) and SakSTAR(E75A) were constructed by the spliced overlap extension polymerase chain reaction (SOE-PCR) (24), using Vent DNA polymerase (New England Biolabs, Leusden, The Netherlands), and available or generated sakSTAR variants as template. Two fragments were amplified by PCR, the first one starting from the 5' end of the staphylokinase gene with primer 5'-CAGGAAACAGAATTCAGGAG-3' to the region to be mutagenized (forward primer), the second one from the same region (backward primer) to the 3' end of the staphylokinase gene with primer 5'-CAAAACAGCCAAGCTTCATTCATTCAGC-3'. The forward and backward primers shared an overlap of around 24 bp (primers not shown). The two purified fragments were then assembled together in a new primerless PCR using Taq polymerase (Boehringer Mannheim). After 7 cycles (1 min at 94°C, 1 min at 70°C), the extended product was reamplified by adding the 5' and 3' end primers (see above) to the PCR reaction and by cycling 25 times (1 min at 94°C, 1 min 55°C, 1 min at 72°C). The final product was purified, digested with EcoRI and HindIII and cloned into the corresponding sites of pMEX602sakB. The plasmid encoding SakSTAR(E38A,K74A,E75A,R77A) was assembled by digestion of pMEX602sakB and pMEX.SakSTAR(K35A,E38A, K74A,E75A,R77A) with BpmI which cuts between the codons for K35 and E38 of SakSTAR, and ligation of the required fragments. The plasmid encoding SakSTAR(K35A,K74A,E75A, R77A) was constructed by digestion of pMEX.SakSTAR(K35A, E38A,K74A,E75A,R77A) and pMEX.SakSTAR(K74A,E75A,R77A) with BpmI and religation of the required fragments. The plasmids encoding SakSTAR(K35A,E38A, E75A,R77A) and SakSTAR(K35A,E38A,K74A,R77A) were constructed by two PCR using pMEX.SakSTAR(K35A,E38A,K74A,E75A,R77A) as template, followed by restriction ligation and recloning into pMEX602sakB.

### 4. Expression and purification of SakSTAR variants

The SakSTAR variants were expressed and purified, as described below, from transformed E. coli WK6 grown either in LB medium [SakSTAR(E38A,K74A,E75A, R77A), SakSTAR(K74A), SakSTAR(E75A) and SakSTAR(E75A, D82A)], or in terrific broth (TB) (28) medium [SakSTAR(K35A,K74A,E75A,R77A), SakSTAR(K35A,E38A,E75A, R77A), SakSTAR(K35A,E38A,K74A,R77A), SakSTAR(K35A, E38A,E75A), SakSTAR(E38A,E75A, R77A), SakSTAR(E38A,E75A), SakSTAR(K35A,E75A,R77A), SakSTAR(K35A,E75A), SakSTAR(E80A), and SakSTAR(D82A)].

For derivatives produced in LB medium, a 20 mL aliquot of an overnight saturated culture was used to inoculate a 2 L volume of LB medium containing 100 g/mL ampicillin. After 3 hours incubation at 37°C, IPTG (200 mol/L) was added to induce expression from the tac promoter. The production phase was allowed to proceed for 4 hours, after which the cells were pelleted by centrifugation at 4,000 rpm for 20 min, resuspended in 1/20 volume (100 mL) of 0.01 mol/L phosphate buffer pH 6.5 and disrupted by sonication at 0°C. Cell debris were removed by centrifugation for 20 min at 20,000 rpm and the supernatant, containing the cytosolic soluble protein fraction, was stored at -20°C until purification.

For the derivatives produced in TB medium, a 4 mL aliquot of an overnight saturated culture in LB medium was used to inoculate a 2 L culture in terrific broth containing 100 µg/mL ampicillin. The culture was grown with vigorous aeration for 20 hours at 30°C. The cells were pelleted by centrifugation, resuspended in 1/10 volume (200 mL) of 0.01 mol/L phosphate buffer pH 6.5 and disrupted by sonication at 0°C. The suspension was then centrifuged for 20 min at 20,000 rpm and the supernatant was stored at -20°C until purification. Cleared cell lysates containing the SakSTAR variants were subjected to chromatography on a 1.6 x 6 cm column of SP-Sephadex, followed by chromatography on a 1.6 x 5 cm column of Q-Sepharose [variants SakSTAR(E38A,K74A,E75A,R77A), SakSTAR(K35A,K74A,E75A, R77A), SakSTAR(K35A,E38A, E75A,R77A), SakSTAR(K35A,E38A,K74A,R77A) and SakSTAR(K35A, E38A,K74A,E75A)] or by chromatography on a 1.6 x 6 cm column of phenyl-Sepharose [variants SakSTAR(E35A,E38A,R77A), SakSTAR(E38A,E75A), SakSTAR(K35A,E75A,R77A), SakSTAR(K35A,E75A), SakSTAR(K74A), SakSTAR(E75A), SakSTAR(E80A), SakSTAR(D82A) and SakSTAR(E75A,D82A)]. The SakSTAR containing fractions, localized by SDS-gel electrophoresis, were pooled for further analysis.

### 5. Physicochemical and biochemical analysis

Protein concentrations were determined according to Bradford (29). The specific activities of SakSTAR solutions were determined with a chromogenic substrate assay carried out in microtiter plates using a mixture of 80 µL SakSTAR solution and 100 µL Glu-plasminogen solution prepared as described elsewhere (30) (final concentration 0.5 µmol/L). After incubation for 30 min at 37°C, generated plasmin was quantitated by addition of 20 µL S2403 (final concentration 1 mmol/L) and measurement of the absorption at 405 nm. The activity was expressed in home units (HU) by comparison with an in-house standard (lot STAN5) which was assigned an activity of 100,000 HU (100 kHU) per mg protein as determined by amino acid composition (7). SDS-PAGE was performed with the Phast System™ (Pharmacia, Uppsala, Sweden) using 10-15% gradient gels and Coomassie Brilliant blue staining. Reduction of the samples was performed by heating at 100°C for 3 min in the presence of 1% SDS and 1% dithiothreitol. The specific activities of the different SakSTAR mutants determined with the chromogenic substrate assay are summarized in Table 1.

### 6. Binding to murine monoclonal antibodies

In agreement with previous observations (22), SakSTAR(K74A,E75A,R77A) did not react with 4 of the 5 MAbs recognizing epitope I, whereas SakSTAR(K35A,E38A) did not react with 3 of the 5 and SakSTAR(E80A,D82A) not with 4 of the 5 Mabs recognizing epitope III. These reduced reactivities were additive in SakSTAR(K35A,E38A, K74A,E75A,R77A) and in SakSTAR(K74A,E75A,R77A,E80A,D82A). The reduced reactivity of SakSTAR(K74A,E75A, R77A) was fully maintained in SakSTAR(K35A,E38A,K74A,E75A) and in SakSTAR(K35A, E75A,R77A), largely in SakSTAR(K35A,E38A, E75A,R77A), SakSTAR(E38A,E75A,R77A), SakSTAR(E38A,E75A) and SakSTAR(E75A), but much less in SakSTAR(K35A,E38A, K74A,R77A) and SakSTAR(K74A), indicating that E75 is the main contributor to the binding of the 4 Mabs recognizing epitope I of SakSTAR. However, surprisingly, binding of epitope I antibodies to SakSTAR(E75A,D82A) was normal in two independent preparations from expression plasmids with confirmed DNA sequences. The reduced reactivity of the 3 MAbs of epitope III with SakSTAR(K35A,E38A) required both K35 and E38, as demonstrated with SakSTAR(E38A,K74A,E75A,R77A) and SakSTAR(K35A,K74A,E75A, R77A), with SakSTAR(E38A,E75A) and SakSTAR(K35A,E75A) and with SakSTAR(E38A,E75A,R77A) and SakSTAR(K35A,E75A,R77A) . The reduced reactivity of the 4 MAbs of cluster III with SakSTAR(E80A,D82A) was maintained in SakSTAR(D82A) but not in SakSTAR(E80A).

### 7. Absorption of antibodies, elicited in patients by treatment with wild-type SakSTAR

Plasma samples from 16 patients with acute myocardial infarction, obtained several weeks after treatment with SakSTAR (4, 31) were used. The staphylokinase-neutralizing activity in these samples was determined as follows. Increasing concentrations of wild-type or variant SakSTAR (50 µL volumes containing 0.2 to 1000 µg/mL) were added to a mixture of 300 µL citrated human plasma and 50 µL buffer or test plasma, immediately followed by addition of 100 µL of a mixture containing thrombin (50 NIH units/mL) and CaCl₂ (25 mmol/L). The plasma clot lysis time was measured and plotted against the concentration of SakSTAR moiety. From this curve the concentration of staphylokinase moiety that produced complete clot lysis in 20 min was determined. The neutralizing activity titer was determined as the difference between the test plasma and buffer values and was expressed in µg per mL test plasma. The results of the individual patients have been reported elsewhere (22). For the present invention, three plasma pools were made, one from 10 patients from whom sufficient residual plasma was available, one from three patients that absorbed less than 50% of the antibodies with SakSTAR(K35A,E38A, K74A,E75A,R77A) (Subpool B) and one from three patients that absorbed >90% of the antibodies with SakSTAR(K35A,E38A,K74A,E75A, R77A) (Subpool C). These plasma pools were diluted (1/30 to 1/200) until their binding to SakSTAR substituted chips in the BIAcore instrument amounted to approximately 2000 RU. From this dilution a calibration curve for antibody binding was constructed using further serial two-fold dilutions. The plasma pools were absorbed for 10 min with 100 nmol/L of the SakSTAR variants, and residual binding to immobilized SakSTAR was determined. Residual binding was expressed in percent of unabsorbed plasma, using the calibration curve.

The results are summarized in Table 1. Whereas wild-type SakSTAR absorbed more than 95% of the binding antibodies from pooled plasma of the 10 patients, incomplete absorption (<60%) was observed with SakSTAR(K74A,E75A,R77A), SakSTAR(K35A,E38A,K74A, E75A,R77A), SakSTAR(E38A,K74A,E75A,R77A), SakSTAR(K35A,K74A,E75A,R77A), SakSTAR(K35A, E38A,K74A,R77A), SakSTAR(K35A,E38A,K74A,E75A), SakSTAR(K74A) and SakSTAR(K74A,E75A,R77A,E80A,D82A) but absorption was nearly complete with SakSTAR(K35A,E38A), SakSTAR(K35A,E38A,E75A,R77A), SakSTAR(E38A,E75A,R77A), SakSTAR(E38A,E75A), SakSTAR(K35A,E75A,R77A), SakSTAR(K35A,E75A), SakSTAR(E75A), SakSTAR(E80A,D82A), SakSTAR(E80A), SakSTAR(D82A) and SakSTAR(E75A,D82A). These results, surprisingly, demonstrate that approximately 40% of the antibodies elicited in patients by treatment with wild-type SakSTAR depend on K74 for their binding (Table 1). Absorption with pooled plasma from 3 patients from which <50% of the antibodies were absorbed with SakSTAR(K35A,E38A,K74A,E75A,R77A) (Subpool B) confirmed the predominant role of K74 for antibody recognition. As expected, absorption with pooled plasma from 3 patients from which >95% of the antibodies were absorbed with SakSTAR(K35A,E38A,K74A,E75A,R77A) (Subpool C) was nearly complete with all variants tested.

### EXAMPLE 3

### Comparative thrombolytic efficacy and immunogenicity of SakSTAR(K74A,E75A,R77A) and SakSTAR(K74A) versus SakSTAR in patients with peripheral arterial occlusion

### 1. Purification of SakSTAR(K74A,E75A,R77A) and SakSTAR(K74A) for use in vivo

A 12 to 24 L culture (in 2 L batches) of the variants SakSTAR(K74A,E75A,R77A), or of SakSTAR(K74A) was grown and IPTG-induced in LB medium supplemented with 100 µg/mL ampicillin, pelleted, resuspended, disrupted by sonication and cleared as described above. The compounds were purified by chromatography on a 5 x 20 cm column of SP-Sephadex, a 5 x 10 cm column of Q-Sepharose and/or a 5 x 13 cm column of phenyl-Sepharose using buffer systems described elsewhere (22, 23). The materials were then gel filtered on sterilized Superdex 75 to further reduce their endotoxin content. The SakSTAR variant containing fractions were pooled, the protein concentration was adjusted to 1 mg/mL and the material sterilized by filtration through a 0.22 µm Millipore filter. The methodology used to determine the biological properties of the final material required for use in vivo is described above and elsewhere (22).

### 2. Materials and Methods

Staphylokinase-neutralizing activity in plasma was determined as described above. Quantitation of antigen-specific IgG and IgM antibodies was performed using enzyme-linked immunosorbent assays in polystyrene microtiter plates essentialy as described previously (22). In the IgG assays, dilution curves of affinospecific anti-SakSTAR IgG antibodies were included on each plate. These antibodies were isolated from plasma obtained from 3 patients, after thrombolytic therapy with wild-type SakSTAR, by chromatography on protein A-Sepharose and on insolubilized SakSTAR, and elution of bound antibodies with 0.1 mol/L glycine-HCl, pH 2.8. The purity of the IgG preparation was confirmed by sodium dodecylsulfate polyacrylamide gel electrophoresis. In the IgM assays, titers defined as the plasma dilution giving an absorbancy at 492 nm equivalent to that of a 1/640 dilution of pooled plasma were determined and compared with the titer of baseline samples before treatment (median value 1/410, interquartile range 1/120-1/700).

### 3. Thrombolytic efficacy

Wild-type SakSTAR or the variants SakSTAR(K74A) or SakSTAR(K74A,E75A,R77A) were administered intra-arterially at or in the proximal end of the occlusive thrombus as a bolus of 2 mg followed by an infusion of 1 mg/hr (reduced overnight in some patients to 0.5 mg/hr) in groups of 6 to 12 patients with angiographically documented occlusion of a peripheral artery or bypass graft of less than 120 days duration. Patients were studied after giving informed consent, and the protocol was approved by the Human Studies Committee of the University of Leuven. Inclusion and exclusion criteria, conjunctive antithrombotic treatment (including continuous intravenous heparin) and the study protocol were essentially as previously described (22).

Relevant baseline characteristics of the individual patients are shown in Table 2. The majority of PAO were at the femoropopliteal level. Two iliac stent and 8 graft occlusions were included. Eight patients presented with incapacitating claudication, 5 with chronic ischemic rest pain, 7 with subacute ischemia and 7 with acute ischemia. One patient (POE) who had 2 years previously been treated with SakSTAR was included in the SakSTAR(K74A) group. This patient was not included in the statistical analyses.

Table 2 also summarizes the individual treatment and outcome. Intra-arterial infusion, at a dose of 6.0 to 25 mg and a duration of 4.0 to 23 hrs, induced complete recanalization in 24 patients and partial recanalization in 3. Complementary endovascular procedures (mainly PTA) were performed in 17 patients and complementary reconstructive vascular surgery following thrombolysis in 3. No patient underwent major amputation. Early recurrence of thrombosis after the end of the angiographic procedure occurred in 4 patients. Bleeding complications were absent or limited to mild to moderate hematoma formation at the angiographic puncture sites except for 5 patients who required transfusion (data not shown). Intracranial or visceral hemorrhage was not observed. Circulating fibrinogen, plasminogen and α₂-antiplasmin levels remained essentially unchanged during infusion of the SakSTAR moieties (data not shown), confirming absolute fibrin specificity of staphylokinase at the dosages used. Significant in vivo fibrin digestion occurred as evidenced by elevation of fibrin fragment D-dimer levels. Intra-arterial heparin therapy prolonged aPTT levels to a variable extent (data not shown).

### 4. Antibody induction

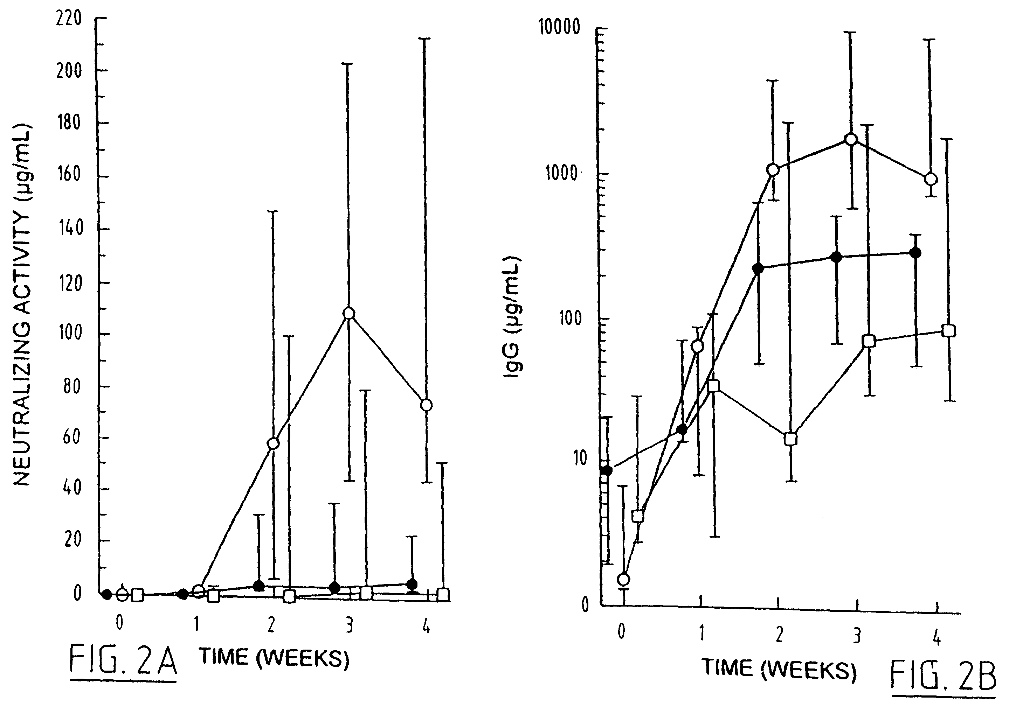

Antibody-related SakSTAR-, SakSTAR(K74A)- and SakSTAR(K74A,E75A,R77A)-neutralizing activity and anti-SakSTAR, anti-SakSTAR(K74A) and anti-SakSTAR(K74A, E75A,R77A) IgG, were low at baseline and during the first week after the infusion (Figure 2). From the second week on, neutralizing activity levels increased to reach median values at 3 to 4 weeks of 20 µg SakSTAR(K74A) and 2.4 µg SakSTAR(K74A,E75A,R77A) neutralized per mL plasma in the patients treated with SakSTAR(K74A) and SakSTAR(K74A,E75A,R77A), respectively, which is significantly lower than the median value of 93 µg wild-type SakSTAR neutralized per mL in the patients treated with SakSTAR (p= 0.024 for differences between the three groups by Kruskal-Wallis analysis and p= 0.01 and p= 0.036, respectively, for variants vs wild-type by Mann-Whitney rank sum test). The levels of anti-SakSTAR(K74A) and of anti-SakSTAR(K74A,E75A,R77A) IgG increased to median values at 3 to 4 weeks of 270 and 82 µg/mL plasma in patients treated with SakSTAR(K74A) and SakSTAR(K74A,E75A,R77A) respectively, which is significantly lower than the median value of 1800 µg anti-SakSTAR per mL plasma in the patients treated with SakSTAR ((p= 0.024 for differences between the three groups by Kruskal-Wallis analysis and p= 0.007 and 0.05, respectively, for variants versus wild-type by Mann-Whitney rank sum test).

The titers of anti-SakSTAR(K74A) and of anti-SakSTAR(K74A,E75A,R77A) IgM increased from median baseline values of 1/460 and 1/410 to median values at 1 week of 1/510 and 1/450 in patients treated with SakSTAR(K74A) and SakSTAR(K74A,E75A,R77A), respectively, which was not significantly different from the median values of 1/320 at baseline and 1/640 at week 1 in patients treated with SakSTAR. Corresponding values at 2 weeks were 1/590 and 1/550 in patients given SakSTAR(K74A) and SakSTAR(K74A,E75A,R77A), not significantly different from 1/930 with SakSTAR (data not shown) . The antibodies induced by treatment with SakSTAR were completely absorbed by SakSTAR but incompletely by SakSTAR(K74A) and by SakSTAR(K74A,E75A,R77A) confirming the immunogenicity of the K74,E75,R77 epitope and the dominant role of K74 in the binding of antibodies directed against this epitope. The antibodies induced by treatment with SakSTAR(K74A) or SakSTAR(K74A,E75A,R77A) were completely absorbed by SakSTAR, by SakSTAR(K74A) and by SakSTAR(K74A,E75A,R77A), indicating that immunization was not due to neoepitopes generated by substitution of Lys74 with Ala, but to epitopes different from the K74,E75,R77 epitope.

Thus, this example illustrates that staphylokinase variants with reduced antibody induction but intact thrombolytic potency can be generated. The present experience in 26 patients treated with SakSTAR (n= 9), SakSTAR(K74A) (n= 11) and SakSTAR(K74A,E75A,R77A) (n= 6) combined with previous experience in 14 patients with SakSTAR (n= 7) and SakSTAR(K35A, E38A,K74A,E75A,R77A) (n= 7) (31) and in 24 patients with SakSTAR (32), and with subsequent non-randomized experience in patients with SakSTAR (n= 30) with SakSTAR(K74A) (n= 12) and with SakSTAR(K74A,E75A,R77A) (n= 7) (data not shown), allows an initial estimation of the prevalence of immunization by intra-arterial treatment with SakSTAR or variants with an altered K74,E75,R77 epitope [SakSTAR(K74A), SakSTAR(K74A, E75A,R77A) and SakSTAR(K35A,E38A,K74A,E75A, R77A)]. Neutralizing activity data after 2 to 4 weeks, available in 70 patients with peripheral arterial occlusion given intra-arterial SakSTAR, revealed that 56 patients (80 percent) had levels > 5 µg compound neutralized per mL plasma. Of the patients given SakSTAR(K74A), SakSTAR(K74A,E75A, R77A) or SakSTAR(K74A,E75A,K74A,E75A, R77A), 27 of the 43 (63 percent) had neutralizing activity levels of > 5 µg compound per mL plasma. This difference is statistically significant (p= 0.05 by Fisher's exact test) indicating that the K74,E75,R77 epitope is a major determinant of antibody induction.

### EXAMPLE 4

### Construction, epitope mapping with murine monoclonal antibodies and absorption with pooled plasma of immunized patients, of alanine-substitution mutants of staphylokinase

### 1. Introduction

Site-directed mutagenesis was applied to residues other than "charged amino acids" in order to identify i) additional residues belonging to epitopes I and III identified with the panel of murine Mabs and ii) amino acids determining absorption to antiserum from immunized patients. Since functional epitopes generally comprise more than one amino acid residue critical for antibody binding, identification of additional residues in these epitopes could lead to the construction of new combination derivatives displaying a lower antigenic profile, while keeping the specific activity and the temperature stability of wild-type staphylokinase. In this example, the construction and characterization of SakSTAR variants in which one or at most two amino acids (adjacent or in close vicinity) were substituted with alanine is described. The mutants described under this example are listed in Table 3. These variants were expressed in E. coli, purified and characterized in terms of specific activity, reactivity with the panel of murine monoclonal antibodies, and absorption of antibodies from plasma of patients treated with wild-type SakSTAR.

### 2. Reagents and Methods

The source of all reagents used in the present study has previously been reported (22), or is specified below. The template vector for mutagenesis, pMEX602sakB (i.e. pMEX.SakSTAR), has been described elsewhere (23). Restriction and modification enzymes were purchased from New England Biolabs (Leusden, The Netherlands), Boehringer Mannheim (Mannheim, Germany) or Pharmacia (Uppsala, Sweden). The enzymatic reactions were performed according to the supplier recommendation. The mutagenic oligonucleotides and primers were obtained from Eurogentec (Seraing, Belgium). Plasmid DNA was isolated using a purification kit from Qiagen (Hilden, Germany) or the BIO 101 RPM kit (Vista, CA), as recommended. Transformation-competent E. coli cells were prepared by the well-known calcium phosphate procedure. Nucleotide sequence determination was performed on double strand plasmid DNA with the dideoxy chain termination method, using the T7 sequencing kit (Pharmacia, Uppsala, Sweden). Polymerase chain reactions (PCR) were performed using Taq polymerase from Boehringer Mannheim (Mannheim, Germany) or Vent polymerase (New England Biolabs, Leusden, The Netherlands). The recombinant DNA methods required to construct the variants described in this example are well established (22, 27).

### 3. Construction of expression plasmids

The variants SakSTAR(Y17A,F18A), Sak-STAR(F104A), SakSTAR(F111A), SakSTAR(Y9A), SakSTAR(Y91A), SakSTAR(Y92A), SakSTAR(I87A), SakSTAR(I106A) and Sak-STAR(I120A) were constructed with the Chameleon Double-Stranded Site-Directed Mutagenesis kit from Stratagene (La Jolla, USA), using the pMEX.SakSTAR vector as template, and following instructions of the supplier. The mutagenic oligonucleotides (not shown) were used in combination with the selection-primer LY34 5' CAAAACAGCCGAGCTTCATTCATTCAGC, which destroys the unique HindIII site located 3' to the staphylokinase encoding gene in pMEX.SakSTAR and allows to counter-select the non-mutant progeny by HindIII digestion. The deletion of the HindIII site was in most cases correlated with the presence of the desired mutation introduced by the mutagenic oligonucleotide. The variant SakSTAR(I133A), was constructed by performing a polymerase chain reaction on the pMEX.SakSTAR plasmid using the primer 818A located at the 5' end of the sakSTAR gene (5' CAGGAAACAGAATTCAGGAG ) and the mutagenic primer LY58 (5' TTCAGCATGCTGCAGTTATTTCTTTTCTGCAACAACCTTGG). The amplified product (30 cycles: 30 sec at 94°C, 30 sec at 50°C, 30 sec at 72°C) was purified, digested with EcoRI and PstI, and ligated into the corresponding sites of pMEXSakSTAR. The variants SakSTAR(I128A), SakSTAR(L127A) and SakSTAR(N126V) were constructed by performing a polymerase chain reaction using the primer 818A located at the 5' end of the sakSTAR gene and mutagenic primers (not shown). The amplified product (30 cycles: 1 sec at 94°C, 1 sec at 50°C, 10 sec at 72°C) was purified, digested with EcoRI and StyI, and ligated into the corresponding sites of pMEXSakSTAR.

The variant SakSTAR(F125A) was constructed by performing two consecutive PCR reactions (30 cycles: : 30 sec at 94°C, 30 sec at 50°C, 30 sec at 72°C). In the first reaction, a fragment of pMEX.SakSTAR was amplified with the primers 818A and a mutagenic primer. This amplified fragment was then used as template in a second PCR reaction with a mutagenic primer in order to further elongate the fragment downstream of the StyI site present in the sakSTAR gene (corresponding to amino acids 130-131 of SakSTAR). The resulting product was digested with EcoRI and StyI, and ligated into the corresponding sites of pMEXSakSTAR.

The plasmids encoding all the other variants listed in Table 3 were constructed by direct PCR or by the spliced overlap extension polymerase chain reaction (SOE-PCR) (24) using pMEX.SakSTAR or available plasmids encoding SakSTAR variants as template. Two fragments were amplified by PCR (30 cycles: 1 sec at 94°C, 1 sec at 50°C, 10 sec at 72°C), the first one starting from the 5' end (primer 818A ) of the staphylokinase gene to the region to be mutagenized (forward primer), the second one from this same region (backward primer) to the 3' end of the gene with primer 818D (5' CAAACAGCCAAGCTTCATTCATTCAGC). The forward and backward primers shared an overlap of around 24 bp (primers not shown). The two purified fragments were then assembled together in a second PCR reaction with the external primers 818A and 818D (30 cycles: 1 sec at 94°C, 1 sec at 50°C, 10 sec at 72°C). The amplified product from this final reaction was purified, digested with EcoRI and HindIII and ligated into the corresponding site of pMEX.SakSTAR. For each construction, the sequence of the variant was confirmed by sequencing the entire SakSTAR coding region.

### 4. Expression and purification of SakSTAR variants

The SakSTAR variants were expressed and purified, as described below, from transformed E. coli grown in terrific broth (TB) medium (28). A 2 to 4 mL aliquot of an overnight saturated culture in LB medium was used to inoculate a 1 to 2 L culture in terrific broth supplemented with 100 µg/mL ampicillin. The culture was incubated with vigorous aeration and at 30°C. After about 16 hours incubation, IPTG (200 µmol/L) was added to the culture to induce expression from the tac promoter. After 3 hours induction, the cells were pelleted by centrifugation at 4,000 rpm for 20 min, resuspended in 1/10 volume of 0.01 mol/L phosphate buffer pH 6-6.5 and disrupted by sonication at 0°C. The suspension was centrifuged for 20 min at 20,000 rpm and the supernatant was stored at 4°C or at -20°C until purification. The material was purified essentially as described above (Example 2): cleared cell lysates containing the SakSTAR variants were subjected to chromatography on a 1.6 x 5 cm column of SP-Sephadex, followed by chromatography on a 1.6 x 8 cm column of phenyl-Sepharose. The SakSTAR containing fractions, localized by SDS-gel electrophoresis, were pooled for further analysis.

### 5. Physicochemical and biochemical analysis

Protein concentrations were determined according to Bradford (29). SDS-PAGE was performed with the Phast SystemTM (Pharmacia, Uppsala, Sweden) using 10-15% gradient gels and Coomassie Brillant blue staining, and the specific activities of SakSTAR solutions were determined with a chromogenic substrate assay carried out in microtiter plates (as described in example 2). The specific activity of the different SakSTAR variants are summarized in Table 3.

### 6. Reactivity of SakSTAR variants with a panel of murine monoclonal antibodies

The methodology used to determine the reactivity of the SakSTAR variants with a panel of murine monoclonal antibodies was described in example 1 above. The results are summarized in Table 3 (the layout of this Table corresponds to the layout of Table 1, as described in example 1). Apparent association constants at least 10-fold lower than those of wild-type staphylokinase were considered as significant and are indicated in bold type in the table.

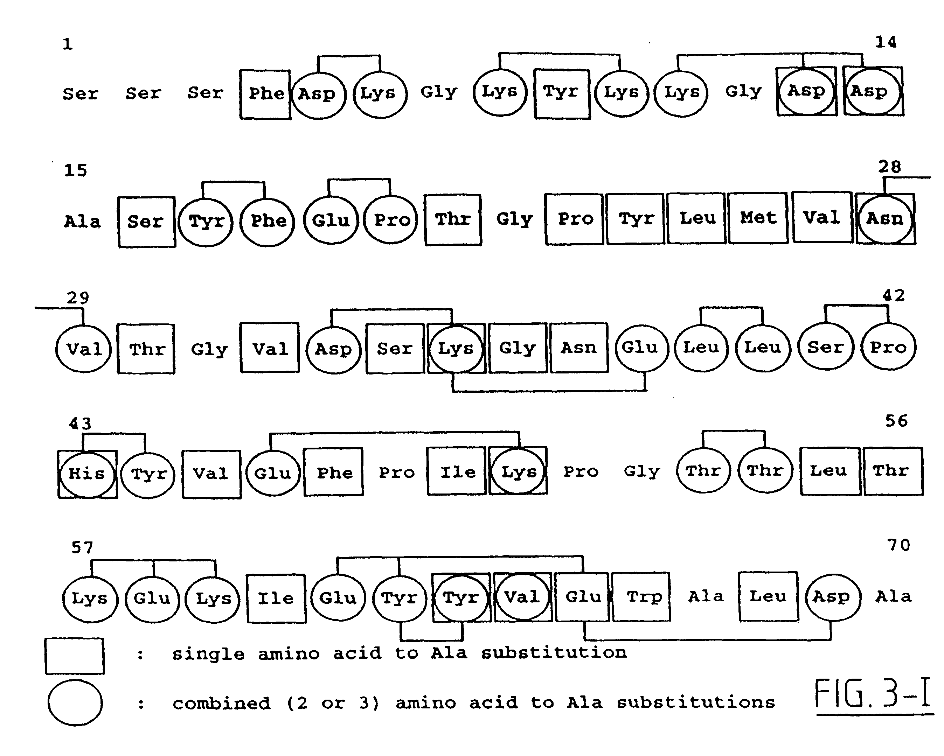

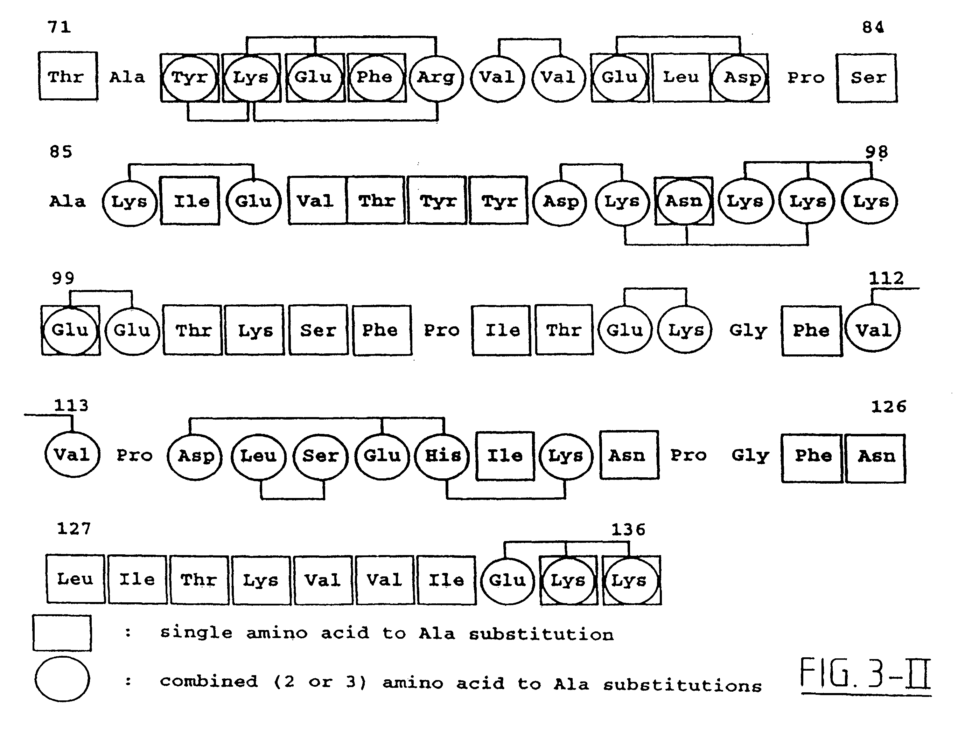

In order to obtain a comprehensive inventory of the properties of Ala-substitution variants of the SakSTAR molecule, 67 plasmids encoding variants with substitution of a single or two adjacent amino acids with Ala were constructed, expressed and purified. Together with the 35 charged residue to Ala-substitution variants previously described (22, and example 2), this analysis covers all residues in SakSTAR except Gly, Ala and Pro, as illustrated in Figure 3. Eight of the variants could not be obtained in purified form, primarily as a result of low expression levels, 11 variants were inactive, 56 had a reduced specific activity, and 27 had a maintained or increased specific activity (≥100 kHU/mg). The yields of purified material from cultures of expressed plasmids were 16 mg/L (median, 10 to 90 percentile range 4 to 41 mg/L). SDS polyacrylamide gel electrophoresis consistently showed one main band with Mᵣ≈ 16,000, usually representing 95% of total protein (not shown).

Substitution of K35, N95, S103 or K135 with Ala yielded variants with specific activities of ≥200 kU/mg. Substitution of W66, Y73 or E75 with Ala reduced the reactivity of the variants with ≥3 antibodies of epitope cluster I, of H43 or V45 with Ala that with 3 antibodies from epitope cluster II and of V32, K35, D82 and K130 with Ala that with ≥3 antibodies of epitope cluster III.

### 7. Absorption of antibodies, elicited in patients by treatment with SakSTAR

For the present example, the three plasma pools, as described in example 2 were used. The methodology used to evaluate the absorption with wild-type staphylokinase and with SakSTAR variants, of antibodies elicited in patients treated with SakSTAR, is described in detail in example 2. The results are summarized in Table 3. Whereas wild-type SakSTAR and most of the variants analyzed in this example absorbed more than 95% of the binding antibodies from pooled plasma of the 10 patients, incomplete absorption (<60%) was observed with SakSTAR(Y73A), and with SakSTAR(K74A). The predominant role of Lys74 for antibody recognition has been demonstrated previously (see example 2). The present results indicate that Tyr73 participates to the same major epitope as Lys74, or, alternatively, that substitution at Tyr73 may indirectly induce a structural modification of the "K74-epitope". Absorption with pooled plasma from 3 patients from which >95% of the antibodies were absorbed with SakSTAR(K35A,E38A,K74A,E75A,R77A) (Subpool C, see example 2) was nearly complete with most variants tested.

### EXAMPLE 5

### Construction, epitope mapping with murine monoclonal antibodies and absorption with pooled plasma of immunized patients, of staphylokinase variants with substitution of S34, G36 and/or H43

The natural variant Sak42D differs from SakSTAR in three amino acids and corresponds to SakSTAR(S34G, G36R,H43R). Sak42D is characterized by reduced reactivity with some murine antibodies of epitope clusters II and III and a slightly reduced absorption of antibodies from plasma of patients treated with SakSTAR (Table 4). Mutagenesis of these residues in SakSTAR revealed that the reduced reactivity with epitope cluster III and with immunized patient plasma could be ascribed to the G36R substitution, the H43R substitution mediated the reduced reactivity with epitope cluster II but had no effect on the reactivity with immunized patient plasma, whereas the S34A substitution had no effect. The G36R substitution could be combined with the K74R but not with the K74A substitution, without significant reduction of the specific activity (Table 4).

### EXAMPLE 6

### Construction, epitope mapping with murine monoclonal antibodies and absorption with nooled plasma of immunized patients, of staphylokinase variants with substitution of K35, E65, Y73, K74, E80+D82. N95. K130, V132 and/or K135

Based on the results of the alanine-substitution analysis in example 4, K35, N95 and K135 were selected for further analysis because SakSTAR(K35A), SakSTAR(N95A) and SakSTAR(K135A) had a two-fold increased specific activity, Y73 and K74 because SakSTAR(Y73A) and SakSTAR(K74A) had a markedly reduced reactivity with antibodies from epitope cluster I and diminished absorption of antibodies from plasma of patients immunized by treatment with SakSTAR, and K35, E80+D82, K130 and V132 because SakSTAR(K35A), SakSTAR(E80A,D82A), SakSTAR(K130A) and SakSTAR(V132A) had a reduced reactivity with antibodies from epitope cluster III.

In an effort to maximize the activity/antigenicity ratio, these amino acids were substituted with other amino acids than Ala. As summarized in Table 5, substitution of K35 with A, E or Q revealed that SakSTAR(K35A) had the most interesting properties, substitution of Y73 with F, H, L, S or W did not rescue the marked reduction in specific activity, and K74 confirmed its key role in binding of antibodies from immunized patient plasma, the best specific activity/antigenicity ratios being obtained with SakSTAR(K74Q) and SakSTAR(K74R). SakSTAR(E80A,D82A) was preferred over the single residue variants SakSTAR(E80A) or SakSTAR(D82A) because of its somewhat lower reactivity with immunized patient plasma. SakSTAR(N95A) could not be further improved by substitution of N95 with E, G, K or R and it was unable to confer its increased specific activity to variants containing K74A or K135R. Finally SakSTAR(K130A) was outperformed in terms of specific activity by SakSTAR(K130T) and SakSTAR(V132A) by SakSTAR(V132R).

### EXAMPLE 7

### Construction, epitope mapping with murine monoclonal antibodies and absorption with pooled plasma of immunized patients of combination variants of SakSTAR(K130T,K135R) and SakSTAR(E80A,D82A,K130T,K135R) with K35A,G36R,E65X,K74X and selected other amino acids

In the present and the following examples an additional plasma pool was made from 40 patients obtained several weeks after treatment with SakSTAR (Pool 40). The original pool from 10 patients is further identified as Pool 10. The absorption of staphylokinase-specific antibodies was quantified as described above and elsewhere (22).

The SakSTAR(K130T,K135R) variant was taken as a template for additive mutagenesis because of its high specific activity with a moderate reduction of binding to antibodies of epitope cluster III and absorption of antibodies from immunized patient plasma (Table 6). Addition of G36R, K74R, or K74Q or both to the template did not markedly reduce the specific activity, reduced the reactivity with monoclonal antibodies against epitope cluster III (G36R substitution) and decreased the absorption of antibodies from immunized patient plasma (K74R or K74Q substitution). Combination of E65A or E65Q with K74Q in the SakSTAR(K130T,K135R) template reduced the absorption of antibodies from Pool 10 and Pool 40 to around 50 and 60 percent respectively, without markedly reducing the specific activity. Addition substitution of selected amino acids in the SakSTAR(E65Q,K74Q,K130T, K135R) template did not further reduce the antibody absorption from Pool 10 or Pool 40. Surprisingly, the substitution of K136 with A and the addition of K in position 137 resulted in a marked increase in specific activity, as measured in the chromogenic substrate assay.

Combination of the SakSTAR(E80A,D82A) and Sak-STAR(K130T,K135R) templates, did not affect the specific activity and had a reduced reactivity with epitope cluster III antibodies (Table 7). Therefore the Sak-STAR(E80A,D82A,K130T,K135R) template was selected for further mutagenesis. Addition of K74R and even more of K74Q drastically reduced the reactivity with immunized patient plasma. Finally, addition of E65D or of K35A or E65S to the SakSTAR(K74R,E80A,D82A,K130T,K135R) or Sak-STAR(K74Q,E80A,D82A,K130T, K135R) templates yielded variants with intact specific activity which only bound ≤45 of the antibodies of pooled immunized patient plasma and less than 15 percent of the subpool reacting for more than 50 percent with the K74,E75,R77 epitope.

### EXAMPLE 8

### Characterization of selected variants of staphylokinase with intact specific activity and less than 50% adsorption of pooled SakSTAR specific human antibodies elicited in patients by treatment with wild-type SakSTAR

### 1. Introduction

Twenty three of the variants constructed and characterized in the above examples combined the properties of a residual specific activity of ≥100 kHU/mg and ≤50 percent absorption with the pool of antisera obtained from 10 patients treated with wild-type SakSTAR. The results are summarized in Table 8. Results obtained with Subpool B and Subpool C and with the pool of 40 patients treated with wild-type SakSTAR are included. SakSTAR(K74Q,E80A,D82A,K130T, K135R), SakSTAR(E65D,K74R, E80A,D82A,K130T,K135R), SakSTAR(K35A,E65D,K74Q, E80A, D82A,K130T,K135R) and SakSTAR(E65Q,K74Q,N95A,E118A, K130A,K135R,K136A,∇137K) were selected for further characterization.

### 2. Fibrinolytic properties of SakSTAR variants in human plasma in vitro

The fibrinolytic and fibrinogenolytic properties of the SakSTAR variants were determined as previously described. Dose- and time-dependent lysis of ¹²⁵I-fibrin labeled human plasma clots submerged in human plasma was obtained with the selected variants (Table 9). Spontaneous clot lysis during the experimental period was ≤5% (not shown). Equi-effective concentrations of test compound (causing 50% clot lysis in 2 hrs; C₅₀), determined graphically from plots of clot lysis at 2 hrs versus the concentration of plasminogen activator (not shown), ranged from 0.11 ± 0.01 to 0.24 ± 0.04 g/mL at which the residual fibrinogen levels ranges between 92 ± 30 and 97 ± 30 percent of baseline (Table 9). The concentrations of compound causing 50% fibrinogen degradation in 2 hrs in human plasma in the absence of fibrin were determined graphically from dose-response curves (not shown). These values (mean ± SD of 3 independent experiments) ranged from 14 ± 3.2 to 29 ± 3.1 µg/mL (Table 9). Surprisingly the very high specific activity of SakSTAR(E65Q,K74Q,N95A,E118A,K130A,K135R, K136A,∇137K) in the chromogenic assay was not associated with an increased thrombolytic potency in a plasma milieu.

### 3. Temperature stability of selected SakSTAR variants

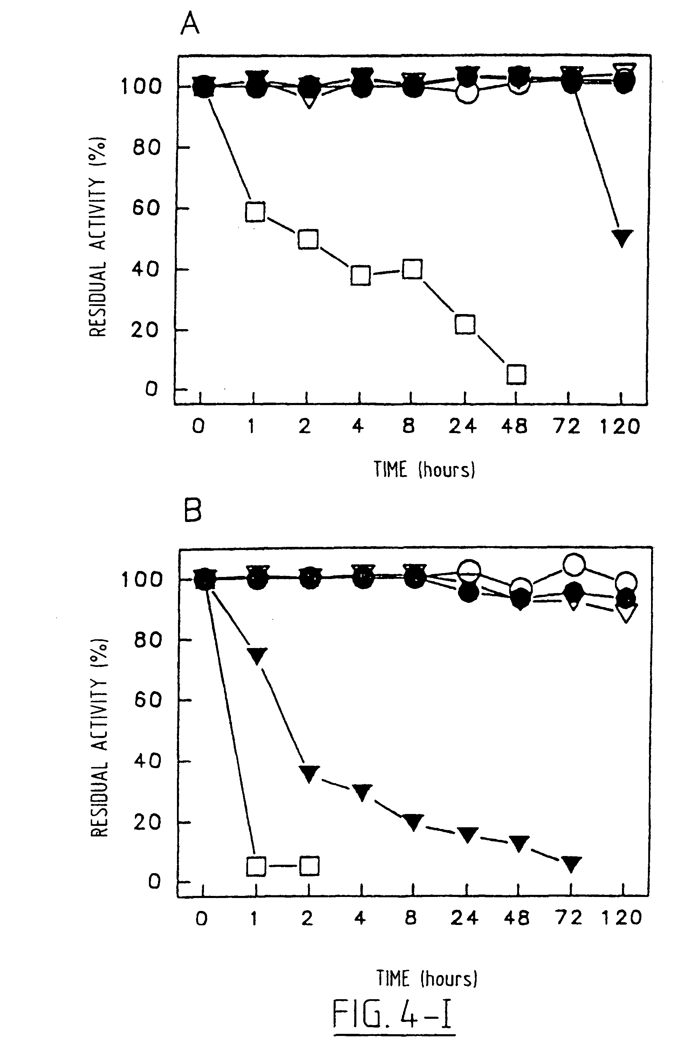

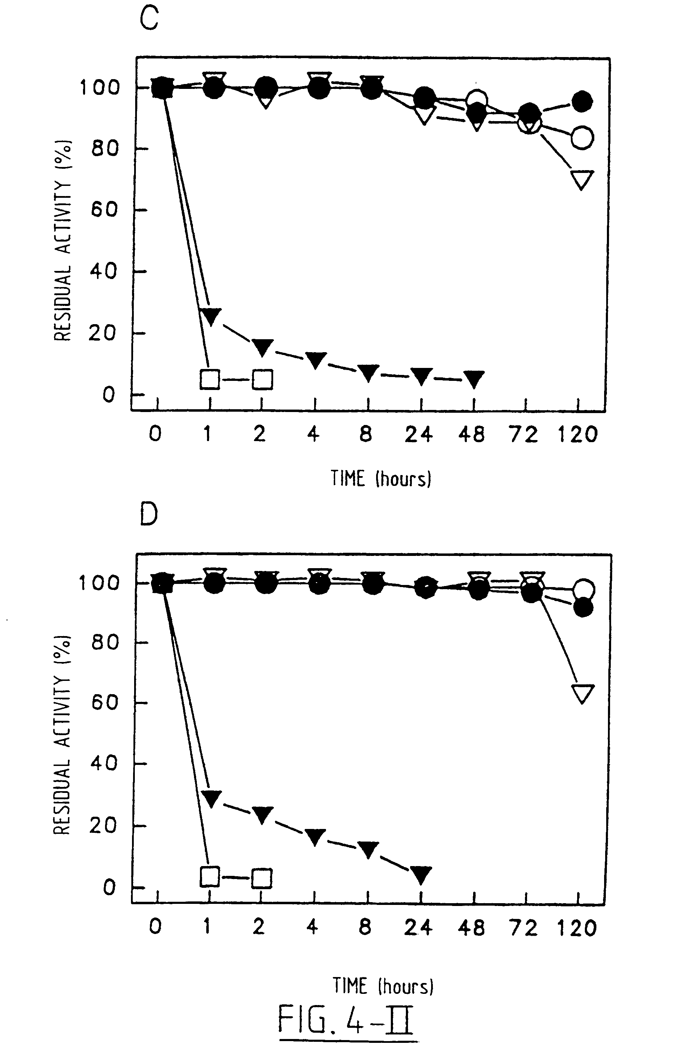

The temperature stability of preparations of SakSTAR(K74Q,E80A,D82A,K130T,K135R), SakSTAR(E65D,K74R, E80A,D82A,K130T,K135R) and SakSTAR(K35A,E65D,K74Q,E80A, D82A,K130T,K135R), dissolved to a concentration of 1.0 mg/mL in 0.15 mol/L NaCl, 0.01 mol/L phosphate buffer, pH 7.5 at various temperatures is illustrated in Fig. 4. At temperatures up to 37°C, all compounds remained fully active for up at least three days. At 56°C and 70°C the three variants were however less stable than wild-type SakSTAR.

### 4. Pharmacokinetic properties of SakSTAR variants following bolus injection in hamsters

The pharmacokinetic parameters of the disposition of SakSTAR variants from blood were evaluated in groups of 4 hamsters following intravenous bolus injection of 100 µg/kg SakSTAR variant. SakSTAR-related antigen was assayed using the ELISA described elsewhere. The ELISA was calibrated against each of the SakSTAR variants to be quantitated. Pharmacokinetic parameters included: initial half-life (in min), t1/2α= ln2/α; terminal half-life (in min), t1/2β=ln2/β; volume of the central (plasma) compartment (in mL), V_{c}= dose/(A+B); area under the curve (in µg.min.mL⁻¹), AUC= A/α + B/β; and plasma clearance (in mL.min⁻¹), Clp= dose/AUC (33).

The disposition rate of staphylokinase-related antigen from blood following bolus injection of 100 µg/kg of the selected SakSTAR variants in groups of 4 hamsters could adequately be described by a sum of two exponential terms by graphical curve peeling (results not shown), from which the pharmacokinetic parameters summarized in Table 10 were derived. The pharmacokinetic parameters of the mutants were not markedly different from those of wild type SakSTAR. Initial plasma half-lives (t1/2(α)) ranged between 2.0 and 3.2 min and plasma clearances (Clp) between 1.6 and 4.1 mL/min.

### EXAMPLE 9

### Comparative thrombolytic efficacy and immunogenicity of SakSTAR(K74Q,E80A,D82A, K130T,K135R) and SakSTAR(E65D,K74R,E80A,D82A,K130T,K135R) versus SakSTAR in patients with peripheral arterial occlusion

### 1. Purification for use in vivo

Eighteen liter cultures (in 2 L batches) of the variants SakSTAR(K74Q,E80A,D82A,K130T, K135R) and SakSTAR(E65D,K74R,E80A,D82A,K130T,K135R) were grown for 20 hours in terrific broth medium (28), supplemented with 100 µg/mL ampicillin and induced with IPTG during the last 3 hours. The cells were pelleted, resuspended in 1/10 volume of 0.01 mol/L phosphate buffer, pH 6.0, disrupted by sonication and cleared by centrifugation. The compounds were purified by chromatography on a 10 x 7 cm column of SP-Sepharose, equilibrated with 0.01 mol/L phosphate buffer, pH 6.0 and eluted with a 1 mol/L NaCl gradient (3 column volumes). The fractions containing SakSTAR variant were pooled, solid NaCl was added to a concentration of 2.5 mol/L and the material was chromatographed on a 10 x 20 cm column of phenyl-Sepharose followed by stepwise elution with 0.01 mol/L phosphate buffer, pH 6.0. The materials were desalted on a 10 x 45 cm column of Sephadex G25, concentrated by application on a 5 x 10 cm column of SP-Sepharose with stepwise elution with 1.0 mol/L NaCl and finally gel filtered on a 6 x 60 cm column of Superdex 75 equilibrated with 0.15 m NaCl, 0.01 mol/L phosphate buffer, pH 7.5 to further reduce their endotoxin content. The SakSTAR variant containing fractions were pooled, the protein concentration was adjusted to 1 mg/mL and the material sterilized by filtration through a 0.22 m Millipore filter. The methodology used to determine specific activity, endotoxin contamination, bacterial sterility and toxicity in mice is described above and elsewhere (22). The purity of the preparation was evaluated by SDS gel electrophoresis on 10% gels to which 40 g of compound was applied.

out of culture volumes of 18 liters of SakSTAR variant, 840 mg of SakSTAR(K74Q,E80A, D82A,K130T,K135R) with a specific activity of 140 kHU/mg and 800 mg Sak-STAR(E65D, K74R,E80A,D82A,K130T,K135R) with a specific activity of 150 were purified. The endotoxin content was <0.1 and 0.26 IU/mg. Gel filtration on HPLC revealed a single main symmetrical peak in the chromatographic range of the column, representing >98% of the eluted material (total area under the curve) (not shown). SDS gel electrophoresis of 40 g samples revealed single main components (not shown). Preparations sterilized by filtration proved to be sterile on 3 day testing as described elsewhere (22). Intravenous bolus injection of SakSTAR variants in groups of 5 mice (3 mg/kg body weight), did not provoke any acute reaction, nor reduced weight gain within 8 days, in comparison with mice given an equal amount of saline (not shown).

### 2. Thrombolytic efficacy

Wild-type SakSTAR or the variants SakSTAR(K74Q, E80A,D82A,K130T,K135R) or SakSTAR(E65D,K74R,E80A,D82A, K130T,K135R) were administered intra-arterially at or in the proximal end of the occlusive thrombus as a bolus of 2 mg followed by an infusion of 1 mg/hr (reduced overnight in some patients to 0.5 mg/hr) in groups of 15, 6 and 6 patients respectively with angiographically documented occlusion of a peripheral artery or bypass graft of less than 30 days duration. Patients were studied after giving informed consent, and the protocol was approved by the Human Studies Committee of the University of Leuven. Inclusion and exclusion criteria, conjunctive antithrombotic treatment (including continuous intravenous heparin) and the study protocol were essentially as previously described (22).

Relevant baseline characteristics of the individual patients and results of treatment and outcome are shown in Table 11. Intra-arterial infusion, at a dose of 3.5 to 27 mg and a duration of 2 to 44 hrs, induced complete recanalization in 22 patients and partial recanalization in 5. Complementary endovascular procedures (mainly PTA) were performed in 13 patients and complementary reconstructive vascular surgery following thrombolysis in 5. One patient underwent major amputation. Bleeding complications were usually absent or limited to mild to moderate hematoma formation at the angiographic puncture sites (data not shown). One patient, given wild-type SakSTAR suffered a non-fatal intracranial bleeding, one (BUE) a retroperitoneal hematoma and two (MAN and STRO) a gastro-intestinal bleeding.

Circulating fibrinogen, plasminogen and α₂-antiplasmin levels remained unchanged during infusion of the SakSTAR moieties (data not shown), reflecting absolute fibrin specificity of these agents at the dosages used (data not shown). Significant in vivo fibrin digestion occurred as evidenced by elevation of fibrin fragment D-dimer levels. Intra-arterial heparin therapy prolonged aPTT levels to a variable extent (data not shown).

### 3. Antibody induction

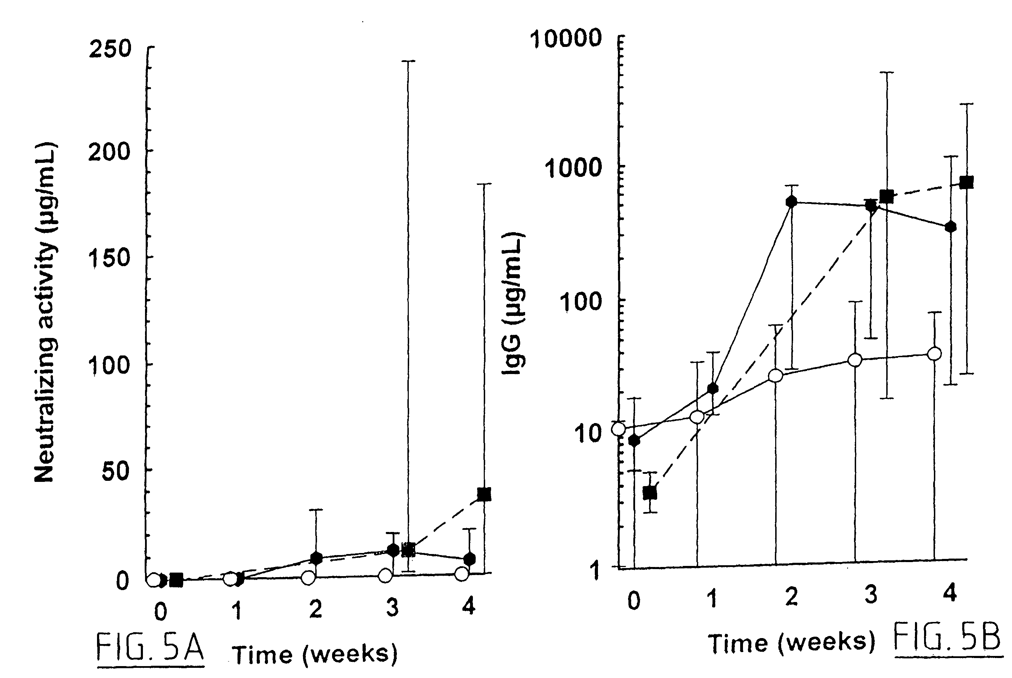

Staphylokinase-neutralizing activity in plasma and antigen-specific IgG antibodies were quantitated essentialy as described above and elsewhere (22). Antibody-related SakSTAR-, SakSTAR(K74Q,E80A,D82A, K130T,K135R)- and SakSTAR(E65D,K74R,E80A,D82A, K130T,K135R)-neutralizing activity and anti-SakSTAR, anti-SakSTAR(K74Q,E80A,D82A,K130T,K135R) and anti-SakSTAR(E65D,K74R,E80A, D82A,K130T,K135R) IgG, were low at baseline and during the first week after the infusion (Figure 5). From the second week on, neutralizing activity levels increased to reach median values at 3 to 4 weeks of 9 µg SakSTAR(K74Q,E80A,D82A, K130T,K135R) and 0.5 µg SakSTAR(E65D,K74R,E80A,D82A, K130T,K135R) neutralized per mL plasma in the patients treated with the corresponding moieties, respectively, as compared to median value of 24 µg wild-type SakSTAR neutralized per mL in the 15 patients treated with SakSTAR. The levels of anti-SakSTAR(K74Q,E80A,D82A, K130T,K135R) and of anti-SakSTAR(E65D,K74R,E80A, D82A,K130T,K135R) IgG increased to median values at 3 to 4 weeks of 420 and 30 µg/mL plasma in patients treated with the corresponding moieties, respectively, as compared to a median value of 590 µg anti-SakSTAR per mL plasma in the patients treated with SakSTAR (Figure 5). The prevalence of immunization, defined as neutralizing activities in plasma after 2 to 4 weeks exceeding 5 g/ml was 3 of 6 patients (50 percent) with SakSTAR(K74Q,E80A, D82A,K130T,K135R), 1 of 6 patients (17 percent) with SakSTAR(E65D,K74R,E80A,D82A, K130T,K135R), as compared to 56 of 70 patients (80 percent) with SakSTAR. This difference is statistically highly significant (p= 0.01 by 2 x 3 Chi square analysis).

The antibodies induced by treatment with SakSTAR were completely absorbed by SakSTAR but incompletely by SakSTAR(K74Q,E80A,D82A,K130T,K135R) and by SakSTAR(E65D,K74R, E80A,D82A,K130T,K135R) (Table 12). Antibodies induced by treatment with Sak-STAR(K74Q,E80A,D82A,K130T,K135R), detectable in 4 of the 6 patients, were completely (≥90 percent) absorbed by SakSTAR, by SakSTAR(K74Q,E80A,D82A,K130T, K135R) and by SakSTAR(E65D,K74R,E80A,D82A,K130T,K135R), indicating that immunization was not due to neoepitopes generated by substitution of wild-type amino acids. Antibodies induced by treatment with SakSTAR(E65D,K74R,E80A,D82A, K130T,K135R) detectable in one patient (URB) were completely absorbed with SakSTAR(K74Q,E80A,D82A, K130T,K135R) and with SakSTAR(E65D,K74Q,E80A,D82A, K130T,K135R) but incompletely (85%) with wild-type SakSTAR, suggesting that a small fraction of the induced antibodies might be directed against a neoepitope in the variant used for infusion.

### EXAMPLE 10

### Construction and absorption with pooled plasma of immunized patients of combination variants of SakSTAR(E65Q,K74Q,K130T,K135R) and other selected amino acids

### 1. Introduction

In a final round of additive substitution mutagenesis, the SakSTAR(E65Q,K74Q,K130T, K135R) variant was taken as a template because it displayed a high specific activity with a significant reduction of absorption (to 65 percent) of antibodies from pooled immunized patient plasma (Pool 40). The intermediate variants which were relevant for the composition of the finally selected variants are summarized in Table 13. Addition of K35A, D82A and S84A, of T90A,E99D and T101S or of E108A and K109A reduced the antibody absorption to around 50 percent, whereas the combined addition of D82A,S84A and E108A, K109A reduced it to 41 percent. Substitution of K136A combined with the addition of a Lys at the COOH terminus (-137K) increased the specific activity in a purified system but not in a plasma milieu nor in a hamster pulmonary embolism model (not shown), and further reduced the absorption of antibodies from pooled patient plasma to 30 percent. Finally, addition of the K35A, and T90A,E99D,T101S substitutions to this template yielded a mutant with intact thrombolytic potency which only bound 24 percent of the antibodies of pooled immunized patient plasma.

Based on this analysis, SakSTAR(E65Q,K74Q,D82A, S84A,E108A,K109A,K130T,K135R, K136A, ∇137K), (SY118), and SakSTAR(K35A,E65Q,K74Q,D82A,S84A,T90A,E99D,T101S, E108A,K109A,K130T,K135R,K136A,V137K), (SY141), were selected for further characterization. In addition, Sak-STAR(K35A,E65Q,K74R,D82A,S84A,T90A,E99D,T101S, E108A,K109A,K130T,K135R,K136A,V137K), (SY145) with a Lys in position 74, was constructed and evaluated.

### 2. Pharmacokinetic properties of SakSTAR variants following bolus injection in hamsters

The disposition rate of staphylokinase-related antigen from blood following bolus injection of 100 µg/kg of the selected SakSTAR variants in groups of 4 hamsters could adequately be described by a sum of two exponential terms by graphical curve peeling (results not shown). The pharmacokinetic parameters of the mutants were derived from these plasma disappearance curves not markedly different from those of wild type SakSTAR (results very similar to those of table 10, data not shown).

### EXAMPLE 11

### Characterization of selected variants derived from SakSTAR(E650,K74Q,K130T,K135R)

### 1. Fibrinolytic properties of selected SakSTAR variants towards human plasma in vitro

Dose- and time-dependent lysis of ¹²⁵I-fibrin labeled human plasma clots submerged in human plasma was obtained with the three selected variants (Table 14). Spontaneous clot lysis during the experimental period was ≤5% (not shown). Equi-effective concentrations of test compound (causing 50% clot lysis in 2 hrs; C₅₀), determined graphically from plots of clot lysis at 2 hrs versus the concentration of plasminogen activator (not shown), ranged from 0.15 ± 0.02 to 0.19 ± 0.01 µg/ml at which no significant fibrinogen degradation occurred. The concentrations of compound causing 50% fibrinogen degradation in 2 hrs in human plasma in the absence of fibrin were determined graphically from dose-response curves (not shown). These values (mean ± SD of 3 independent experiments) ranged from 7.0 ± 0.6 to 24 ± 3.6 µg/ml (Table 14).

### 2. Temperature stability of selected SakSTAR variants

The temperature stability of preparations of SakSTAR(E65Q,K74Q,D82A,S84A,E108A,K109A,K130T,K135R, K136A,∇137K), SakSTAR(K35A,E65Q,K74Q,D82A,S84A,T90A, E99D,T101S,E108A,K109A,K130T,K135R,K136A,∇137K), and SakSTAR(K35A,E65Q, K74R,D82A,S84A,T90A,E99D,T101S,E108A, K109A,K130T,K135R,K136A,∇137K) dissolved to a concentration of 1.0 mg/ml in 0.15 M NaCl, 0.01 M phosphate buffer, pH 7.5 at various temperatures. At temperatures up to 37°C, all compounds remained fully active for up to at least three days. At 56°C and 70°C the variants were generally less stable than wild type SakSTAR (results very similar to those of Figure 4, data not shown).

### EXAMPLE 12

### Comparative thrombolytic efficacy and immunogenicity of SakSTAR(E65Q, K74Q, D82A,S84A,E108A,K109A,K130T,K135R, K136A, ∇137K), (SY118), SakSTAR(K35A, E65Q,K74Q,D82A,S84A, T90A,E99D,T101S,E108A,K109A,K130T,K135R,K136A, V∇137K), (SY141), and SakSTAR(K35A,E65Q,K74R,D82A,S84A,T90A, E99D,T101S, E108A,K109A,K130T,K135R,K136A,∇137K), (SY145), in patients with peripheral arterial occlusion

### 1. Large scale purification and conditioning of SakSTAR variants for use in vivo

Material was purified to homogeneity out of culture volumes of 18 liters. The endotoxin content was below 2 IU/mg. Gel filtration on HPLC revealed a single main symmetrical peak in the chromatographic range of the column, representing >98% of the eluted material (total area under the curve) (not shown). SDS gel electrophoresis of 30 µg samples revealed single main components. Preparations sterilized by filtration proved to be sterile on 3 day testing. Intravenous bolus injection of SakSTAR variants in groups of 5 mice (3 mg/kg body weight), did not provoke any acute reaction, nor reduced weight gain within 8 days, in comparison with mice given an equal amount of saline (not shown).

Groups of 6 patients with angiographically documented peripheral arterial occlusion (PAO) were studied. Relevant baseline characteristics of the individual patients are shown in Table 15. Table 16 summarizes the individual treatment and outcome. Intra-arterial infusion, at a dose of 6 to 24 mg and a duration of 4 to 29 hrs, induced complete recanalization in most patients. Circulating fibrinogen, plasminogen and α₂-antiplasmin levels remained essentially unchanged during infusion of the SakSTAR variants (data not shown), reflecting absolute fibrin specificity of these agents at the dosages used. Antibody-related SakSTAR(E65Q,K74Q, D82A,S84A,E108A,K109A,K130T,K135R,K136A,∇137K)-, Sak-STAR(K35A,E65Q,K74Q,D82A,S84A,T90A,E99D,T101S,E108A, K109A, K130T,K135R,K136A,V137K)- and SakSTAR(K35A,E65Q, K74R,D82A,S84A,T90A,E99D, T101S,E108A,K109A,K130T,K135R, K136A,∇137K)-neutralizing activity, were low at baseline and during the first week after the infusion (Table 17). From the second week on neutralizing activity levels increased to reach median values at 3 to 4 weeks of 19 µg SakSTAR(E65Q,K74Q,D82A,S84A,E108A,K109A,K130T,K135R, K136A,∇137K), (SY118), 0.7 µg SakSTAR(K35A,E65Q,K74Q, D82A, S84A,T90A,E99D,T101S,E108A,K109A,K130T,K135R, K136A,∇137K), (SY141), and 4.3 µg SakSTAR(K35A,E65Q,K74R, D82A,S84A,T90A,E99D,T101S,E108A,K109A,K130T,K135R, K136A,∇137K), (SY145), neutralized per ml plasma in the patients treated with the respective compounds, which for SY141 and SY145, but not for SY118 is lower than the median value of 12 µg wild type SakSTAR neutralized per ml in 69 patients treated with wild type SakSTAR.

Overt immunization (neutralizing activity at 3 to 4 weeks of 5 g compound per ml plasma) was observed in 56 of 70 patients treated with SakSTAR, in 5 of the 6 patients exposed to SakSTAR(E65Q,K74Q,D82A,S84A,E108A, K109A,K130T,K135R,K136A,∇137K), (SY118), only in 2 of the 6 patients given SakSTAR(K35A,E65Q,K74Q,D82A,S84A,T90A, E99D, T101S,E108A,K109A,K130T,K135R,K136A,∇137K), (SY141), and in 1 of the 3 patients given SakSTAR(K35A, E65Q,K74R,D82A,S84A,T90A,E99D,T101S,E108A,K109A,K130T, K135R,K136A,∇137K), (SY145).

The results with respect to immunogenicity of the main variants studied in patients are summarized in Table 18. Clearly, variants SakSTAR(E65D,K74R,E80A,D82A, K130T,K135R) and SakSTAR(K35A,E65Q,K74Q,D82A,S84A,T90A, E99D,T101S,E108A,K109A,K130T, K135R,K136A,∇137K) have a significantly reduced immunogenicity when compared to the wild type protein.

### EXAMPLE 13

### Construction, purification and characterization of cysteine-substitution mutants of staphylokinase

### 1. Introduction

Site-directed mutagenesis was applied to substitute exposed amino acids with single cysteine residues in order to construct i) homodimeric forms of staphylokinase, upon formation of an intermolecular disulfide bridge, and ii) polyethylene glycol-conjugated molecules (PEG-derivatives). The aim of this example was twofold: first, the clearance can be reduced by increasing the size of the injected molecule (via dimerization or conjugation with large molecule such as PEG) and second, PEG-derivatives have also been shown to induce a reduced immunoreactivity in animal models (for review, see ref. 34). In both cases, a prolonged half-life in vivo could help to reduce the pharmacological dose of staphylokinase in patients. This reduction could be accompanied with a reduced immunogenic reaction against the thrombolytic agent, thus enhancing its pharmacological activity as a thrombolytic agent.

In this example, the construction and characterization of two SakSTAR variants in which one single amino acid was substituted with cysteine is described. The mutants described under this example are listed in Table 19. These variants were expressed in E. coli, purified and characterized in terms of specific activity, fibrinolytic properties in human plasma in vitro and pharmacokinetic properties following bolus injection in hamsters.

### 2. Reagents and Methods

The source of all reagents used in the present study has previously been reported (22), or is specified below. The template vector for mutagenesis, pMEX602sakB (i.e. pMEX.SakSTAR), has been described elsewhere (23). Restriction and modification enzymes were purchased from New England Biolabs (Leusden, The Netherlands), Boehringer Mannheim (Mannheim, Germany) or Pharmacia (Uppsala, Sweden). The enzymatic reactions were performed according to the supplier recommendation. The mutagenic oligonucleotides and primers were obtained from Eurogentec (Seraing, Belgium). Plasmid DNA was isolated using a purification kit from Qiagen (Hilden, Germany), as recommended. Transformation-competent E. coli cells were prepared by the well-known calcium phosphate procedure. Nucleotide sequence determination was performed on double strand plasmid DNA with the dideoxy chain termination method, using the T7 sequencing kit (Pharmacia, Uppsala, Sweden). Polymerase chain reactions (PCR) were performed using Taq polymerase from Boehringer Mannheim (Mannheim, Germany). The recombinant DNA methods required to construct the variants described in this example are well established (22, 27).

### 3. Construction of expression plasmids

The variants SakSTAR(K102C) and SakSTAR(K109C), were constructed by the spliced overlap extension polymerase chain reaction (SOE-PCR) (24) using pMEX.SakSTAR encoding SakSTAR as template. Two fragments were amplified by PCR (30 cycles: 1 sec at 94°C, 1 sec at 50°C, 10 sec at 72°C), the first one starting from the 5' end (primer 818A) of the staphylokinase gene to the region to be mutagenized (forward primer), the second one from this same region (backward primer) to the 3' end of the gene with primer 818D (5' CAAACAGCCAAGCTTCATTCATTCAGC). The forward and backward primers shared an overlap of around 24 bp (for the construction of K102C: TAT GAT AAG AAT TGC AAA AAA GAA GAA (backward) and TTC TTC TTT TTT GCA ATT CTT ATC ATA (forward), for the construction of K109C: AAA AAG AAG AAA CGT GCT CTT TCC CTA (backward) and TAG GGA AAG AGC ACG TTT CTT CTT TTT (forward)). The two purified fragments were then assembled together in a second PCR reaction with the external primers 818A and 818D (30 cycles: 1 sec at 94°C, 1 sec at 50°C, 10 sec at 72°C). The amplified product from this final reaction was purified, digested with EcoRI and HindIII and ligated into the corresponding site of pMEX.SakSTAR. For each construction, the sequence of the variant was confirmed by sequencing the entire coding region.

### 4. Expression and purification of SakSTAR variants

The SakSTAR variants were expressed and purified, as described below, from transformed E. coli grown in terrific broth (TB) medium (28). A 2 to 4 mL aliquot of an overnight saturated culture in LB medium was used to inoculate a 1 to 2 L culture in terrific broth supplemented with 100 µg/mL ampicillin. The culture was incubated with vigorous aeration and at 30°C. After about 16 hours incubation, IPTG (200 µmol/L) was added to the culture to induce expression from the tac promoter. After 3 hours induction, the cells were pelleted by centrifugation at 4,000 rpm for 20 min, resuspended in 1/10 volume of 0.01 mol/L phosphate buffer pH 6-6.5 and disrupted by sonication at 0°C. The suspension was centrifuged for 20 min at 20,000 rpm and the supernatant was stored at 4°C or at -20°C until purification. The material was purified essentially as described above (Example 2): cleared cell lysates containing the SakSTAR variants were subjected to chromatography on a 1.6 x 5 cm column of SP-Sephadex, followed by chromatography on a 1.6 x 8 cm column of phenyl-Sepharose. The SakSTAR containing fractions, localized by SDS-gel electrophoresis, were pooled for further analysis.

### 5. Biochemical analysis

Protein concentrations were determined according to Bradford (29). SDS-PAGE was performed with the Phast System™ (Pharmacia, Uppsala, Sweden) using 10-15% gradient gels and Coomassie Brillant blue staining, and the specific activities of SakSTAR solutions were determined with a chromogenic substrate assay carried out in microtiter plates (as described in example 2). The specific activity of the different SakSTAR variants are summarized in Table 19.

Mutant SakSTAR(K102C) was essentially monomeric as visualized by SDS-PAGE and Coomassie Brillant blue staining. Its specific activity was comparable to that of wild-type staphylokinase. In contrast, SakSTAR(K109C) showed a propensity to form dimers (> 60%). This resulted in a markedly increased specific activity in the plasminogen-coupled chromogenic substrate assay (see Table 19). Upon reduction with dithiothreitol (DTT) (20-fold molar excess during 1.5 hour at 37°C) and alkylation with iodoacetamide (100-fold molar excess during 1 hour at 37°C), the K109C dimer is converted into a stable monomer and its resulting specific activity is within the expected range towards wild-type staphylokinase (Table 19). This result confirms that formation of homodimers is the unique determinant for this large increase in specific activity. Dimeric SakSTAR(K109C) was separated from monomeric SakSTAR(K109C) by chromatography on Source S (Pharmacia) (5 x 50mm). Loading buffer was 10 mM phosphate, pH 6.0 and dimeric SakSTAR(K109C) was eluted by a salt gradient (up to 1 M) in the same buffer. The dimeric SakSTAR(K109C) (>95% pure) containing fractions, localized by SDS-gel electrophoresis, were pooled for further analysis.

### 6. Chemical crosslinking of cysteine mutants of SakSTAR with polyethylene glycol

The thiol group of the cysteine mutant SakSTAR(K102C) was targeted for coupling with an activated polyethylene glycol, OPSS-PEG (Shearwater Polymers Europe, Enschede, The Netherlands). OPSS-PEG is a 5 kDa PEG molecule carrying a single activated thiol group at one end that react specifically at slightly alkaline pH with free thiols. Modification of SakSTAR(K102C) was achieved by incubating the molecule (100 µM) with a three-fold excess of SS-PEG in a 5 mM phosphate, pH 7.9 solution at room temperature. The extent of the reaction was monitored by following the release of 2-thiopyridone from OPSS-PEG at 412 nm. After reaction (about 15 min), the excess of OPSS-PEG was removed by purifying the derivatized SakSTAR(K102C-PEG) on a 1.6 x 5 cm column of SP-Sephadex as described above (see Example 2). The SakSTAR(K102C-PEG) containing fractions, localized by optical density at 280 nm, were pooled for further analysis. SDS-PAGE analysis and Coomassie blue staining confirmed that PEG crosslinking on SakSTAR(K102C) was quantitative. As shown in Table 19, the specific activity of the PEG-derivative was only marginally affected when compared to that of wild-type staphylokinase.

### 7. Fibrinolytic properties of SakSTAR variants in human plasma in vitro

The fibrinolytic and fibrinogenolytic properties of SakSTAR variants were determined as previously described. Dose- and time-dependent lysis of ¹²⁵I-fibrin labeled human plasma clots submerged in human plasma was obtained with four molecules: SakSTAR(K109C) as dimer and as monomer (after reduction and alkylation with iodoacetamide), the monomeric SakSTAR(K102C) and the PEG-derivatized SakSTAR(K102C). Spontaneous clot lysis during the experimental period was ≤5% (not shown). Equi-effective concentrations of test compound (causing 50% clot lysis in 2 hrs; C₅₀), determined graphically from plots of clot lysis at 2 hrs versus the concentration of plasminogen activator (not shown), were comparable to that of SakSTAR, for monomeric SakSTAR(K109C) and SakSTAR(K102C) (Table 19). However, it was observed that the C₅₀ for clot lysis by dimeric SakSTAR(K109C) was only 0.12 µg/ml, which is approximately three-fold lower than for wild-type staphylokinase. In contrast, a C₅₀ of 0.60 µg/ml was measured for SakSTAR(K102C-PEG), which is only two-fold higher than for wild-type staphylokinase. Thus, dimerization of SakSTAR via disulfide bridges or increasing the size of the molecule via PEG-derivatization does not preclude the fibrinolytic activity of staphylokinase. While a PEG-molecule appears to reduce the diffusion and therefore fibrinolytic potency of the derivatized staphylokinase within a fibrin clot, dimerization of staphylokinase results in a synergistic fibrinolytic effect on human fibrin clots.

### 8. Pharmacokinetic properties of dimeric SakSTAR(K109C) and SakSTAR(K102C-PEG) following bolus iniection in hamsters

The pharmacokinetic parameters of the disposition of dimeric SakSTAR(K109C) and SakSTAR(K102C-PEG) from blood were evaluated in groups of 4 hamsters following intravenous bolus injection of 100 µg/kg SakSTAR variant. SakSTAR-related antigen was assayed using the ELISA described elsewhere. The ELISA was calibrated against each of the SakSTAR variants to be quantitated. Pharmacokinetic parameters included: initial half-life (in min), t1/2α= ln2/α; terminal half-life (in min), t1/2β= ln2/β; volume of the central (plasma) compartment (in mL), VC= dose/(A+B); area under the curve (in µg.min.mL⁻¹), AUC= A/α + B/β; and plasma clearance (in mL.min⁻¹), Clp= dose/AUC (32).

The disposition rate of staphylokinase-related antigen from blood following bolus injection of 100 µg/kg of the selected SakSTAR variants in groups of 4 hamsters could adequately be described by a sum of two exponential terms by graphical curve peeling (results not shown), from which the pharmacokinetic parameters t1/2α and Clp, summarized in Table 19 were derived. The pharmacokinetic parameters of dimeric SakSTAR(K109C) and SakSTAR(K102C-PEG) were markedly different from those of wild type SakSTAR. Initial plasma half-lives (t1/2(α)) were 3.6 and 3.0 min and plasma clearances (Clp) were 0.52 and 0.32 mL/min, for dimeric SakSTAR(K109C) and SakSTAR(K102C-PEG), respectively. These results may be due to the increase of the Stokes radius of SakSTAR as a result of the dimerization or crosslinking with PEG. According to size-exclusion chromatography on Superdex50 by HPLC, dimeric SakSTAR(K109C) and SakSTAR(K102C-PEG) have apparent molecular weights of 33 kDa and 40 kDa, respectively.

### EXAMPLE 14

### Construction, purification and characterization of cysteine-substitution mutants of variants of staphylokinase with reduced immunogenicity

### 1. Introduction

Based on the results of example 13, additional polyethylene glycol derivatives of SakSTAR variants were constructed, purified and characterized. The least immunogenic variants SakSTAR(E65D,R74R,E80A, D82A,K130T,K135R), (SY19), and SakSTAR(K35A,E65Q, K74Q,D82A,S84A,T90A,E99D,T101S,E108A,K109A,K1-30T,K135R,K136A,V137K), (SY141), were used as templates, with the proviso that the COOH-terminus of the latter was reverted to the wild type sequence, S84A was replaced with E80 and K74Q replaced with K74R, yielding Sak-STAR(K35A,E65Q,K74R,E80A,D82A,T90A,E99D,T101S,E108A, K109A,K130T,K135R), (SY161). The introduced cysteine, which functions as acceptor of the polyethylene glycol molecule was located in the amino terminal region (preferably, but not exclusively, the Ser in position number 3 of the mature staphylokinase variant) in order to be released upon activation of staphylokinase (release of the 10 NH₂-terminal amino acids); finally polyethylene glycol molecules of different molecular weights (Mᵣ 5,000 to 20,000) were used, substituted with either OPSS or maleimide.

The mutants described under this example are listed in Table 20. These variants were expressed in E.coli, purified and characterized in terms of specific activity, fibrinolytic properties in human plasma in vitro, pharmacokinetic properties following bolus injection in hamsters, thrombolytic properties following bolus injection in a hamster pulmonary embolism model, and absorption of antibodies from pooled immunized patient plasma (Pool 40).

### 2. Reagents and Methods

The source of all reagents used in the present study has previously been reported (22), or is specified below. The template vector for mutagenesis, pMEX602sakB (i.e. pMEX.SakSTAR), has been described elsewhere (23). Restriction and modification enzymes were purchased from New England Biolabs (Leusden, The Netherlands), Boehringer Mannheim (Mannheim, Germany) or Pharmacia (Uppsala, Sweden). The enzymatic reactions were performed according to the supplier recommendation. The mutagenic oligonucleotides and primers were obtained from Eurogentec (Seraing, Belgium). Plasmid DNA was isolated using a purification kit from Qiagen (Hilden, Germany), as recommended. Transformation-competent E. coli cells were prepared by the well-known calcium phosphate procedure. Nucleotide sequence determination was performed on double strand plasmid DNA with the dideoxy chain termination method, using the T7 sequencing kit (Pharmacia, Uppsala, Sweden). Polymerase chain reactions (PCR) were performed using Taq polymerase from Boehringer Mannheim (Mannheim, Germany). The recombinant DNA methods required to construct the variants described in this example are well established (22, 27).

### 3. Construction of expression plasmids

The variants SakSTAR(S3C,E65D,K74R,E80A,D82A, K130T,K135R), (SY19(S3C)), SakSTAR(S2C,S3C,E65D,K74R, E80A,D82A,K130T,K135R), (SY19(2SC,3SC)), SakSTAR(S3C, K35A,E65Q,K74Q,D82A,S84A,T90A,E99D,T101S,E108A,K109A, K130T,K135R,K136A,∇137K), (SY141(S3C)), SakSTAR(S2C, S3C,K35A,E65Q,K74Q,D82A,S84A,T90A,E99D,T101S,E108A,-K109A,K130T,K135R,K136A,∇137K), (SY141(S2C,S3C)), Sak-STAR(S3C,K35A,E65Q,K74Q,E80A,D82A,T90A,E99D,T101S,E108A, K109A,K130T, K135R), (SY160(S3C)) and SakSTAR(S3C,K35A, E65Q,K74R,E80A,D82A,T90A,E99D, T101S,E108A,K109A, K130T,K135R), (SY161(S3C)), were constructed by the spliced overlap extension polymerase chain reaction (SOE-PCR) (24) using pMEX.SakSTAR encoding SakSTAR as template, two fragments were amplified by PCR (30 cycles: 1 sec at 94°C, 1 sec at 50°C, 10 sec at 72°C), the first one starting from the 5' end (primer 818A) of the staphylokinase gene to the region to be mutagenized (forward primer), the second one from this same region (backward primer) to the 3' end of the gene with primer 818D (5' CAAACAGCCAAGCTTCATTCATTCAGC). The forward and backward primers shared an overlap of around 24 bp. The two purified fragments were then assembled together in a second PCR reaction with the external primers 818A and 818D (30 cycles: 1 sec at 94°C, 1 sec at 50°C, 10 sec at 72°C). The amplified product from this final reaction was purified, digested with EcoRI and HindIII and ligated into the corresponding site of pMEX.SakSTAR. For each construction, the sequence of the variant was confirmed by sequencing the entire SakSTAR coding region.

### 4. Expression and purification of SakSTAR variants

The SakSTAR variants were expressed and purified, as described below, from transformed E. coli grown in terrific broth (TB) medium (28). A 2 to 4 mL aliquot of an overnight saturated culture in LB medium was used to inoculate a 1 to 2 L culture in terrific broth supplemented with 100 µg/mL ampicillin. The culture was incubated with vigorous aeration and at 30°C. After about 16 hours incubation, IPTG (200 µmol/L) was added to the culture to induce expression from the tac promoter. After 3 hours induction, the cells were pelleted by centrifugation at 4,000 rpm for 20 min, resuspended in 1/10 volume of 0.01 mol/L phosphate buffer pH 6-6.5 and disrupted by sonication at 0°C. The suspension was centrifuged for 20 min at 20,000 rpm and the supernatant was stored at 4°C or at -20°C until purification. The material was purified essentially as described above (Example 2): cleared cell lysates containing the SakSTAR variants were subjected to chromatography on a 1.6 x 5 cm column of SP-Sephadex, followed by chromatography on a 1.6 x 8 cm column of phenyl-Sepharose. The SakSTAR containing fractions, localized by SDS-gel electrophoresis, were pooled for further analysis.

### 5. Biochemical analysis

Protein concentrations were determined according to Bradford (29). SDS-PAGE was performed with the Phast System™ (Pharmacia, Uppsala, Sweden) using 10-15% gradient gels and Coomassie Brillant blue staining, and the specific activities of SakSTAR solutions were determined with a chromogenic substrate assay carried out in microtiter plates (as described in example 2).

### 6. Chemical crosslinking of cysteine mutants of SakSTAR with polyethylene glycol

The thiol group of the cysteine mutants was targeted for coupling with an activated polyethylene glycol, either OPSS-PEG or MAL-PEG (Shearwater Polymers Europe, Enschede, The Netherlands). OPSS-PEG is a 5 kDa PEG molecule carrying a single activated thiol group at one end that reacts specifically at slightly alkaline pH with free thiols. MAL-PEG is a 5 kDa, 10 kDa or 20 kDa molecule carrying a maleimide group that reacts specifically with thiol groups under mild conditions in the presence of other functional groups. Modification of the variants was achieved by incubating the molecule (100 µM) with a three-fold excess of OPSS-PEG or MAL-PEG in a 5 mM phosphate, pH 7.9 solution at room temperature. After reaction (about 15 min), the excess of OPSS-PEG or MAL-PEG was removed by purifying the derivatized SakSTAR variant on a 1.6 x 5 cm column of SP-Sephadex as described above (see Example 2). The "pegylated" SakSTAR variant containing fractions, localized by optical density at 280 nm, were pooled for further analysis. SDS-PAGE analysis and Coomassie blue staining confirmed that PEG crosslinking was quantitative. As shown in Table 20, the specific activities of the PEG-derivatives were only marginally affected when compared to that of wild-type staphylokinase.

### 7. Fibrinolytic properties of SakSTAR variants in human plasma in vitro

The fibrinolytic and fibrinogenolytic properties of SakSTAR variants were determined as previously described. Dose- and time-dependent lysis of ¹²⁵I-fibrin labeled human plasma clots submerged in human plasma was obtained with all molecules tested. Equi-effective concentrations of test compound (causing 50% clot lysis in 2 hrs; C₅₀), determined graphically from plots of clot lysis at 2 hrs versus the concentration of plasminogen activator (not shown), were comparable to or only slightly lower than that of SakSTAR (Table 20). The C₅₀ for clot lysis by variants derivatized with P20 (PEG with Mᵣ 20 kDa) was about twice as high as the non-derivatized variants. Thus increasing the size of the molecule via PEG-derivatization does not markedly affect the fibrinolytic activity of staphylokinase. The PEG-molecules appear to reduce the diffusion and therefore fibrinolytic potency of the derivatized staphylokinase within a fibrin clot, but this appears to be less pronounced with variants substituted in their NH₂-terminal region - which is released during processing of staphylokinase - than with variants substituted in the core of the molecule (cfr. Tables 19 and 20).

### 8. Pharmacokinetic properties of SakSTAR variants chemically modified with polyethylene glycol following bolus injection in hamsters

The pharmacokinetic parameters of the disposition of the pegylated variants from blood were evaluated in groups of 4 hamsters following intravenous bolus injection of 100 µg/kg SakSTAR variant. SakSTAR-related antigen was assayed using the ELISA described elsewhere. The ELISA was calibrated against each of the SakSTAR variants to be quantitated. Pharmacokinetic parameters included: initial half-life (in min), t1/2α = ln2/α; terminal half-life (in min), t1/2β = ln2/β; volume of the central (plasma) compartment (in mL), VC= dose/(A+B); area under the curve (in µg.min.mL⁻¹), AUC= A/α + B/β; and plasma clearance (in mL.min⁻¹), Clp= dose/AUC (32).

The disposition rate of staphylokinase-related antigen from blood following bolus injection of 100 µg/kg of the selected SakSTAR variants in groups of 4 hamsters could adequately be described by a sum of two exponential terms by graphical curve peeling (results not shown), from which the plasma clearances Clp, summarized in Table 20 were derived. The clearances of pegylated variants were markedly different from those of wild type SakSTAR and were inversely proportional to the molecular weight of the PEG molecules, with an average reduction of 5-fold with PEG 5 kDa, 10-fold with PEG 10 kDa and 30-fold with PEG 20 kDa. These results may be due to the increase of the Stokes radius of SakSTAR as a result of crosslinking with PEG.

### EXAMPLE 15

### Comparative thrombolytic efficacy and clearance of Sak-STAR(S3C-P20,E65D,K74R, E80A,D82A,K130T,K135R), (SY19(S3C-P20)). in two patients with acute myocardial infarction

### Large scale purification and conditioning of the SakSTAR variant for use in vivo

Material was purified to homogeneity out of culture volumes of 18 liters. The endotoxin content was below 1 IU/mg. Gel filtration on HPLC revealed a single main symmetrical peak in the chromatographic range of the column, representing >98% of the eluted material (total area under the curve) (not shown). SDS gel electrophoresis of a 30 µg sample revealed single main component. The preparation sterilized by filtration proved to be sterile on 3 day testing as described in methods. Intravenous bolus injection of the SakSTAR variant in 5 mice (3 mg/kg body weight), did not provoke any acute reaction, nor reduced weight gain within 8 days, in comparison with mice given an equal amount of saline (not shown).

Two patients with acute myocardial infarction were given a bolus injection of 5 mg SY19(S3C-P20). These patients had a complete recanalization of the occluded infarct-related artery as determined by coronary angiography at 90 min after the bolus injection. The material was cleared from the plasma with an initial half-life of 3 to 4 hours, as compared to 4 to 6 minutes for wild-type SakSTAR. These data confirm that pegylated variants of SakSTAR may be useful for thrombolytic therapy by single bolus injection at a reduced dose.

### CONCLUSION

In summary, the present invention shows that staphylokinase variants with markedly reduced antibody induction but intact thrombolytic potency can be generated. This observation constitutes the first case in which a heterologous protein, with the use of protein engineering techniques, is rendered significantly less immunogenic in man without reducing its biological activity. In addition, the present invention shows that selective chemical modification of staphylokinase or its variants with polyethylene glycol of varying molecular weights is feasible, resulting in a reduction of the plasma clearance proportional to the molecular weight. In the preferred embodiment an amino acid in the NH₂-terminal region of staphylokinase, the portion that is removed by processing, is substituted with Cys and the introduced thiol group is chemically modified with OPSS-PEG or MAL-PEG. This results in homogeneous products which, upon single intravenous bolus injection in experimental animals and in patients have a maintained thrombolytic potency at markedly reduced doses.

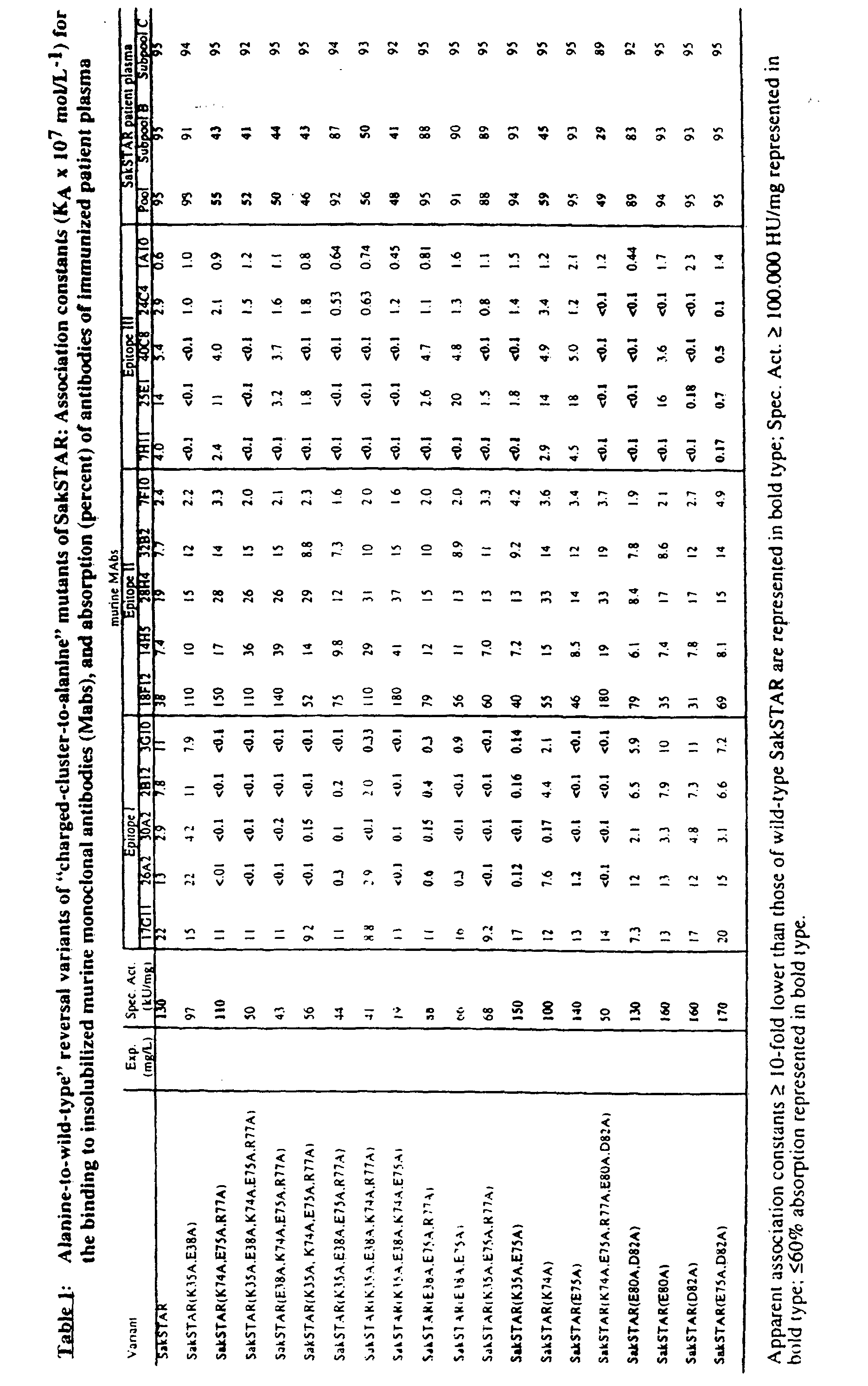

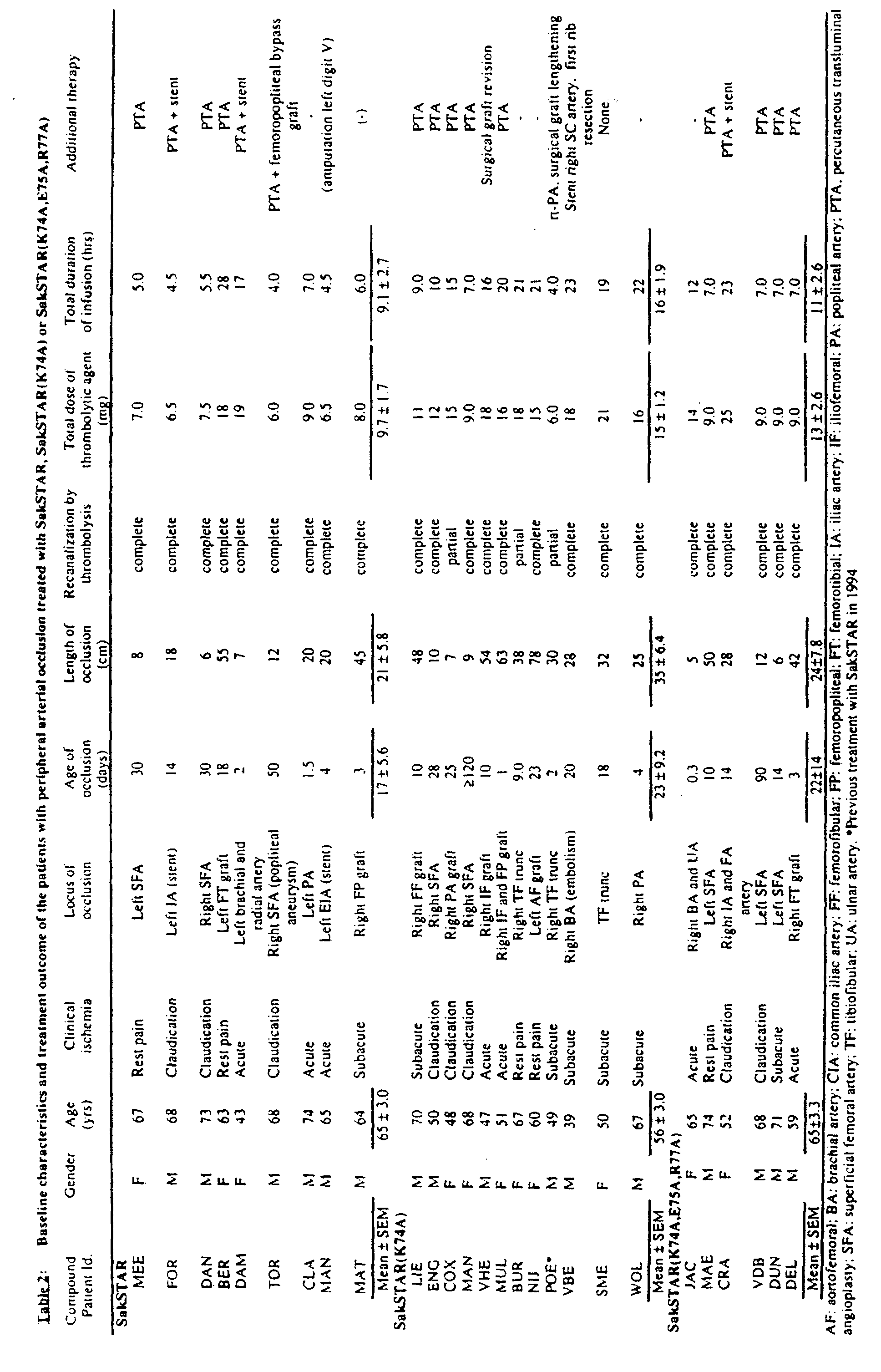

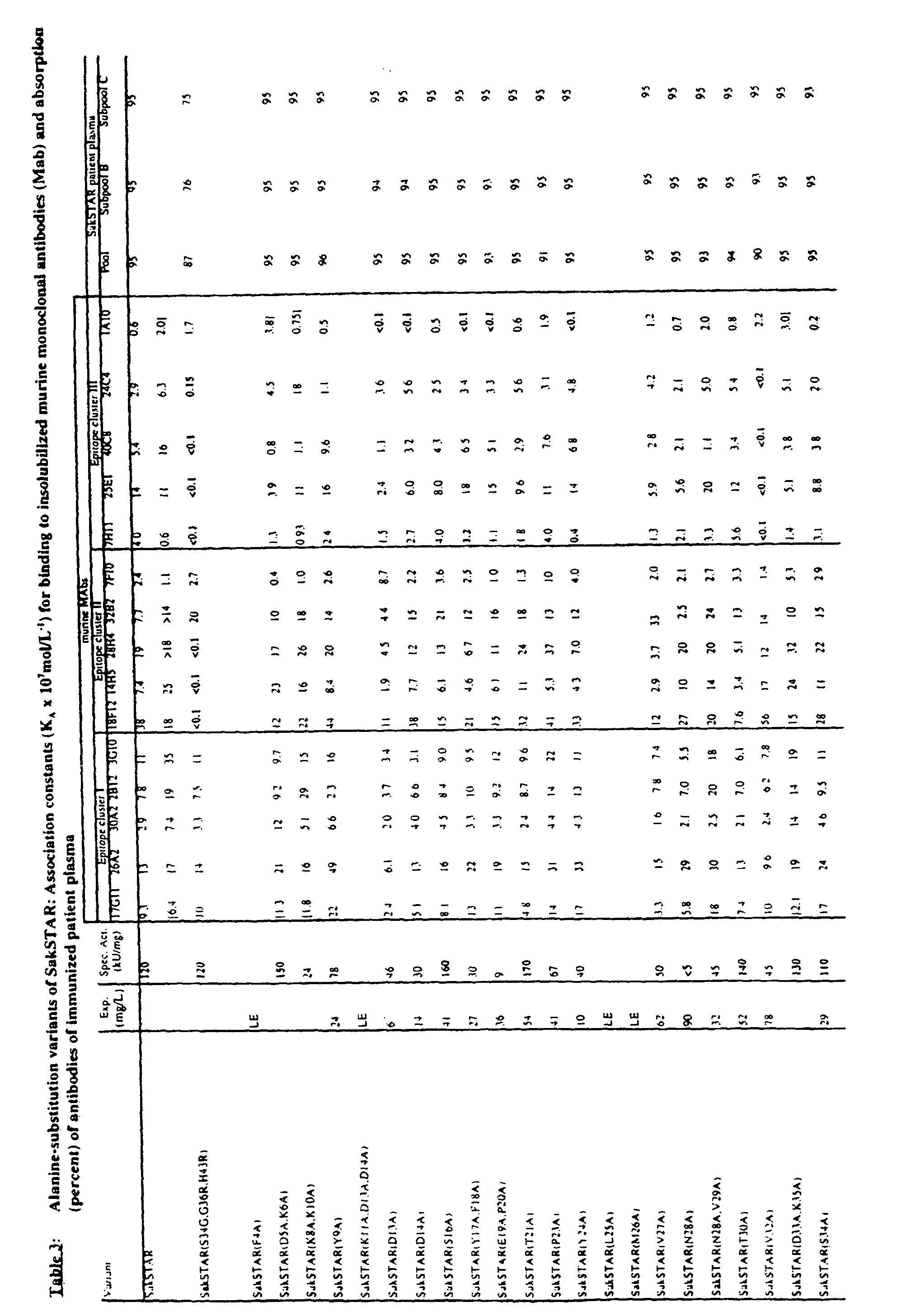

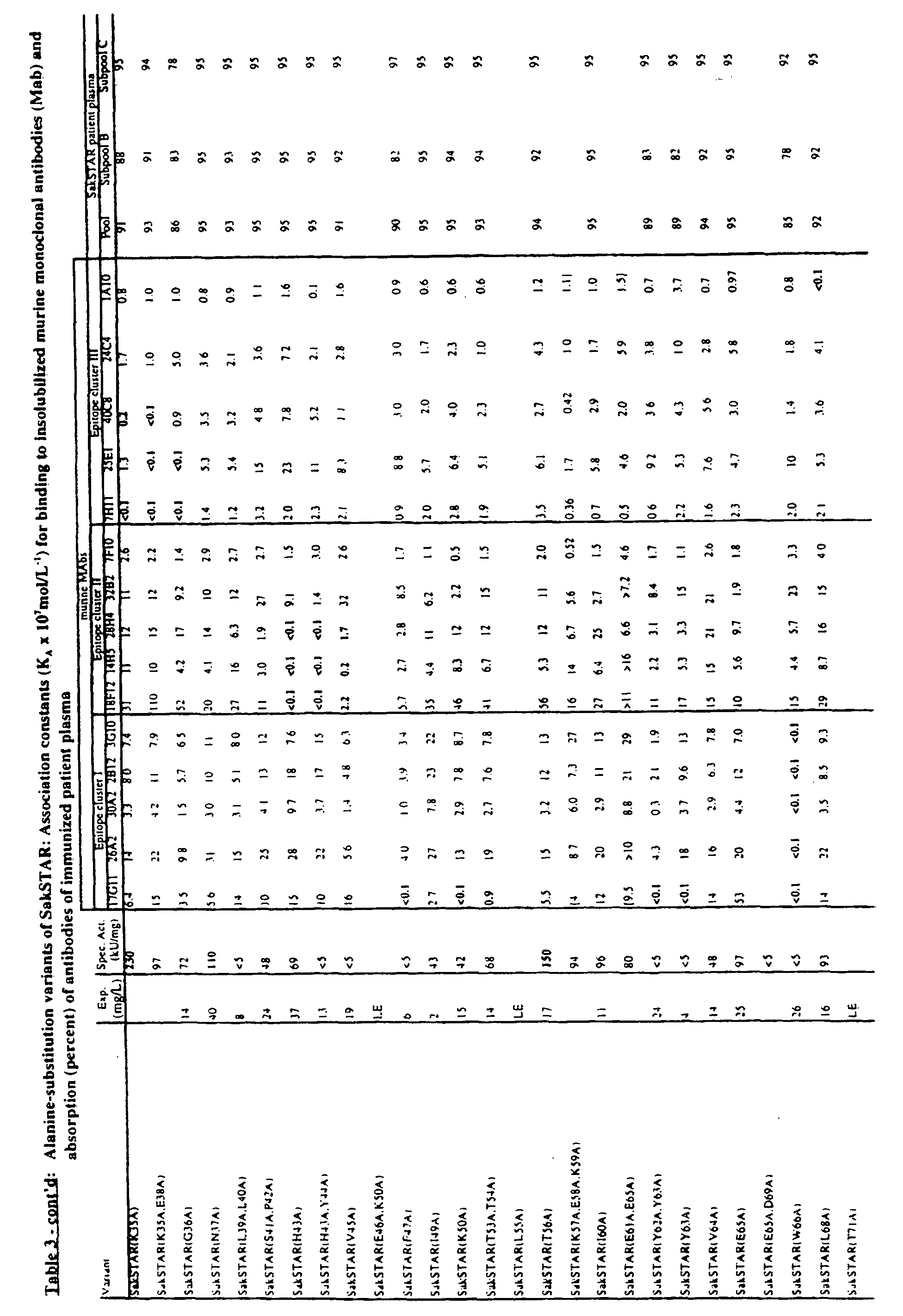

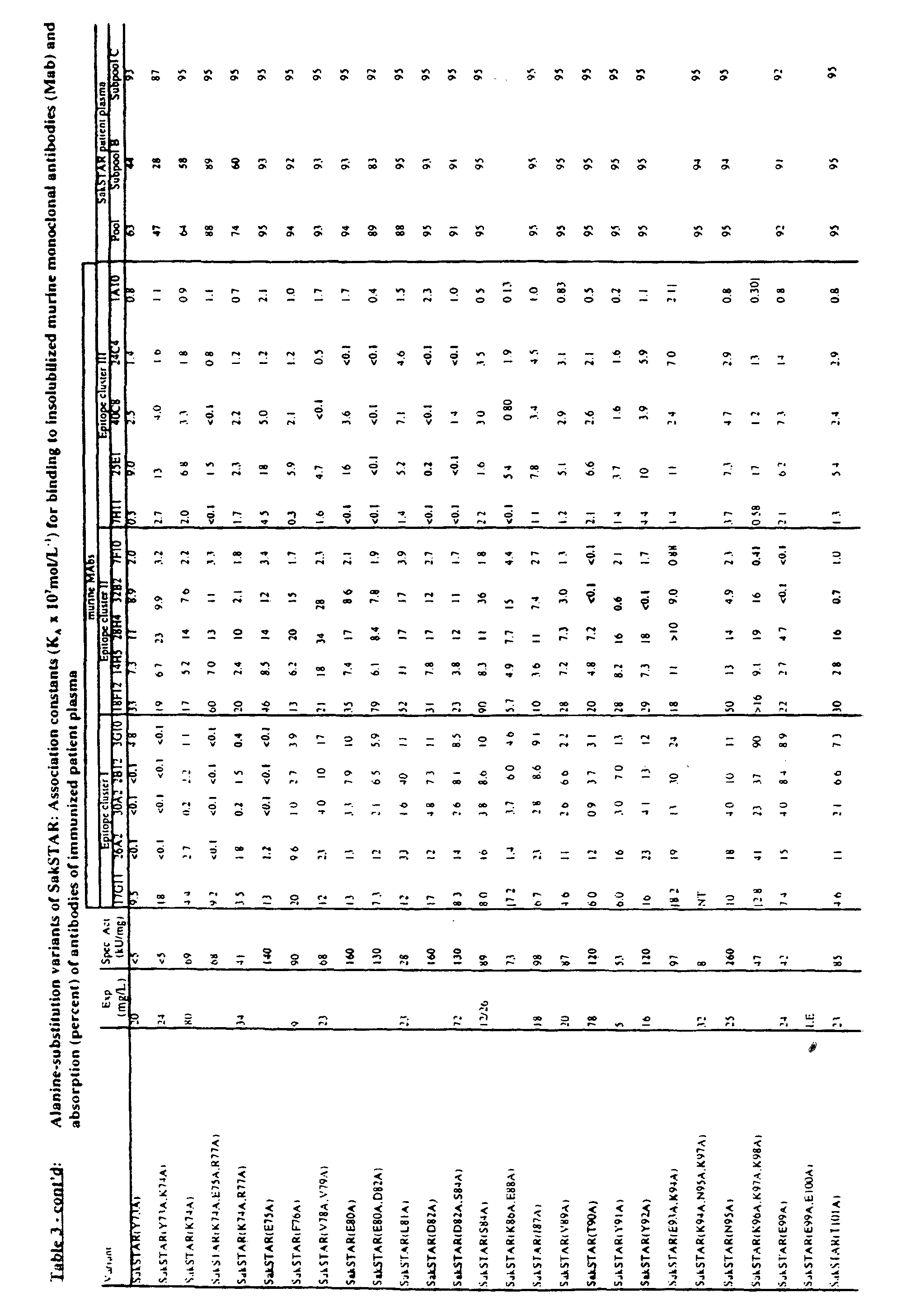

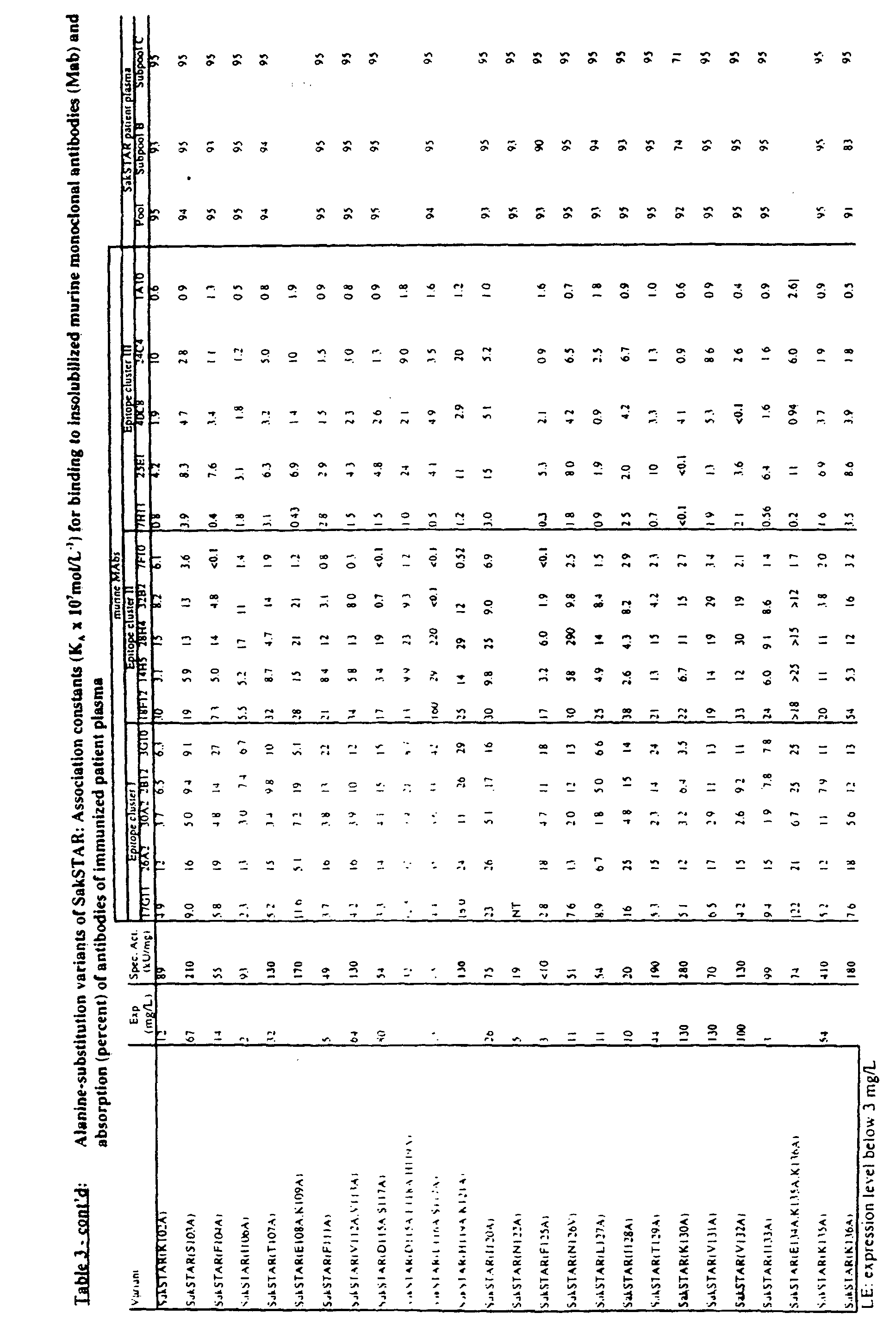

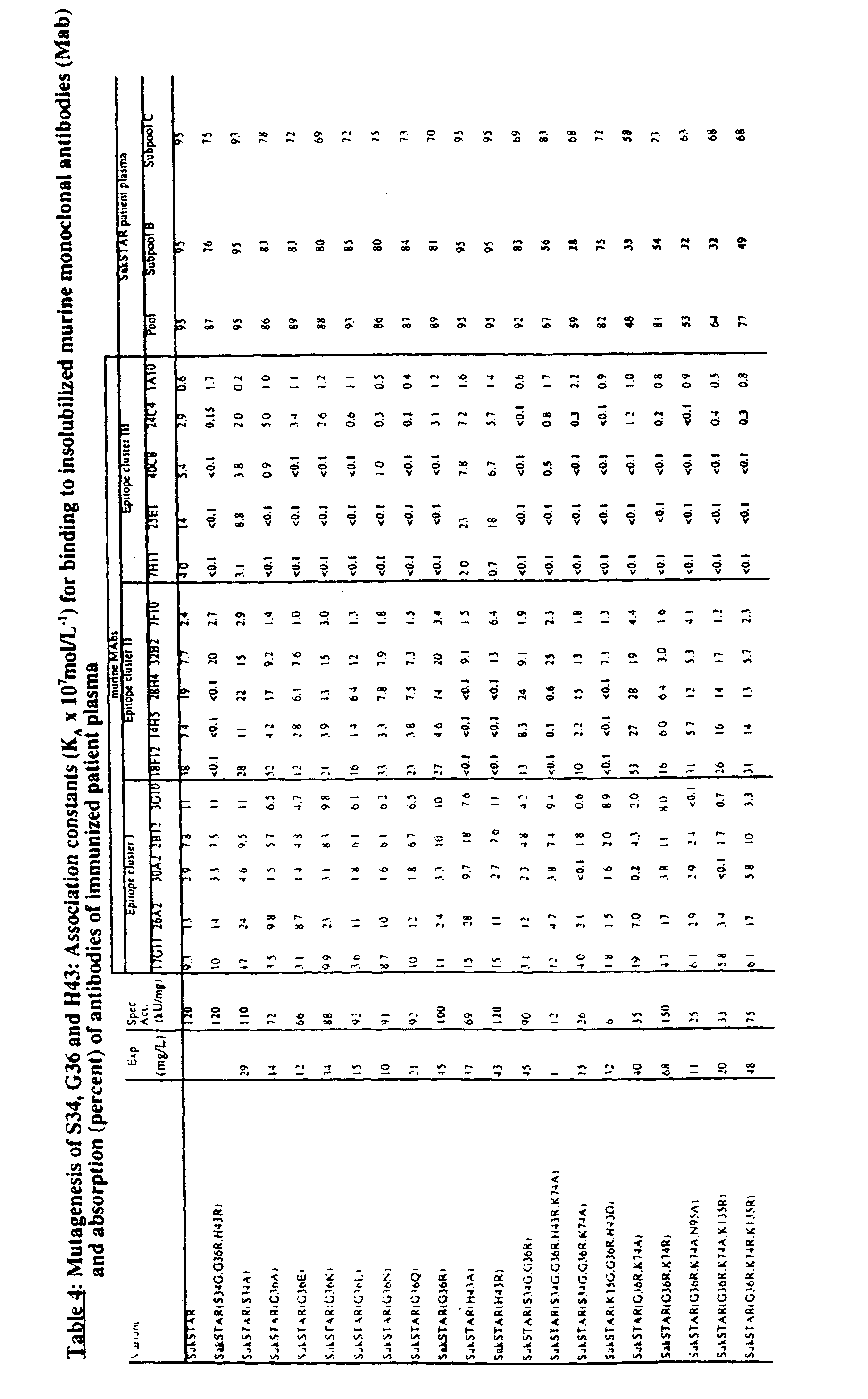

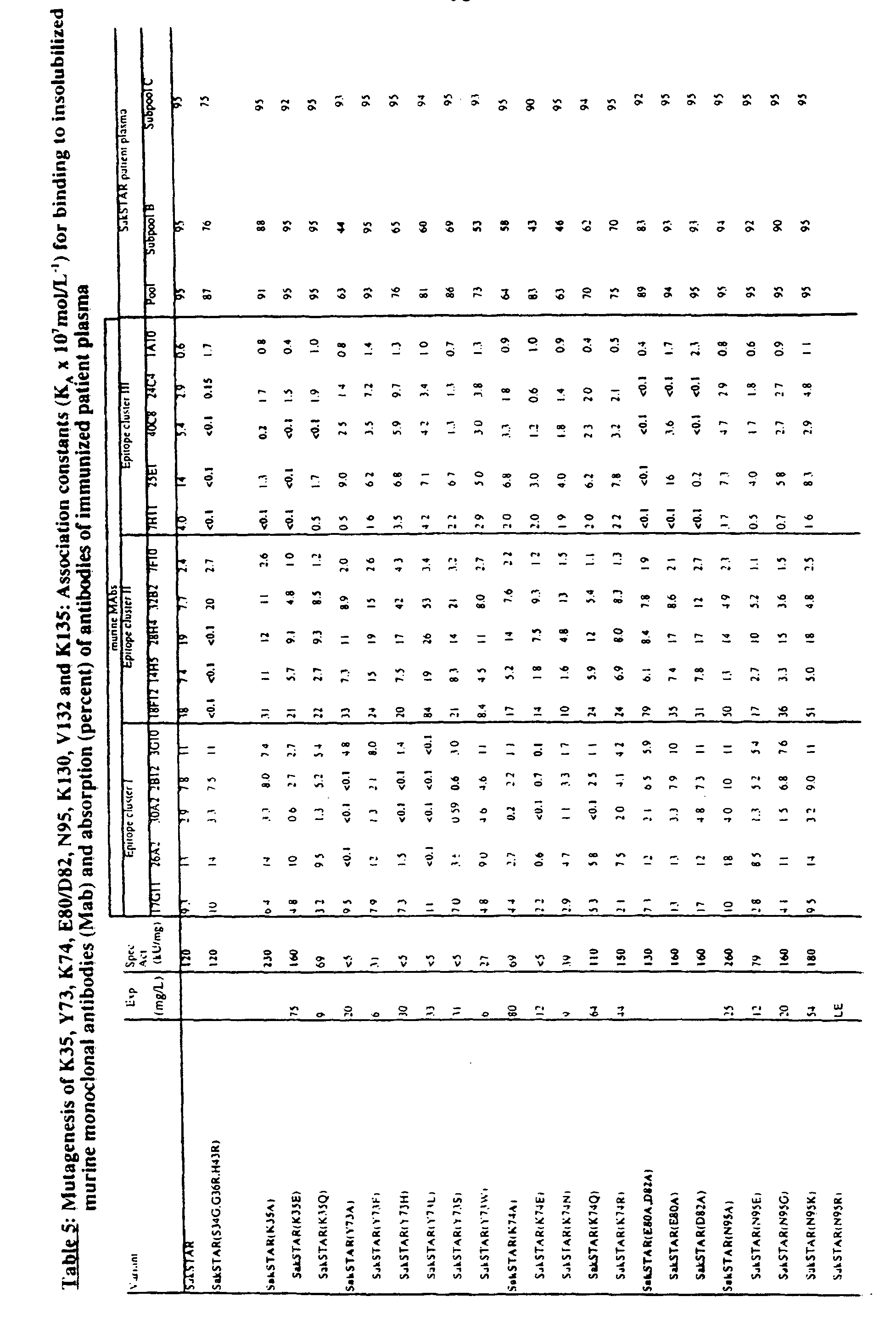

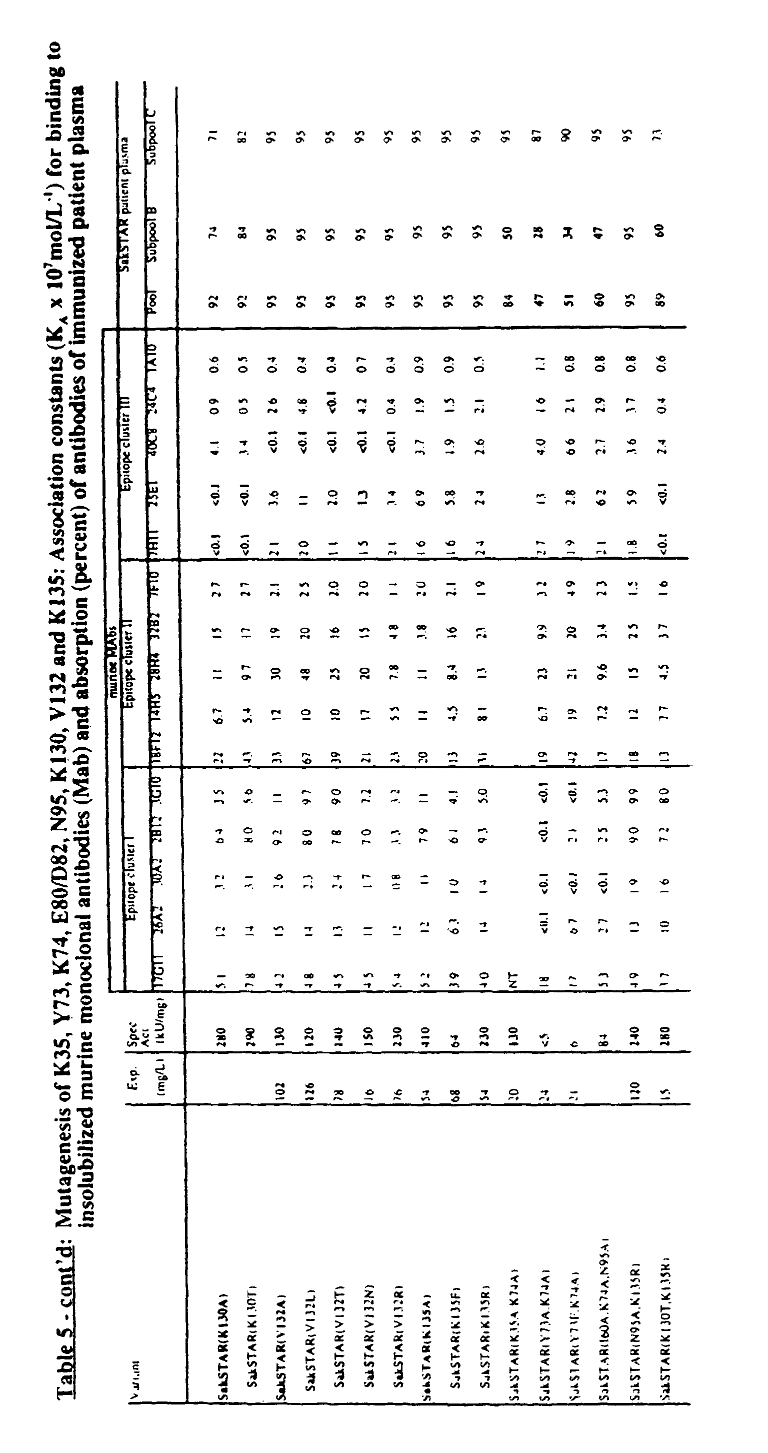

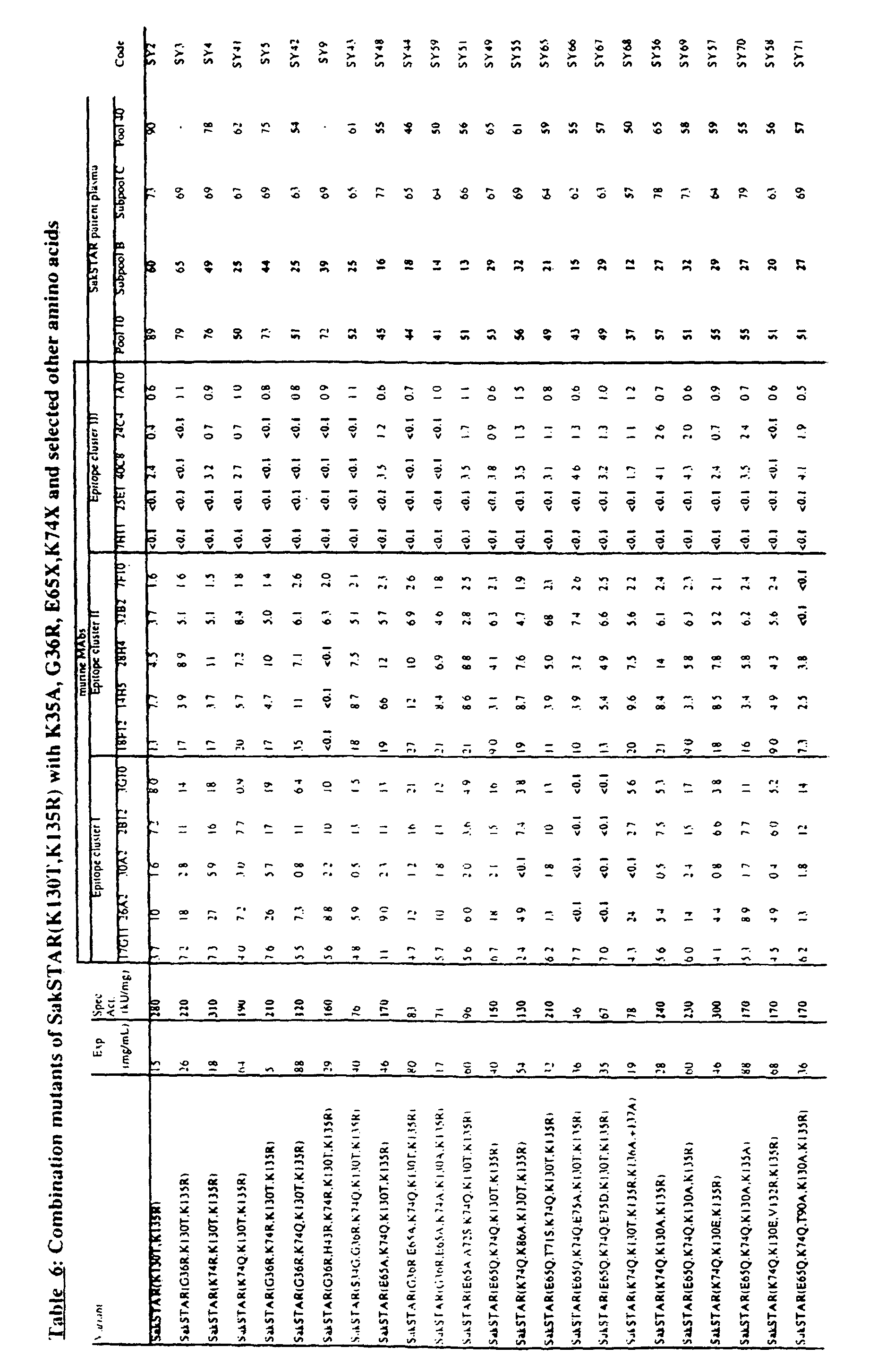

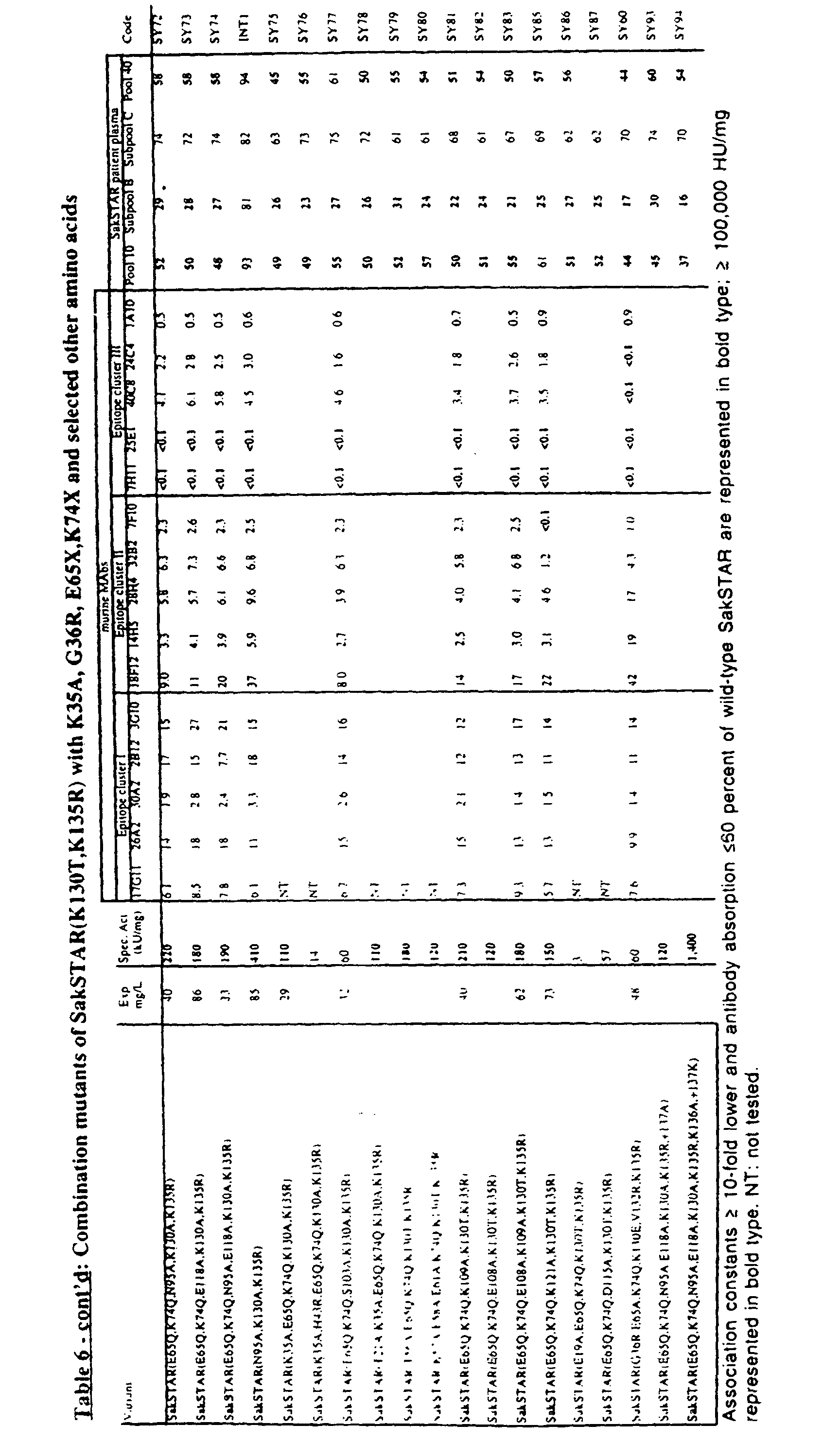

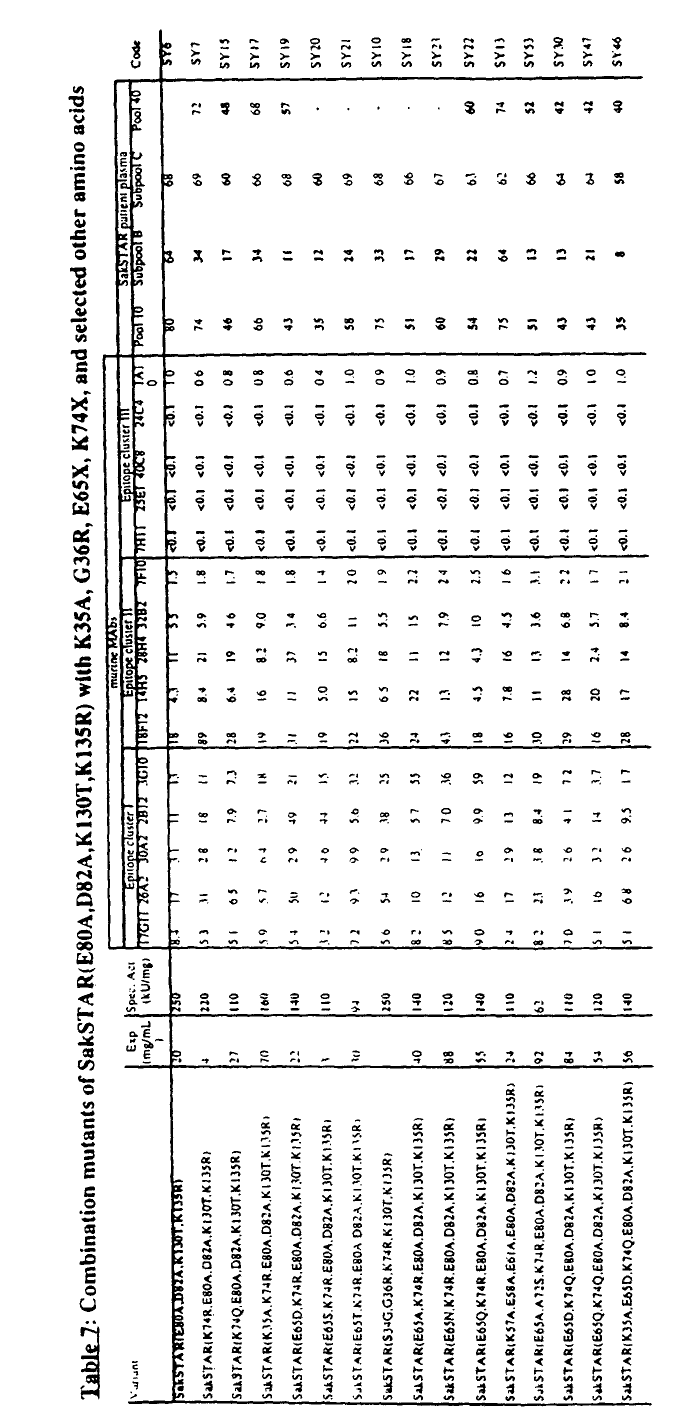

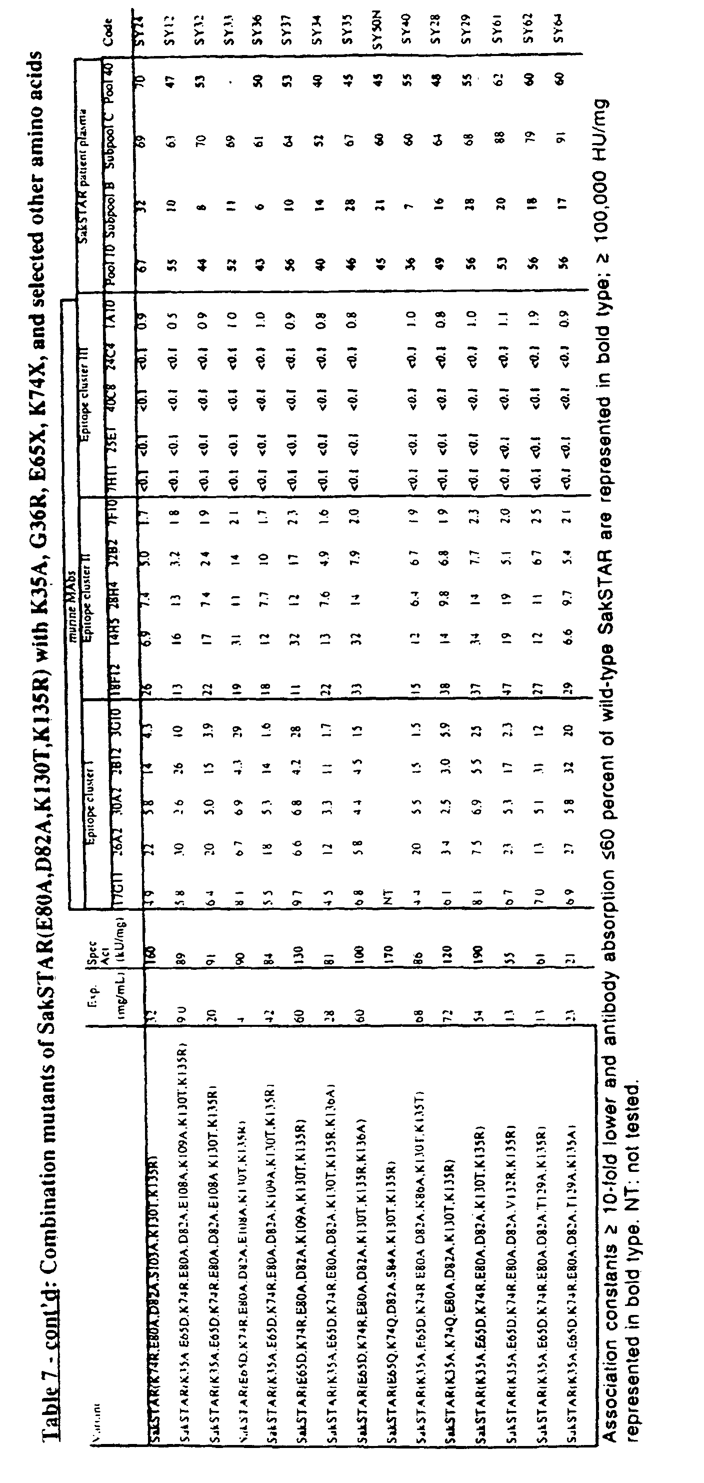

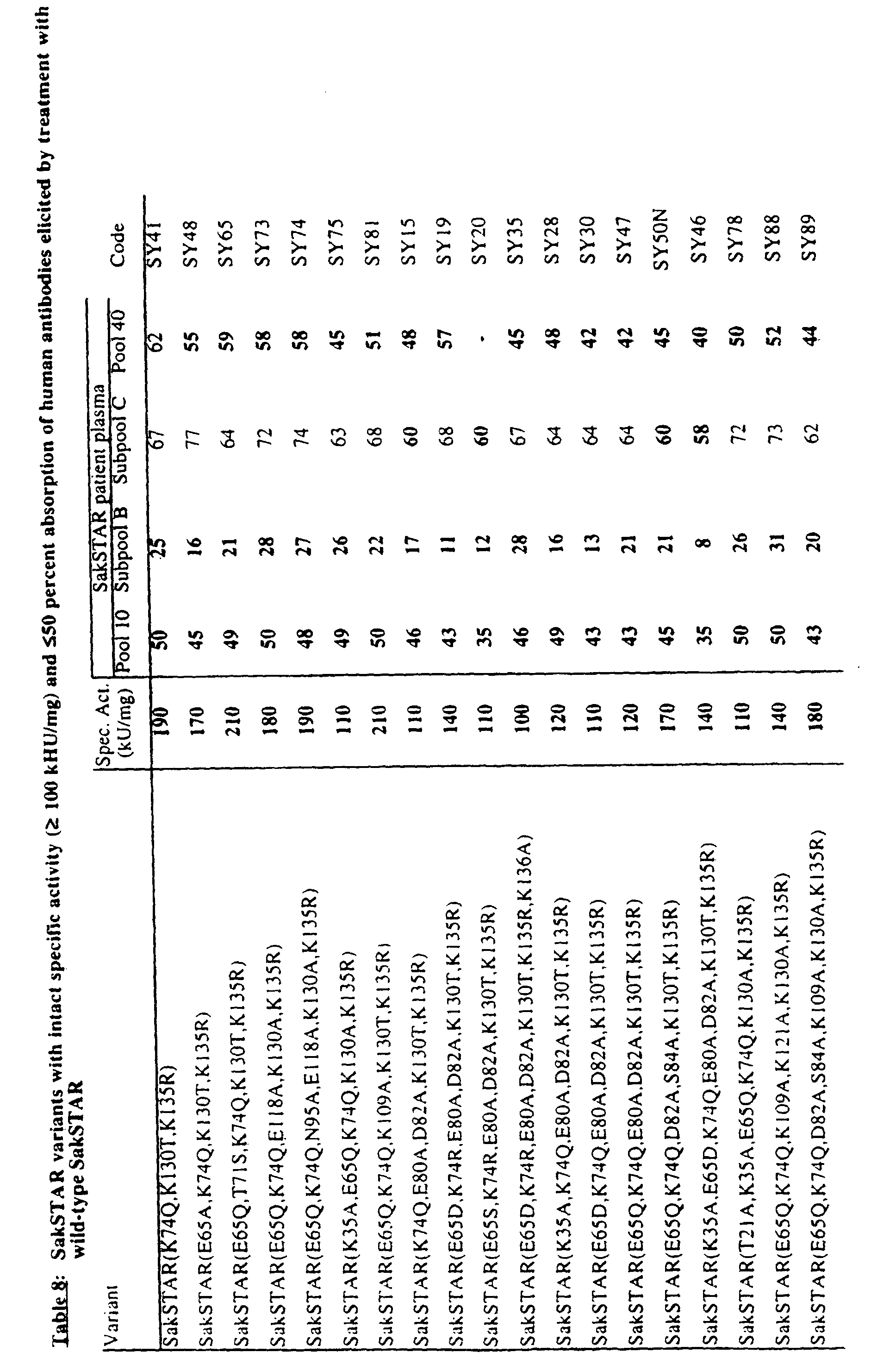

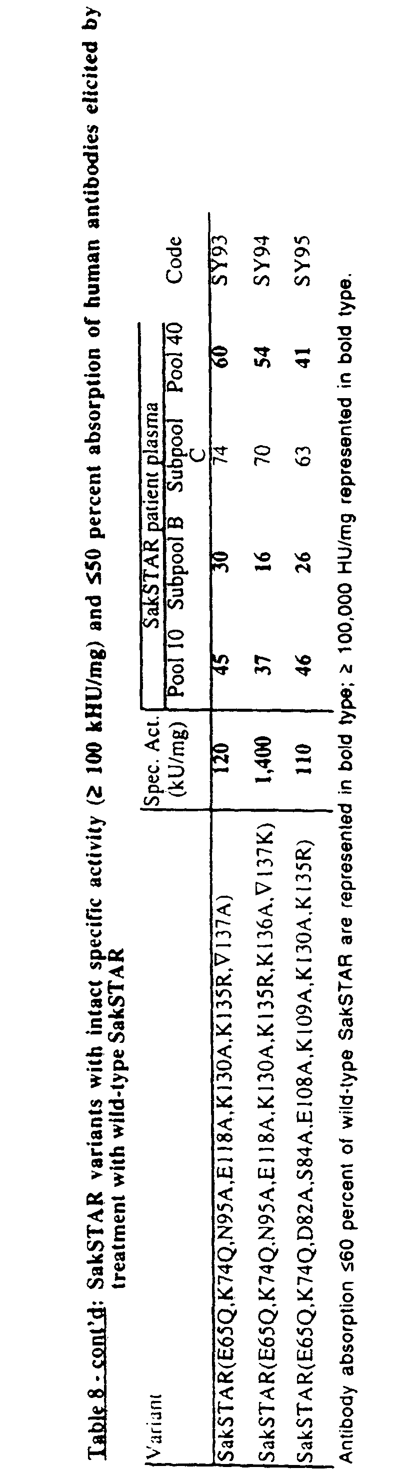

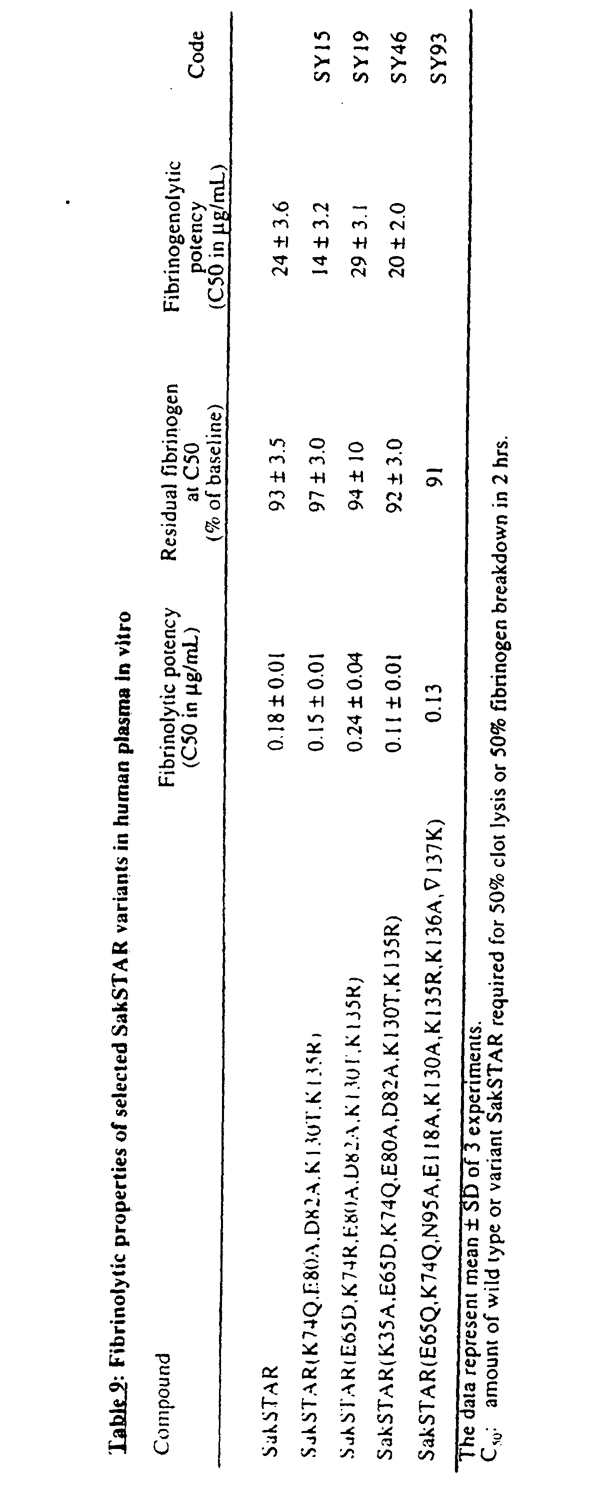

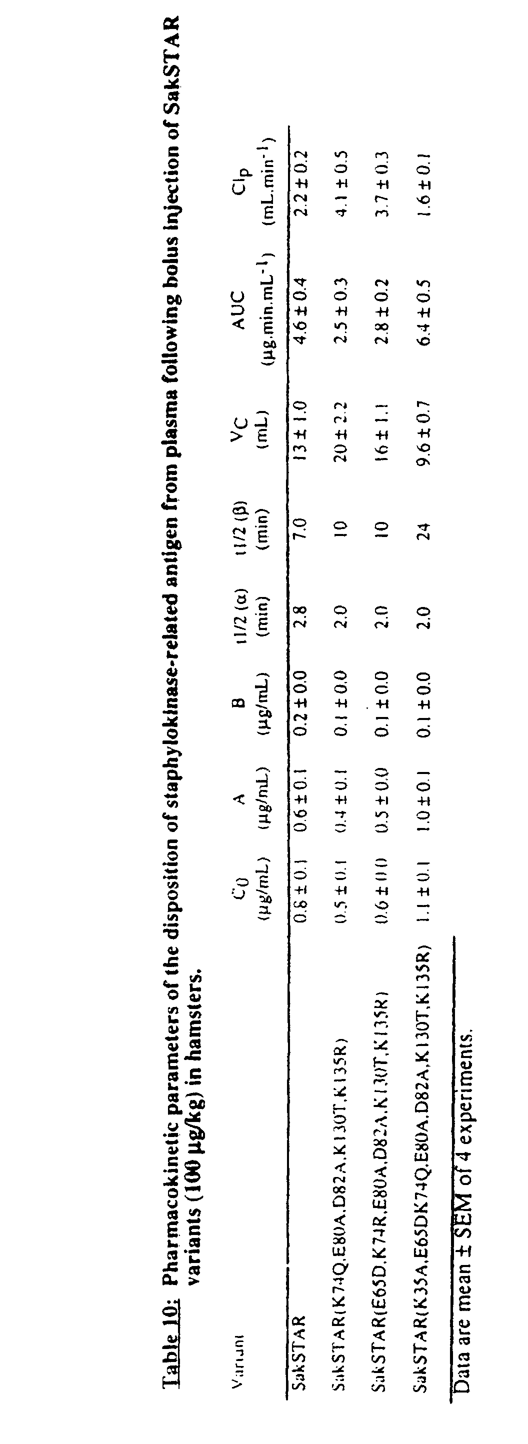

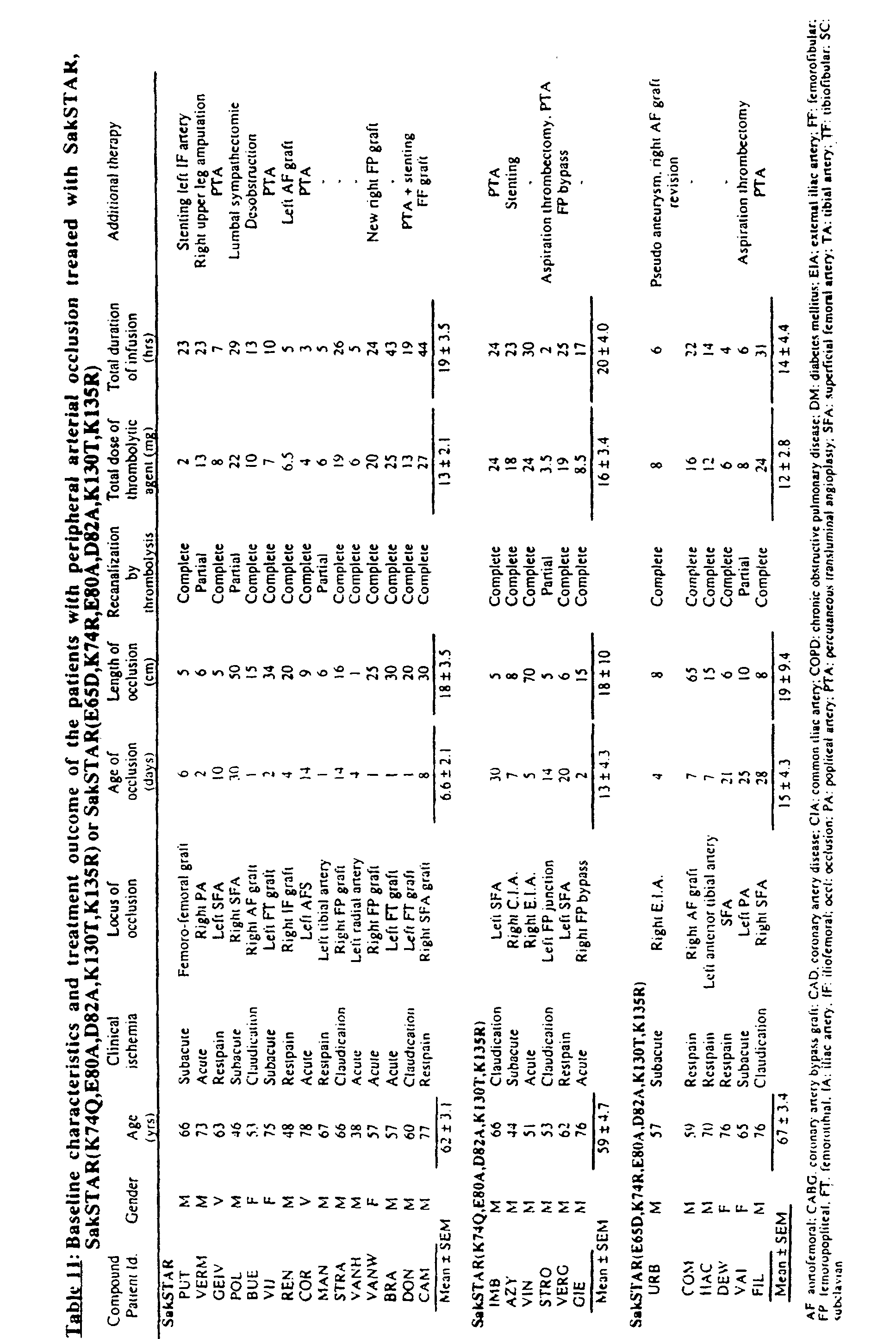

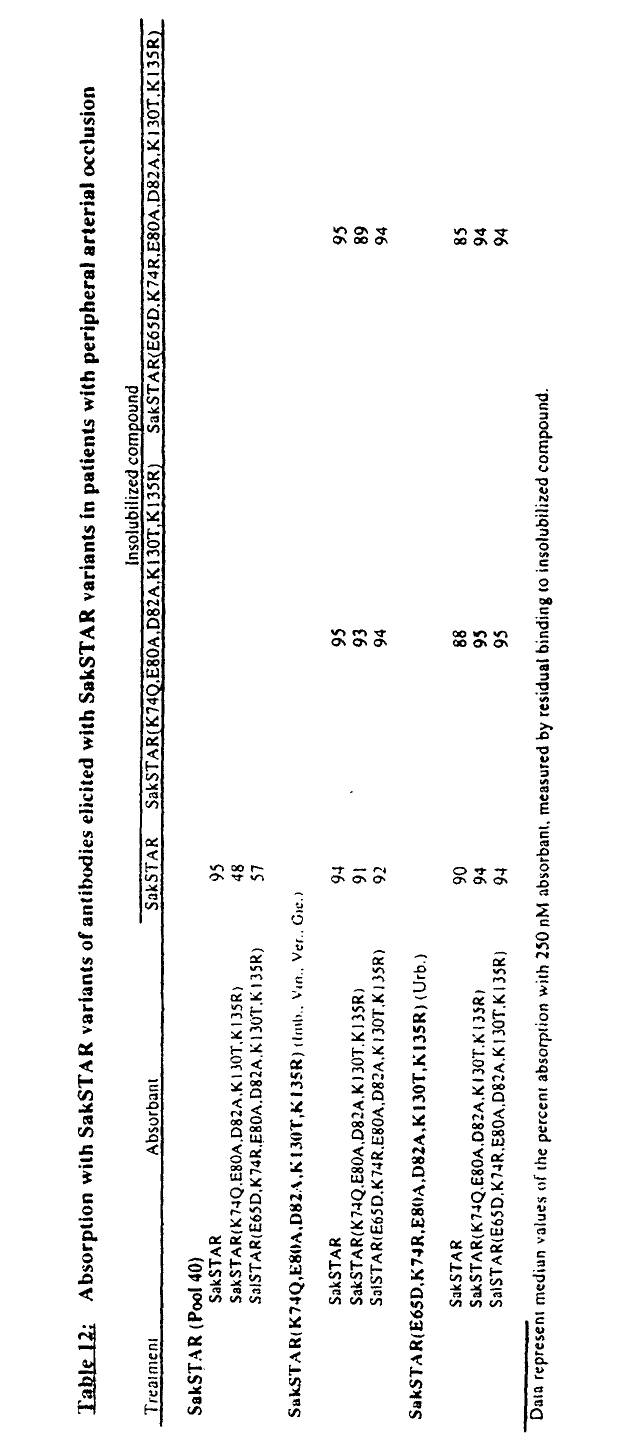

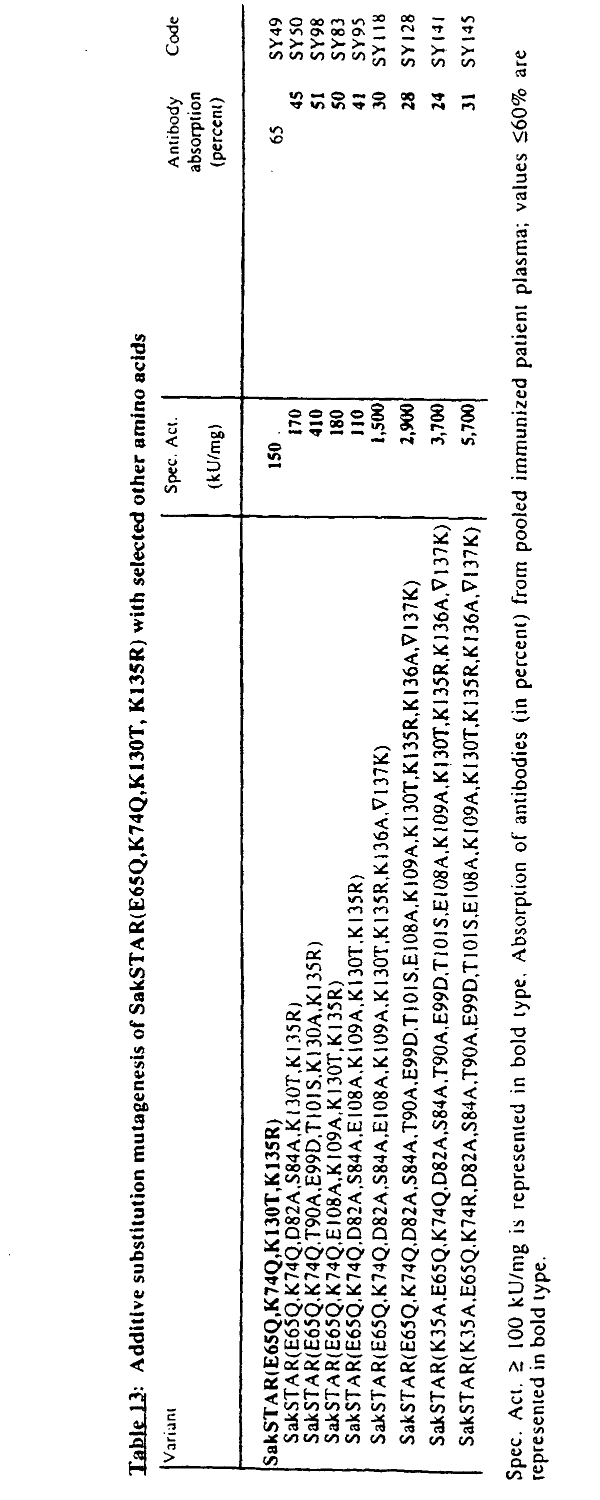

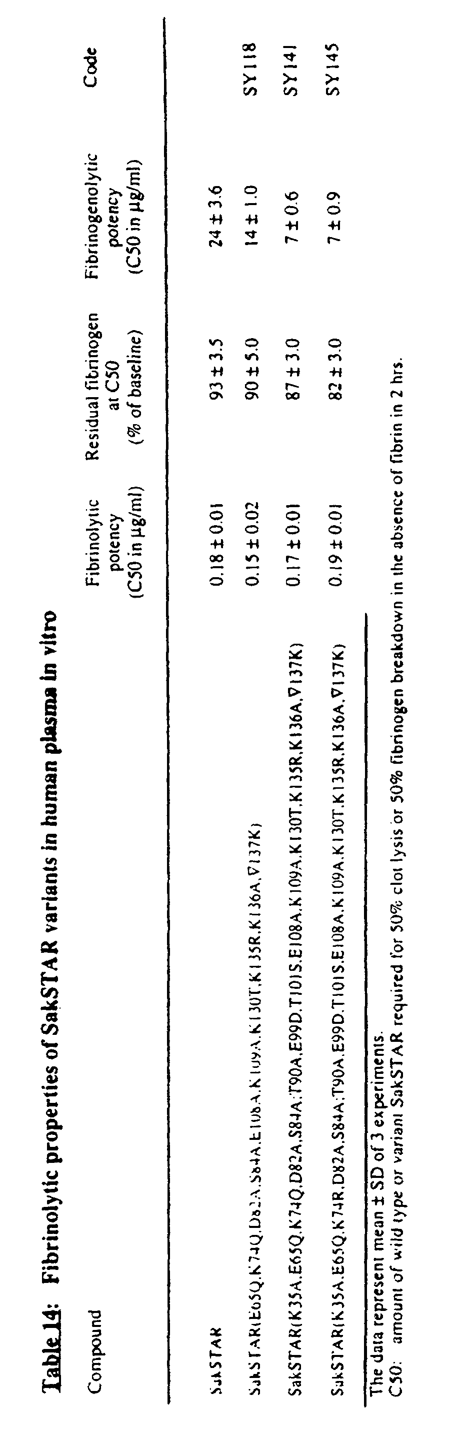

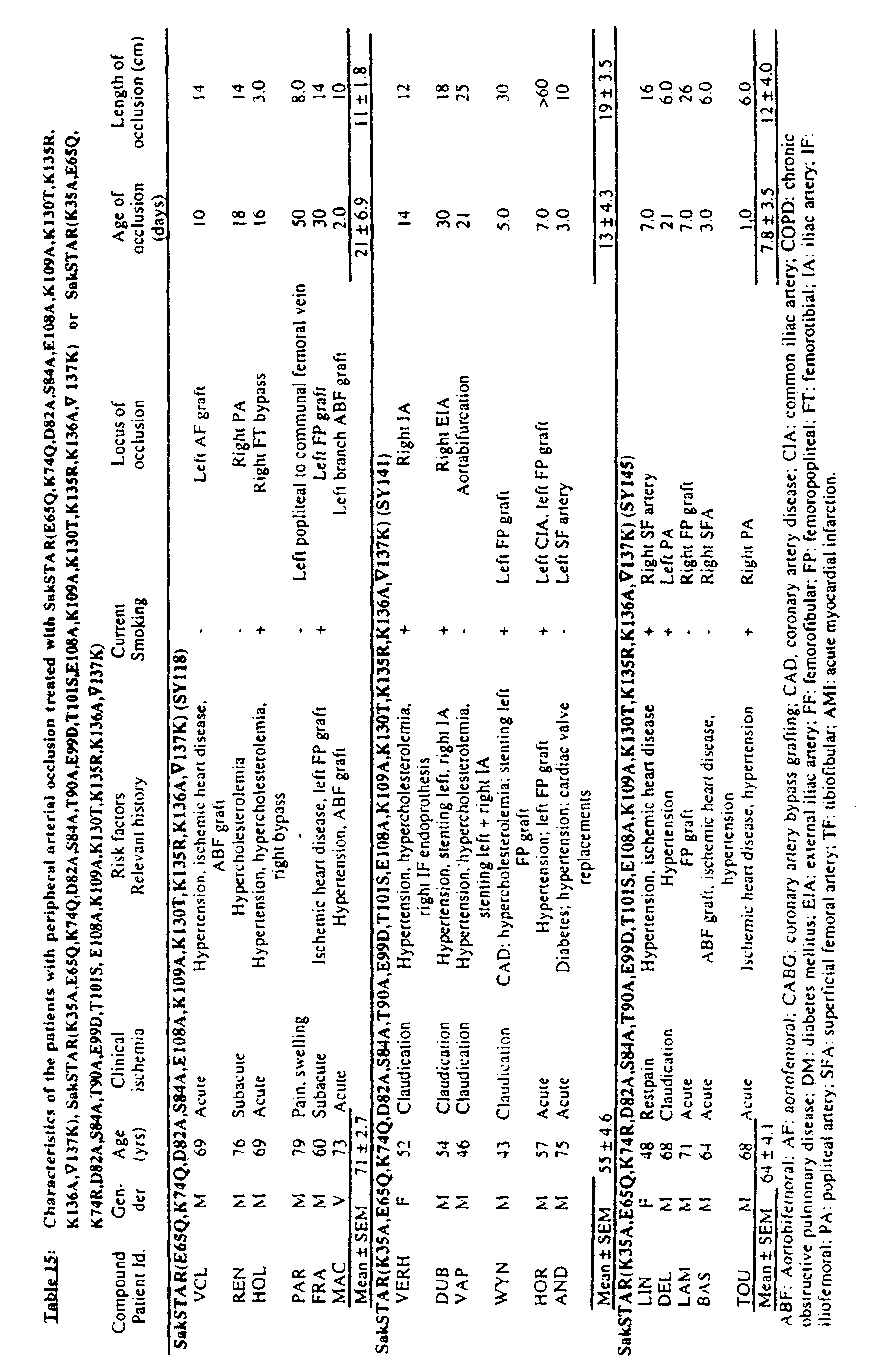

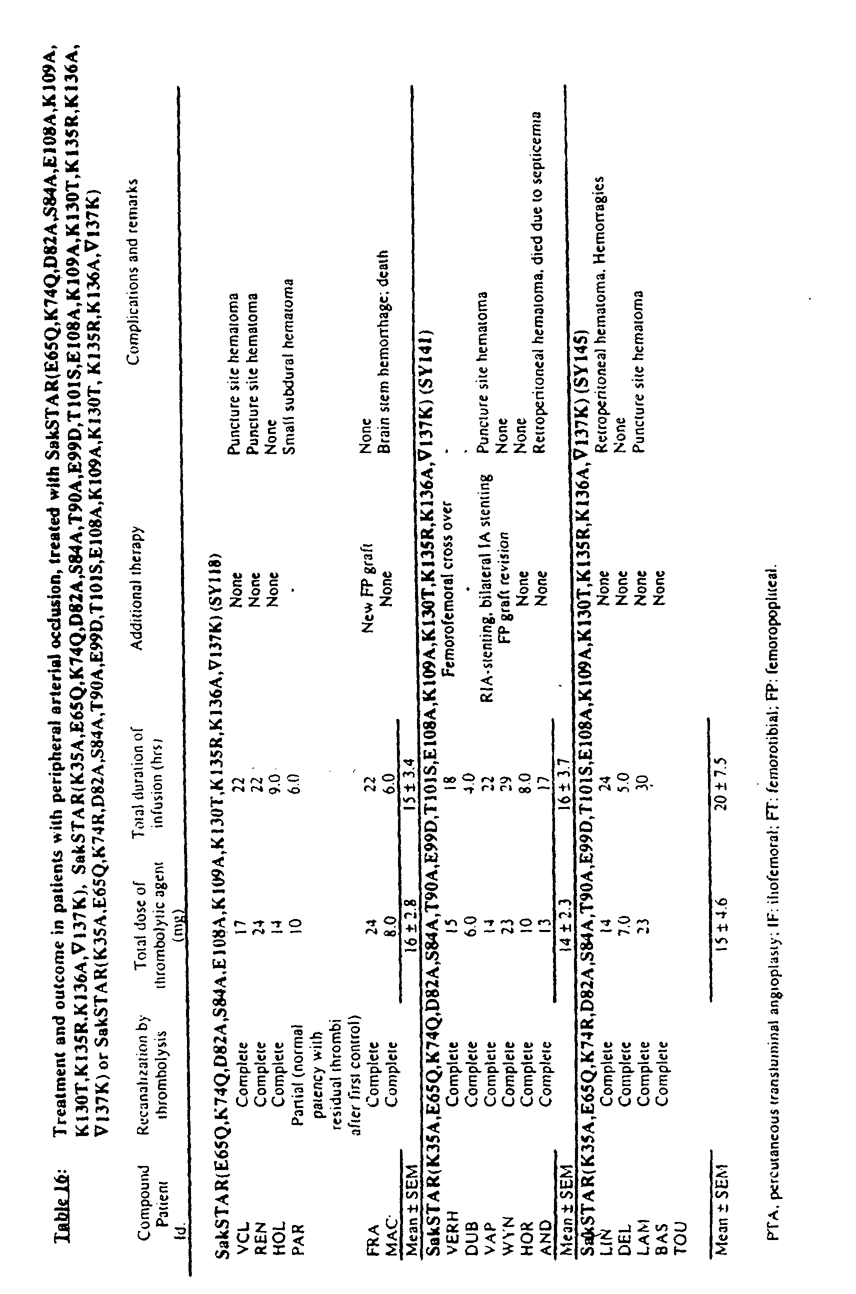

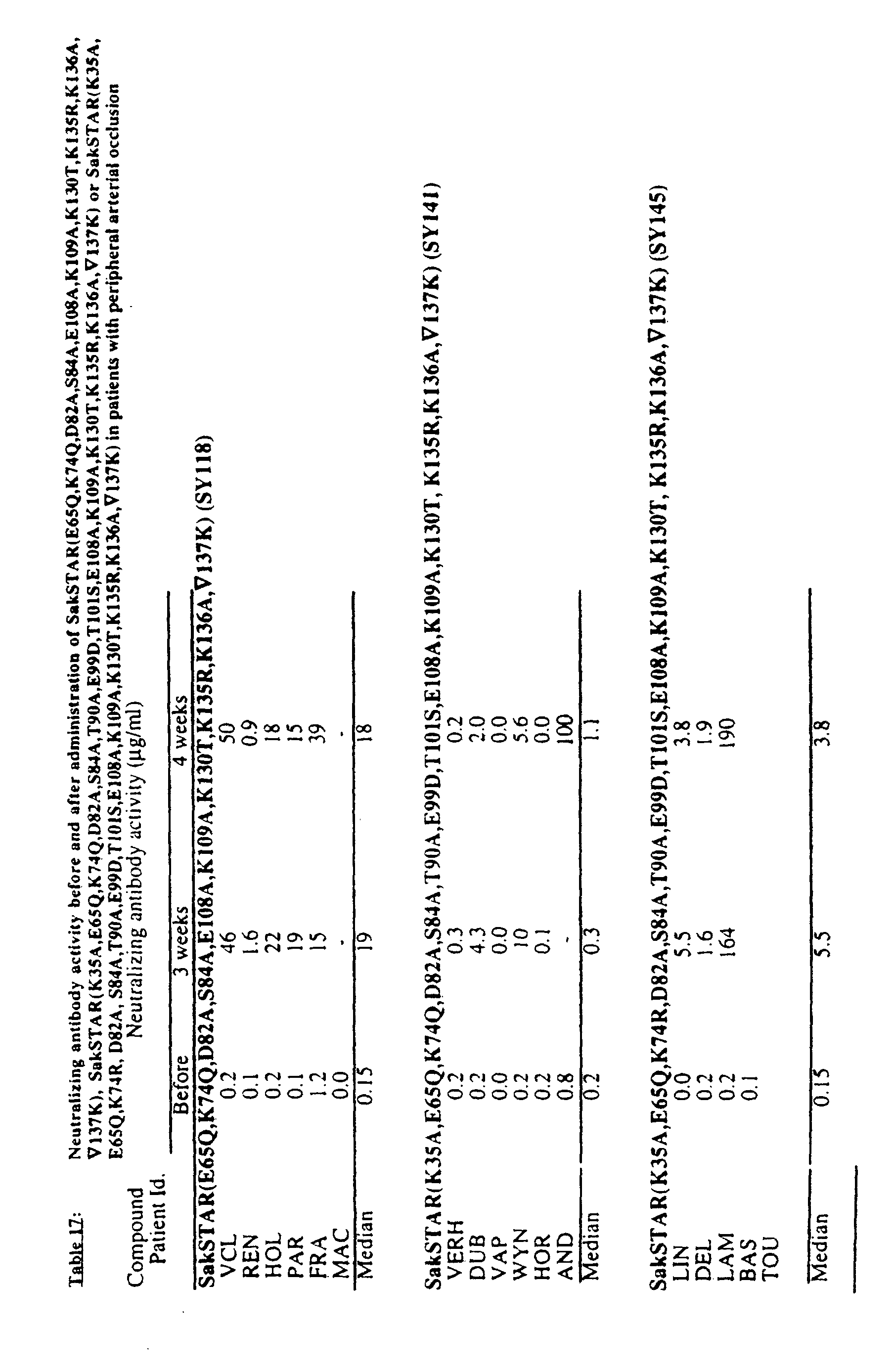

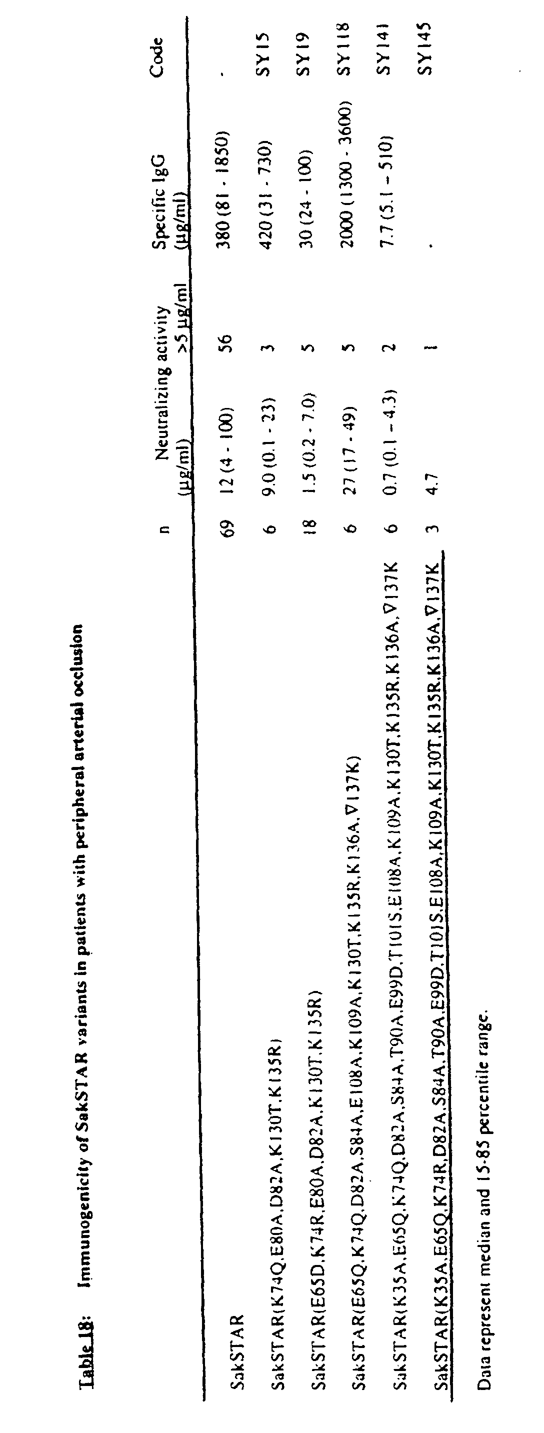

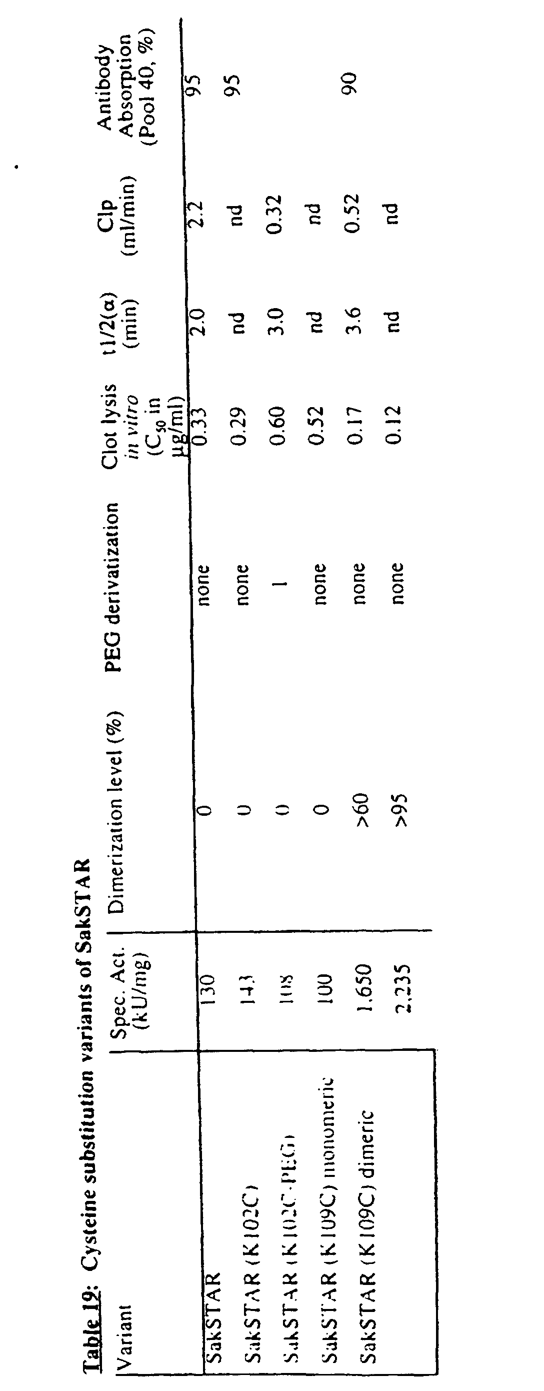

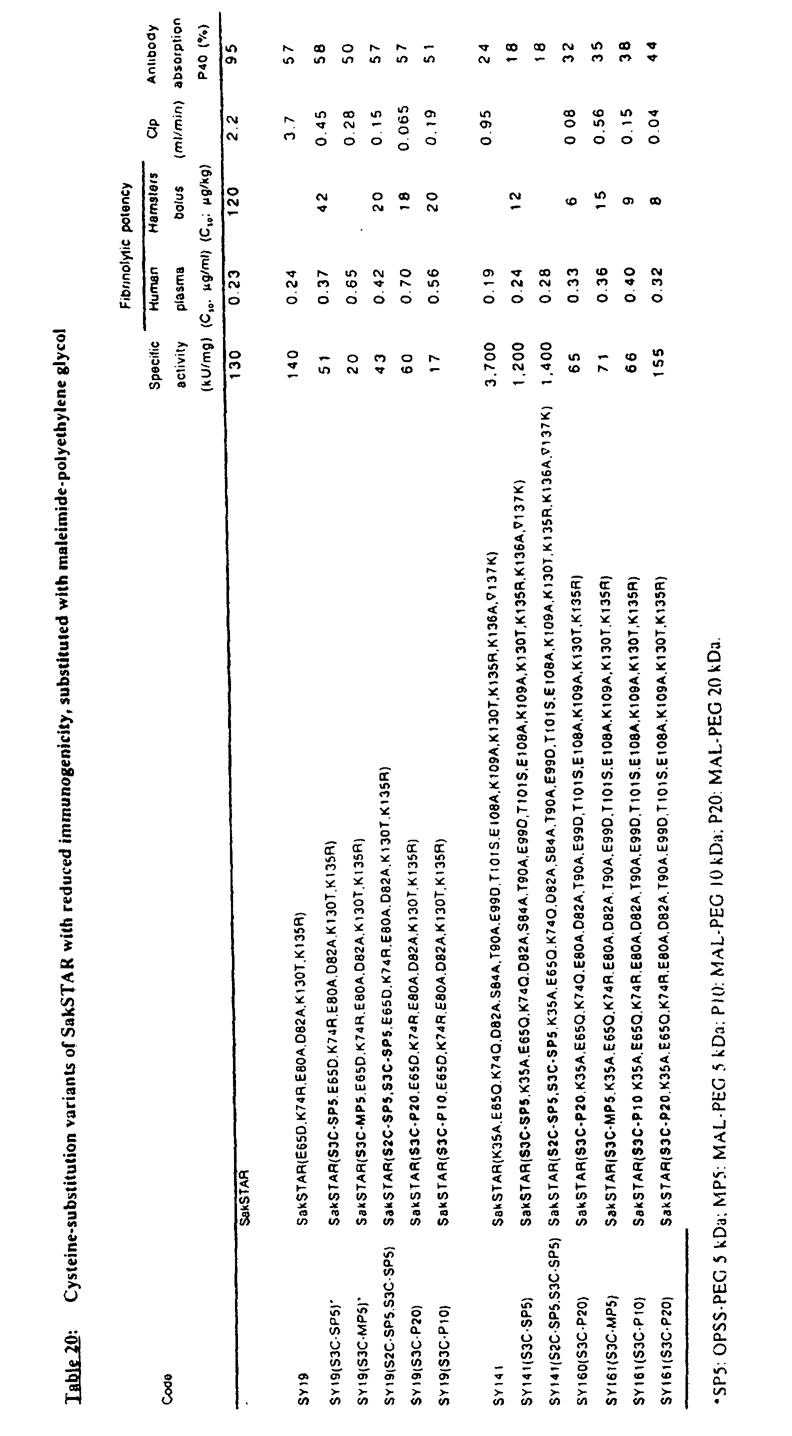

**REFERENCES**
1. Lack CH: Staphylokinase: an activator of plasma protease. Nature 161: 559, 1948.
2. Lewis JH, Ferguson JH: A proteolytic enzyme system of the blood. III. Activation of dog serum profibrinolysin by staphylokinase. Am J Physiol 166: 594, 1951.
3. US5336495 (issued 94.08.09).
4. Vanderschueren S, Barrios L, Kerdsinchai P, Van den Heuvel P, Hermans L, Vrolix M, De Man F, Benit E, Muyldermans L, Collen D, Van de Werf F: A randomized trial of recombinant staphylokinase versus alteplase for coronary artery patency in acute myocardial infarction. Circulation 92: 2044-2049, 1995.
5. Sako T, Sawaki S, Sakurai T, Ito S, Yoshizawa Y, Kondo I: Cloning and expression of the staphylokinase gene of Staphylococcus aureus in Escherichia coli. Molec Gen Genet 190: 271-277, 1983.
6. Behnke D, Gerlach D: Cloning and expression in Escherichia coli, Bacillus subtilis, and Streptococcus sanguis of a gene for staphylokinase - a bacterial plasminogen activator. Molec Gen Genet 210: 528-534, 1987.
7. Collen D, Silence K, Demarsin E, De Mol M, Lijnen HR: Isolation and characterization of natural and recombinant staphylokinase. Fibrinolysis 6: 203-213, 1992.
8. Sako T, Tsuchida N: Nucleotide sequence of the staphylokinase gene from Staphylococcus aureus. Nucleic Acids Res 11: 7679-7693, 1983.
9. Collen D, Zhao ZA, Holvoet P, Marynen P: Primary structure and gene structure of staphylokinase. Fibrinolysis 6: 226-231, 1992.
10. Sakai M, Watanuki M, Matsuo O: Mechanism of fibrin-specific fibrinolysis by staphylokinase: participation of α₂-plasmin inhibitor. Biochem Biophys Res Comm 162: 830-837, 1989.
11. Matsuo O, Okada K, Fukao H, Tomioka Y, Ueshima S, Watanuki M, Sakai M: Thrombolytic properties of staphylokinase. Blood 76: 925-929, 1990.
12. Lijnen HR, Van Hoef B, De Cock F, Okada K, Ueshima S, Matsuo O, Collen D: On the mechanism of fibrin-specific plasminogen activation by staphylokinase. J Biol Chem 266: 11826-11832, 1991.
13. Lijnen HR, Van Hoef B, Matsuo O, Collen D: On the molecular interactions between plasminogen-staphylokinase, α₂-antiplasmin and fibrin. Biochim Biophys Acta 1118: 144-148, 1992.
14. Silence K, Collen D, Lijnen HR: Interaction between staphylokinase, plasmin(ogen) and α₂-antiplasmin. Recycling of staphylokinase after neutralization of the plasmin-staphylokinase complex by α₂-antiplasmin. J Biol Chem 268: 9811-9816, 1993.
15. Silence K, Collen D, Lijnen HR: Regulation by α₂-antipiasmin and fibrin of the activation of plasminogen with recombinant staphylokinase in plasma. Blood 82: 1175-1183, 1993.
16. Sakharov DV, Lijnen HR, Rijken DC. Interactions between staphylokinase, plasmin(ogen), and fibrin. J Biol Chem 271: 27912-27918, 1996.
17. Schlott B, Ghrs KH, Hartmann M, Rcker A, Collen D. Staphylokinase requires NH₂-terminal proteolysis for plasminogen activation. J Biol Chem (in press).
18. Collen D, De Cock F, Vanlinthout I, Declerck PJ, Lijnen HR, Stassen JM. Comparative thrombolytic and immunogenic properties of staphylokinase and streptokinase. Fibrinolysis 6: 232-242, 1992.
19. Collen D, De Cock F, Stassen JM. Comparative immunogenicity and thrombolytic properties toward arterial and venous thrombi of streptokinase and recombinant staphylokinase in baboons. Circulation 87: 996-1006, 1993.
20. White H: Thrombolytic treatment for recurrent myocardial infarction. Br Med J 302: 429-430, 1991.
21. Gase A, Hartmann M, Ghrs KH, Rcker A, Collen D, Behnke D, Schlott B: Functional significance of NH₂- and COOH-terminal regions of staphylokinase in plasminogen activation. Thromb Haemost 76: 755-760, 1996.
22. EP 95200023.0 (January 6, 1995) and US 08/499,092 (July 6, 1995).
23. Schlott B, Hartmann M, Ghrs KH, Birch-Hirschfeid E, Pohl HD, Vanderschueren S, Van de Werf F, Michoel A, Collen D, Behnke D: High yield production and purification of recombinant staphylokinase for thrombolytic therapy. Bio/technology 12: 185-189, 1994.
24. Horton RM, Hunt HD, Ho SN, Pullen JK, Pease LR. Engineering hybrid genes without the use of restriction enzymes: gene splicing by overlap extension. Gene 77: 61-68, 1989.
25. BIAcore system manual, 5-2, Pharmacia Biosensor AB, Uppsala, Sweden.
26. Karlsson R, Michaelsson A, Mattsson L: Kinetic analysis of monoclonal antibody-antigen interactions with a new biosensor based analytical system. J Immunol Methods 145: 229-240, 1991.
27. Sambrook J, Fritsch EF, Maniatis T: Molecular cloning: a laboratory mannual. 2nd Ed. Cold Spring Harbor, NY. Cold Spring Harbor Laboratory Press, 1989.
28. Tartof KD, Hobbs CA: Improved media for growing plasmid and cosmid clones. Bethesda Res Lab Focus 9: 12, 1987
29. Bradford MM: A rapid and sensitive method for the quantitation of microgram quantities of protein utilizing the principle of protein-dye binding. Anal Biochem 72: 248, 1976.
30. Deutsch DG, Mertz ET: Plasminogen: purification from human plasma by affinity chromatography. Science 170: 1095-1096, 1970.
31. Collen D, Moreau H, Stockx L, Vanderschueren S. Recombinant staphylokinase variant with altered immunoreactivity. II. Thrombolytic properties and antibody induction. Circulation 94: 207-216, 1996.
32. Vanderschueren S, Stockx L, Wilms G, Lacroix H, Verhaeghe R, Vermylen J, Collen D: Thrombolytic therapy of peripheral arterial occlusion with recombinant staphylokinase. Circulation 92: 2050-2057, 1995.
33. Gibaldi M, Perrier D. Pharmacokinetics, Marcel Dekker, New York, N.Y., 1983, 45-111.
34. Inada Y, Furukawa M, Sasaki H, Kodera Y, Hiroto M, Nishimura H, Matsushima A. Biomedical and biotechnological applications of PEG- and PM-modified proteins, TIBTECH 13: 86-91, 1995.
35. Collen D, Bernaerts R, Declerck P, De Cock F, Demarsin E, Jenn S, Laroche Y, Lijnen HR, Silence K, Verstreken M. Recombinant staphylokinase variants with altered immunoreactivity. I. Construction and characterization. Circulation 94: 197-206, 1996.
36. Rabijns A, De Bondt HL, De Ranter C. Three-dimensional structure of staphylokinase, a plasminogen activator with therapeutic potential. Nature Struct Biol 4: 357-360, 1997.

## Claims

1. Staphylokinase derivative having essentially the amino acid sequence as depicted in figure 1, **characterized in that** the derivative has at least the amino acid substitutions K130T and K135R, and said derivative being selected from the group consisting of:
SakSTAR (K130T, K135R),
SakSTAR (G36R, K130T, K135R),
SakSTAR (K74R, K130T, K135R,
SakSTAR (K74Q, K130T, K135R),
SakSTAR (G36R, K74R, K130T, K135R),
SakSTAR (G36R, K74Q, K130T, K135R),
SakSTAR (G36R, H43R, K74R, K130T, K135R),
SakSTAR (E65A, K74Q, K130T, K135R),
SakSTAR (E65Q, K74Q, K130T, K135R),
SakSTAR (K74Q, K86A, K130T, K135R),
SakSTAR (E65Q, T71S, K74Q, K130T, K135R),
SakSTAR (E65Q, K74Q, E108A, K109A, K130T, K135R), SakSTAR (E65Q, K74Q, K121A, K130T, K135R),
SakSTAR (E80A, D82A, K130T, K135R),
SakSTAR (K74R, E80A, D82A, K130T, K135R),
SakSTAR (K74Q, E80A, D82A, K130T, K135R),
SakSTAR (K35A, K74R, E80A, D82A, K130T, K135R),
SakSTAR (E65D, K74R, E80A, D82A, K130T, K135R),
SakSTAR (E65S, K74R, E80A, D82A, K130T, K135R),
SakSTAR (S34G, G36R, K74R, K130T, K135R),
SakSTAR (E65A, K74R, E80A, D82A, K130T, K135R),
SakSTAR (E65N, K74R, E80A, D82A, K130T, K135R),
SakSTAR (E65Q, K74R, E80A, D82A, K130T, K135R),
SakSTAR (K57A, E58A, E61A, E80A, D82A, K130T, K135R),
SakSTAR (E65Q, K74Q, E80A, D82A, K130T, K135R),
SakSTAR (K35A, E65D, K74Q, E80A, D82A, K130T, K135R),
SakSTAR (K74R, E80A, D82A, S103A, K130T, K135R),
SakSTAR (E65D, K74R, E80A, D82A, K109A, K130T, K135R),
SakSTAR (K35A, E65Q, K74R, E80A, D82A, T90A, E99D, T101S,
E108A, K109A, K130T, K135R).

2. Staphylokinase derivative as claimed in claim 1, which derivative has in addition
- an amino acid substituted with Cys.

3. Staphylokinase derivative as claimed in claim 1 or 2 with polyethylene glycol substitution, **characterized by** a maintained specific activity and a significantly reduced plasma clearance.

4. Staphylokinase derivative as claimed in claim 2 or 3, wherein the Cys is chemically modified with polyethylene glycol with molecular weights up to 20 kDa.

5. Staphylokinase derivatives as claimed in claim 2, 3 or 4 wherein selected amino acids in the NH₂-terminal region of 10 amino acids, are substituted with Cys, which is chemically modified with polyethylene glycol with molecular weights up to 20 kDa, which derivatives are **characterized by** a significantly reduced plasma clearance and maintained thrombolytic potency upon single intravenous bolus administration at a reduced dose.

6. Staphylokinase derivative as claimed in claim 5, wherein the serine in position 2 and/or 3 is substituted with a cysteine and the cysteine is chemically modified with polyethylene glycol having a molecular weight of 5, 10 or 20 kDa.

7. Staphylokinase derivative as claimed in claim 6, which derivative is SakSTAR(S3C-MP5,K35A,E65Q, K74R,E80A,D82A,T90A,E99D,T101S,E108A,K109A,K130T,E135R), wherein MP5 is maleimide-polyethylene glycol 5 kDa.

8. Staphylokinase derivative as claimed in claim 6, which derivative is SakSTAR(S3C-P10,K35A, E65Q,K74R,E80A,D82A,T90A,E99D,T101S,E108A,K109A,K130T, K135R), wherein P10 is maleimide-polyethylene glycol 10 kDa.

9. Staphylokinase derivative as claimed in claim 6, which derivative is SakSTAR(S3C-P20,K35A, E65Q,K74R,E80A,D82A,T90A,E99D,T101S,E108A,K109A,K130T, K135R), wherein P20 is maleimide-polyethylene glycol 20 kDa.

10. Staphylokinase derivative as claimed in claim 6, which derivative is SakSTAR(S3C-MP5,E65D,K74R, E80A,D82A,K130T,K135R) wherein MP5 is maleimide-polyethylene glycol 5 kDa.

11. Staphylokinase derivative as claimed in claim 6, which derivative is SakSTAR(S3C-SP5,E65D,K74R, E80A,D82A,K130T,K135R), wherein SP5 is OPS5-PEG 5 kDa.

12. Staphylokinase derivative as claimed in claim 6, which derivative is SakSTAR(S2C-SP5, S3C-SP5, E65D,K74R,E80A,D82A,K130T,K135R) wherein SP5 is OPS5-PEG 5 kDa.

13. Staphylokinase derivative as claimed in claim 6, which derivative is SakSTAR(S3C-P20,E65D,K74R, E80A,D82A,K130T,K135R), wherein P20 is maleimide-polyethylene glycol 20 kDa.

14. Staphylokinase derivative as claimed in claim 6, which derivative is SakSTAR(S3C-P20,E65D,K74-R, E80A,DB2A, K130T, K135R), wherein P20 is maleimide-polyethylene glycol 20 kDa.

15. Dimer of two staphylokinase derivatives as claimed in claim 2.

16. Pharmaceutical composition comprising at least one of the staphylokinase derivatives as claimed in any one of the claims 1-15 together with a suitable excipient.

17. Pharmaceutical composition as claimed in claim 16 for treating arterial thrombosis.

## Patentansprüche

1. Staphylokinase-Derivat, im wesentlichen mit der in Figur 1 gezeigten Aminosäuresequenz, **dadurch gekennzeichnet, dass** das Derivat mindestens die Aminosäuresubstitutionen K130T und K135R aufweist, und dass das Derivat aus der Gruppe ausgewählt ist, bestehend aus:
SakSTAR (K130T, K135R),
SakSTAR (G36R, K130T, K135R),
SakSTAR (K74R, K130T, K135R),
SakSTAR (K74Q, K130T, K135R),
SakSTAR (G36R, K74R, K130T, K135R),
SakSTAR (G36R, K74Q, K130T, K135R),
SakSTAR (G36R, H43R, K74R, K130T, K135R),
SakSTAR (E65A, K74Q, K130T, K135R),
SakSTAR (E65Q, K74Q, K130T. K135R),
SakSTAR (K74Q, K86A, K130T, K135R),
SakSTAR (E65Q, T71S, K74Q, K130T, K135R),
SakSTAR (E65Q, K74Q, E108A. K109A, K130T, K135R),
SakSTAR (E65Q, K74Q, K121A, K130T, K135R),
SakSTAR (E80A, D82A, K130T, K135R),
SakSTAR (K74R, E80A, D82A, K130T. K135R),
SakSTAR (K74Q, E80A, D82A, K130T, K135R),
SakSTAR (K35A, K74R, E80A, D82A, K130T, K135R),
SakSTAR (E65D, K74R, E80A, D82A, K130T, K135R),
SakSTAR (E65S, K74R, E80A, D82A, K130T, K135R),
SakSTAR (S34G, G36R, K74R, K130T, K135R),
SakSTAR (E65A, K74R, E80A, D82A, K130T, K135R),
SakSTAR (E65N, K74R, E80A, D82A, K130T, K135R),
SakSTAR (E65Q, K74R, E80A, D82A, K134T, K135R),
SakSTAR (K57A, E58A, E61A, E80A, D82A, K130T, K135R),
SakSTAR (E65Q, K74Q, E80A, D82A, K130T, K135R),
SakSTAR (K35A, E65D, K74Q, E80A, D82A, K130T, K135R),
SakSTAR (K74R, E80A, D82A, S103A, K130T, K135R),
SakSTAR (E65D, K74R, E80A, D82A, K109A, K130T, K135R),
SakSTAR (K35A, E65Q, K74R, E80A, D82A, T90A, E99D, T101S, E108A,
K109A, K130T, K135R).

2. Staphylokinase-Derivat nach Anspruch 1, wobei bei dem Derivat zusätzlich eine Aminosäure gegen Cys ausgetauscht ist.

3. Staphylokinase-Derivat nach Anspruch 1 oder 2 mit Polyethylenglycol-Substitution, **gekennzeichnet durch** eine aufrechterhaltene spezifische Aktivität und eine signifikant verringerte Plasma-Clearance.

4. Staphylokinase-Derivat nach Anspruch 2 oder 3, wobei das Cys mit Polyethylenglycol mit Molekulargewichten bis zu 20 kDa chemisch modifiziert ist.

5. Staphylokinase-Derivate nach Anspruch 2, 3 oder 4, wobei ausgewählte Aminosäuren im NH₂-terminalen Bereich von 10 Aminosäuren durch Cys substituiert sind, welches mit Polyethylenglycol mit Molekulargewichten bis zu 20 kDa chemisch modifiziert ist, wobei die Derivate **gekennzeichnet sind durch** eine signifikant verringerte Plasma-Clearance und eine aufrecht erhaltene thrombolytische Wirksamkeit nach einer einzelnen intravenösen Bolus-Verabreichung bei einer verringerten Dosis.

6. Staphylokinase-Derivat nach Anspruch 5, wobei das Serin in Stellung 2 und/oder 3 gegen ein Cystein ausgetauscht ist und das Cystein mit Polyethylenglycol mit einem Molekulargewicht von 5, 10 oder 20 kDa chemisch modifiziert ist.

7. Staphylokinase-Derivat nach Anspruch 6, wobei das Derivat SakSTAR(S3C-MP5, K35A, E65Q, K74R, E80A, D82A, T90A, E99D, T101S, E108A, K109A, K130T, K135R) ist und wobei MP5 Maleimid-Polyethylenglycol mit 5 kDA ist.

8. Staphylokinase-Derivat nach Anspruch 6, wobei das Derivat SakSTAR(S3C-P10, K35A, E65Q, K74R, E80A, D82A, T90A, E99D, T101S, E108A, K109A, K130T, K135R) ist und wobei P10 Mäleimid-Polyethylenglycol mit 10 kDA ist.

9. Staphylokinase-Derivat nach Anspruch 6, wobei das Derivat SakSTAR(S3C-P20, K35A, E65Q, K74R, E80A, D82A, T90A, E99D, T101S, E108A, K109A, K130T, K135R) ist und wobei P10 Maleimid-Polyethylenglycol mit 20 kDA ist.

10. Staphylokinase-Derivat nach Anspruch 6, wobei das Derivat SakSTAR(S3C-MP5, E65D, K74R, E80A, D82A, K130T, K135R) ist und wobei MP5 Maleimid-Polyethylenglycol mit 5 kDA ist.

11. Staphylokinase-Derivat nach Anspruch 6, wobei das Derivat SakSTAR(S3C-SP5, E65D, K74R, E80A, D82A, K130T, K135R) ist und wobei SP5 OPSS-PEG mit 5 kDA ist.

12. Staphylokinase-Derivat nach Anspruch 6, wobei das Derivat SakSTAR(S2C-SP5, S3C-SP5, E65D, K74R, E80A, D82A, K130T, K135R) ist und wobei SP5 OPSS-PEG mit 5 kDA ist.

13. Staphylokinase-Derivat nach Anspruch 6, wobei das Derivat SakSTAR(S3C-P20, E65D, K74R, E80A, D82A, K130T, K135R) ist und wobei P20 Maleimid-Polyethylenglycol mit 20 kDA ist.

14. Staphylokinase-Derivat nach Anspruch 6, wobei das Derivat SakSTAR(S3C-P20, E65D, K74R, E80A, D82A, K130T, K135R) ist und wobei P20 Maleimid-Polyethylenglycol mit 20 kDA ist.

15. Dimer aus zwei Staphylokinase-Derivaten nach Anspruch 2.

16. Pharmazeutische Zusammensetzung, umfassend mindestens eines der Staphylokinase-Derivate nach einem der Ansprüche 1 bis 15, zusammen mit einem geeigneten Exzipienten.

17. Pharmazeutische Zusammensetzung nach Anspruch 16 zur Behandlung von arterieller Thrombose.

## Revendications

1. Dérivé de staphylokinase ayant essentiellement la séquence d'acides aminés telle que décrite à la figure 1, **caractérisé en ce que** le dérivé a au moins les substitutions d'acides aminés K130T et K135R, et ledit dérivé étant choisi dans le groupe consistant en :
SakSTAR (K130T, K135R),
SakSTAR (G36R, K130T, K135R),
SakSTAR (K74R, K130T, K135R),
SakSTAR (K74Q, K130T, K135R),
SakSTAR (G36R, K74R, K130T, K135R),
SakSTAR (G36R, K74Q, K130T, K135R),
SakSTAR (G36R, H43R, K74R, K130T, K135R),
SakSTAR (E65A, K74Q, K130T, K135R),
SakSTAR (E65Q, K74Q, K130T, K135R),
SakSTAR (K74Q, K86A, K130T, K135R),
SakSTAR (E65Q, T71S, K74Q, K130T, K135R),
SakSTAR (E65Q, K74Q, E108A, K109A, K130T, K135R),
SakSTAR (E65Q, K74Q, K121A, K130T, K135R),
SakSTAR (E80A, D82A, K130T, K135R),
SakSTAR (K74R, E80A, D82A, K130T, K135R),
SakSTAR (K74Q, E80A, D82A, K130T, K135R),
SakSTAR (K35A, K74R, E80A, D82A, K130T, K135R),
SakSTAR (E65D, K74R, E80A, D82A, K130T, K135R),
SakSTAR (E65S, K74R, E80A, D82A, K130T, K135R),
SakSTAR (S34G, G36R, K74R, K130T, K135R),
SakSTAR (E65A, K74R, E80A, D82A, K130T, K135R),
SakSTAR (E65N, K74R, E80A, D82A, K130T, K135R),
SakSTAR (E65Q, K74R, E80A, D82A, K130T, K135R),
SakSTAR (K57A, E58A, E61A, E80A, D82A, K130T, K135R),
SakSTAR (E65Q, K74Q, E80A, D82A, K130T, K135R),
SakSTAR (K35A, E65D, K74Q, E80A, D82A, K130T, K135R),
SakSTAR (K74R, E80A, D82A, S103A, K130T, K135R),
SakSTAR (E65D, K74R, E80A, D82A, K109A, K130T, K135R),
SakSTAR (K35A, E65Q, K74R, E80A, D82A, T90A, E99D, T101S, E108A, K109A, K130T, K135R).

2. Dérivé de staphylokinase tel que revendiqué dans la revendication 1, lequel dérivé a en plus un acide aminé remplacé par Cys.

3. Dérivé de staphylokinase tel que revendiqué dans la revendication 1 ou 2, avec une substitution par du polyéthylèneglycol, **caractérisé par** une activité spécifique maintenue et une clairance plasmatique significativement réduite.

4. Dérivé de staphylokinase tel que revendiqué dans la revendication 2 ou 3, dans lequel le Cys est modifié par du polyéthylèneglycol avec des masses moléculaires allant jusqu'à 20 kDa.

5. Dérivé de staphylokinase tel que revendiqué dans la revendication 2, 3 ou 4, dans lequel des acides aminés sélectionnés dans la région NH₂-terminale de 10 acides aminés sont remplacés par Cys, qui est chimiquement modifié par du polyéthylèneglycol avec des masses moléculaires allant jusqu'à 20 kDa, lesquels dérivés sont **caractérisés par** une clairance plasmatique significativement réduite et une efficacité thrombolytique maintenue par une seule administration en bolus intraveineux à une dose réduite.

6. Dérivé de staphylokinase tel que revendiqué dans la revendication 5, dans lequel dans lequel la sérine en position 2 et/ou 3 est remplacée par une cystéine et la cystéine est chimiquement modifiée par un polyéthylèneglycol avec une masse moléculaire de 5, 10 ou 20 kDa.

7. Dérivé de staphylokinase tel que revendiqué dans la revendication 6, lequel dérivé est SakSTAR(S3C-MP5,K35A,E65Q,K74R,E80A,D82A,T90A,E99D,T101S,E108A,K109A, K130T,K135R), où MP5 représente le maléimide-polyéthylèneglycol 5 kDa.

8. Dérivé de staphylokinase tel que revendiqué dans la revendication 6, lequel dérivé est SakSTAR(S3C-P10,K35A,E65Q,K74R,E80A,D82A,T90A,E99D,T101S,E108A,K109A, K130T,K135R), où P10 représente le maléimide-polyéthylèneglycol 10 kDa.

9. Dérivé de staphylokinase tel que revendiqué dans la revendication 6, lequel dérivé est SakSTAR(S3C-P20,K35A,E65Q,K74R,E80A,D82A,T90A,E99D,T101S,E108A,K109A, K130T,K135R), où P20 représente le maléimide-polyéthylèneglycol 20 kDa.

10. Dérivé de staphylokinase tel que revendiqué dans la revendication 6, lequel dérivé est SakSTAR(S3C-MP5,E65D,K74R,E80A,D82A,K130T,K135R), où MP5 représente le maléimide-polyéthylèneglycol 5 kDa.

11. Dérivé de staphylokinase tel que revendiqué dans la revendication 6, lequel dérivé est SakSTAR(S3C-SP5,E65D,K74R,E80A,D82A,K130T,K135R), où SP5 représente OPSS-PEG 5 kDa.

12. Dérivé de staphylokinase tel que revendiqué dans la revendication 6, lequel dérivé est SakSTAR(S2C-SP5,S3C-SP5,E65D,K74R,E80A,D82A,K130T,K135R), où SP5 représente OPSS-PEG 5 kDa.

13. Dérivé de staphylokinase tel que revendiqué dans la revendication 6, lequel dérivé est SakSTAR(S3C-P20,E65D,K74R,E80A,D82A,K130T,K135R), où P20 représente le maléimide-polyéthylèneglycol 20 kDa.

14. Dérivé de staphylokinase tel que revendiqué dans la revendication 6, lequel dérivé est SakSTAR(S3C-P20,E65D,K74R,E80A,D82A,K130T,K135R), où P20 représente le maléimide-polyéthylèneglycol 20 kDa.

15. Dimère de deux dérivés de staphylokinase tels que revendiqués dans la revendication 2.

16. Composition pharmaceutique comprenant au moins un des dérivés de staphylokinase tels que revendiqués dans l'une quelconque des revendications 1 à 15 avec un excipient approprié.

17. Composition pharmaceutique telle que revendiqué dans la revendication 16 pour le traitement d'une thrombose artérielle.
